# EUROPEAN PATENT APPLICATION

(11) **EP 4 627 938 A1**
(43) Date of publication of application: **08.10.2025**
(21) Application number: 23896871.3
(22) Date of filing: 30.11.2023
(51) Int. Cl.: A23L 33/19, A23L 33/17, A23L 33/16, A23L 33/00, A23C 9/152, A23C 9/16, A23C 9/00, A23C 19/09, A23C 21/06, A23C 21/10, A23C 21/08

(54) **COMPOSITION FOR RESISTING ENTERITIS AND/OR PROTECTING INTEGRITY OF INTESTINAL BARRIER, AND USE THEREOF**

(30) Priority: 30.11.2022 CN 202211521747; 30.11.2022 CN 202211521748; 02.03.2023 CN 202310191772; 14.03.2023 CN 202310251121; 17.03.2023 CN 202310273125; 17.03.2023 CN 202310273151; 17.03.2023 CN 202310273091; 17.03.2023 CN 202310273135
(71) Applicant: Inner Mongolia Yili Industrial Group Co. Ltd., Hohhot, Inner Mongolia 010000 (CN); Inner Mongolia Dairy Technology Research Institute Co., Ltd., Hohhot, Inner Mongolia 010000 (CN)
(72) Inventor: DUAN, Sufang, Hohhot, Inner Mongolia 010000 (CN); LIU, Biao, Hohhot, Inner Mongolia 010000 (CN); SZETO, Ignatius Man-Yau, Hohhot, Inner Mongolia 010000 (CN); HE, Tingchao, Hohhot, Inner Mongolia 010000 (CN); YE, Wenhui, Hohhot, Inner Mongolia 010000 (CN); KONG, Xiaoyu, Hohhot, Inner Mongolia 010000 (CN); ZHANG, Xiaoying, Hohhot, Inner Mongolia 010000 (CN); LIU, Bin, Hohhot, Inner Mongolia 010000 (CN); LI, Yanjie, Hohhot, Inner Mongolia 010000 (CN); GAO, Jiajia, Hohhot, Inner Mongolia 010000 (CN); GUAN, Shangwei, Hohhot, Inner Mongolia 010000 (CN); LI, Fenxin, Hohhot, Inner Mongolia 010000 (CN)
(74) Representative: HGF
(86) International application number: PCT/CN2023/135413
(87) International publication number: WO 2024/114729

(57) **Abstract**

A composition of lactoferrin, osteopontin and calcium in defined proportions, which can alleviate and inhibit the expression of pro-inflammatory factors at the mRNA level in RAW264.7 macrophages induced by LPS, and down-regulate the expression of inflammatory pathway-associated proteins at the protein level, thereby alleviating the effects of inflammatory responses. Use of the composition in the preparation of a product for resisting enteritis and/or protecting the integrity of the intestinal barrier.

## Description

This application claims the priority of the Chinese patent application filed with the Chinese Patent Office on November 30, 2022, with application number 202211521747.2 and invention name "A composition and its application", the entire contents of which are incorporated by reference into this application.

This application claims the priority of the Chinese patent application filed with the Chinese Patent Office on March 02, 2023, with application number 202310191772.7 and invention name "A composition and its application", the entire contents of which are incorporated by reference into this application.

This application claims the priority of the Chinese patent application filed with the Chinese Patent Office on November 30, 2022, with application number 202211521748.7 and invention name "Application of a nutritional component in the manufacture of products for resisting enteritis and/or protecting integrity of intestinal barrier", the entire contents of which are incorporated by reference into this application.

This application claims the priority of the Chinese patent application filed with the Chinese Patent Office on March 14, 2023, with application number 202310251121.2 and invention name "Application of a nutritional component in the manufacture of a product for resisting enteritis and/or protecting integrity of intestinal barrier", the entire contents of which are incorporated by reference into this application.

This application claims the priority of the Chinese patent application filed with the Chinese Patent Office on March 17, 2023, with application number 202310273125.0 and invention name "Application of a nutritional component in the manufacture of formula foods for special medical purpose for resisting enteritis and/or protecting integrity of intestinal barrier", the entire contents of which are incorporated by reference into this application.

This application claims the priority of the Chinese patent application filed with the Chinese Patent Office on March 17, 2023, with application number 202310273151.3 and invention name "Application of a protein complex in the manufacture of formula foods for resisting enteritis and/or protecting integrity of intestinal barrier", the entire contents of which are incorporated by reference into this application.

This application claims the priority of the Chinese patent application filed with the Chinese Patent Office on March 17, 2023, with application number 202310273091.5 and invention name "Dairy product and its use in the manufacture of anti-inflammation products", the entire contents of which are incorporated by reference into this application.

This application claims the priority of the Chinese patent application filed with the Chinese Patent Office on March 17, 2023, with application number 202310273135.4 and invention name "Nutritional composition and its use in the manufacture of anti-inflammation products", the entire contents of which are incorporated by reference into this application.

### FIELD

The present invention belongs to the technical field of food, and in particular relates to a composition having functions of resisting enteritis and/or protecting integrity of intestinal barrier and use thereof.

### BACKGROUND

Lactoferrin (LF) is an iron-binding glycoprotein, a member of the red transferrin family, expressed in most biologically active fluids including milk, and is a major part of the mammalian innate immune system. The protection ranges from direct antibacterial activity against a wide range of microorganisms, including bacteria, pathogens, fungi, and parasites, to anti-inflammatory and anti-cancer activity. Lactoferrin was first discovered in bovine milk in 1939, and was isolated from human milk and characterized in 1960. The content of lactoferrin in bovine milk (normal milk) is generally 30 mg/L, and that in human milk (normal milk) is 1000 mg/L. The content of lactoferrin in colostrum is several times higher.

Osteopontin (OPN) is a highly phosphorylated glycoprotein rich in sialic acid, and is also an important cell adhesion and chemotactic factor, which can be synthesized and secreted by a variety of cells in the body (such as osteoblasts, epithelial cells, activated T lymphocytes, monocyte/macrophage cell lines, and nerve cells). Osteopontin is present in most tissues and body fluids, with the highest concentration in breast milk. Osteopontin in breast milk accounts for about 5% to 10% of the total protein, and the content thereof in breast milk is reported to be 99.7 to 266.2 mg/L. Studies have found that osteopontin can significantly promote the proliferation of human small intestinal crypt cells, reduce the activity of intestinal neutrophils in mice with acute enteritis, reduce the secretion of a series of proinflammatory factors such as tumor necrosis factor (TNF-α) and interferon γ, and reduce harmful host responses by enhancing and restoring the epithelial barrier.

At present, research on lactoferrin and osteopontin has attracted more and more attention, but in terms of anti-inflammatory efficacy, especially anti-intestinal inflammation and protection of the intestinal mucosal barrier, the combined use of lactoferrin and osteopontin, and the further effect of the combined use of the two with calcium are still unclear and need further exploration.

### SUMMARY

**In** view of this, the technical problem to be solved by the present disclosure is to provide a composition having a function of resisting enteritis and/or protecting integrity of intestinal barrier and use thereof. The composition provided by the present disclosure can enhance the intestinal immunity of the human body, protect the intestinal permeability, promote the secretion of intestinal mucin and maintain the intestinal mucosal barrier.

The present disclosure provides a composition, consisting of lactoferrin, osteopontin and calcium salt, and a mass ratio of the lactoferrin, osteopontin and calcium in the calcium salt is 1: (0.05-5): (0.001-40).

Preferably, the mass ratio of the lactoferrin, osteopontin and calcium in the calcium salt is 1: (0.1-5): (0.001-40).

Preferably, the mass ratio of the lactoferrin, osteopontin and calcium in the calcium salt is 1: (0.15-5): (0.006-20).

Preferably, the mass ratio of the lactoferrin, osteopontin and calcium in the calcium salt is 1: (0.15-5): (0.01-10).

Preferably, the mass ratio of the lactoferrin, osteopontin and calcium in the calcium salt is 1: (0.15-5): (0.01-0.1).

Preferably, the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

The present disclosure further provides use of the above composition in the manufacture of an inhibitor of inflammatory factor expression.

Preferably, the inflammatory factor is IL-1β and/or IL-6.

The present disclosure further provides use of the above composition in the manufacture of an anti-inflammation product.

Preferably, the anti-inflammation includes inhibiting the expression of inflammatory pathway proteins.

The present disclosure further provides a food, comprising the above composition.

The present disclosure further provides a dairy product, comprising lactoferrin, osteopontin and calcium salt;
a mass fraction of the lactoferrin in the dairy product is 0.006%-0.7%;
a mass fraction of the osteopontin in the dairy product is 0.01%-0.04%;
a mass fraction of calcium in the calcium salt in the dairy product is 0.01%-1.5%.

Preferably, a mass ratio of the lactoferrin, osteopontin and calcium is 1:(0.1-5):(0.001-40).

Preferably, the lactoferrin is derived from one or more selected from the group consisting of cow milk or its products, goat milk or its products, and camel milk or its products.

Preferably, the osteopontin is derived from one or more selected from the group consisting of cow milk or its products, goat milk or its products, and camel milk or its products.

Preferably, the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

Preferably, the dairy product further comprises one or more selected from the group consisting of milk fat, vegetable oil, fish oil, dietary fiber and probiotic.

Preferably, the vegetable oil is one or more selected from the group consisting of sunflower oil, corn oil, coconut oil, rapeseed oil, flaxseed oil, peanut oil and soybean oil;

the dietary fiber is one or more selected from the group consisting of galacto-oligosaccharides, fructo-oligosaccharides and breast milk oligosaccharides;

the probiotic is one or more selected from the group consisting of bifidobacteria, yeast, probiotic spore bacteria, *Clostridium butyricum* and lactobacillus.

Preferably, the dairy product is one or more selected from the group consisting of dairy products for infants, dairy products for pregnant women or new mothers and dairy products for pets.

Preferably, the dairy product comprises, in parts by weight, 0-2000 parts of raw milk, 120-350 parts of lactose, 0-300 parts of whole milk powder, 0-450 parts of skimmed milk powder, 0.01-375 parts of desalted whey powder, 0.01-170 parts of whey protein powder, 0-80 parts of sunflower oil, 0-40 parts of corn oil, 0-80 parts of soybean oil, 0-30 parts of coconut oil, 0-85 parts of low-erucic acid rapeseed oil, 0-20 parts of flaxseed oil. 0-240 parts of 1,3-dioleyl-2-palmitoyl triglyceride, 0-55 parts of α-lactalbumin powder, 0-40 parts of β-casein powder, 0-5 parts of soybean phospholipid, 0-2 parts of anhydrous milk fat, 0-25 parts of fructo-oligosaccharide powder, 0-100 parts of galacto-oligosaccharide syrup, 0.01-5 parts of milk mineral salt, 2-5 parts of vitamins, 0-3 parts of choline chloride, 0-15 parts of minerals, 0-3 parts of magnesium chloride, 0-5 parts of potassium chloride, 0-20 parts of docosahexaenoic acid, 0-25 parts of eicosatetraenoic acid, 0-0.2 parts of bifidobacteria, 0-8.5 parts of lactoferrin, and 0-1 part of nucleotides.

The present disclosure further provides use of the above-mentioned dairy product in the manufacture of an anti-inflammation product.

The present disclosure further provides a nutritional composition, comprising protein, fat, carbohydrate and calcium salt;
the protein comprises lactoferrin and osteopontin;
in the nutritional composition, a total content of the protein is (9-50g)/100g, a content of the fat is (12-30g)/100g, a content of the carbohydrate is (30-60g)/100g, a content of the lactoferrin is (0.005-3g)/100g, a content of the osteopontin is (0.01-1g)/100g, and a content of the calcium is (0.1-2g)/100g.

Preferably, a mass ratio of the lactoferrin, osteopontin and calcium is 1: (0.1-5): (0.001-40).

Preferably, in the nutritional composition, a total content of the protein is (15-35g)/100g, a content of the fat is (12-20g)/100g, a content of the carbohydrate is (32-50g)/100g, a content of the lactoferrin is (0.005-0.8g)/100g, a content of the osteopontin is (0.03-0.5g)/100g, and a content of the calcium is (0.1-1g)/100g.

Preferably, the lactoferrin is one or more selected from the group consisting of cow milk or its products, goat milk or its products, and camel milk or its products;
the osteopontin is one or more selected from the group consisting of cow milk or its products, goat milk or its products, and camel milk or its products;
the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

Preferably, a raw material for providing the protein includes one or more of sodium caseinate, calcium caseinate, casein, hydrolyzed whey protein, hydrolyzed casein, hydrolyzed milk protein, hydrolyzed milk fat globule membrane protein, and isolated whey protein;
a raw material for providing the fat includes one or more of milk fat, vegetable oil, fish oil, and triglycerides.

Preferably, the nutritional composition further includes one or more of amino acids, nucleotides, vitamins, probiotics, and inorganic salts.

Preferably, the nutritional composition specifically comprises 0-200 parts by weight of whey protein powder; 0-100 parts by weight of sodium caseinate; 0-100 parts by weight of casein; 0-400 parts by weight of solid corn syrup; 0-400 parts by weight of maltodextrin; 0-50 parts by weight of resistant dextrin; 0-50 parts by weight of fructo-oligosaccharide; 0-150 parts by weight of sunflower oil; 0-40 parts by weight of corn oil; 20-80 parts by weight of soybean oil; 0-100 parts by weight of medium-chain triglycerides; 0-10 parts by weight of tryptophan; 0-10 parts by weight of arginine; 0.01-7.5 parts by weight of lactoferrin; 0.75-37.5 parts by weight of osteopontin; 0.0075-300 parts by weight of calcium salt; 7-50 parts by weight of a compound nutrient package comprising calcium powder, vitamins and minerals; 5-80 parts by weight of DHA; 0-100 parts by weight of glutamine; and 10-140 parts by weight of EPA.

The present disclosure further provides use of the above-mentioned nutritional composition in the manufacture of an anti-inflammation product.

Preferably, the anti-inflammation product is a food.

Preferably, the anti-inflammation product is a formula food for special medical purpose.

Preferably, the anti-inflammation is to resist a chronic inflammation.

Preferably, a patient who has the chronic inflammation is a patient with tumor or a patient with traumatic stress.

The present disclosure further provides use of a nutritional component in the manufacture of a product for resisting enteritis and/or protecting integrity of intestinal barrier, wherein the nutritional component comprises a complex of lactoferrin and osteopontin.

Preferably, the lactoferrin and osteopontin are combined to each other by electrostatic interaction;
preferably, a mass ratio of lactoferrin to osteopontin is 1:10-1:0.05, preferably 1:5-1:0.1.

Preferably, sources of lactoferrin and osteopontin include cow milk, goat milk and their products, including but not limited to whole milk powder, skimmed milk powder, casein, and whey protein powder.

Preferably, the enteritis includes *Escherichia coli* enteritis.

Preferably, the resisting enteritis includes reducing the number of serum cytokines IL-1β, IL-4, and IL-10, reducing the number of neutrophils in intestinal tissue, and reducing the number of CD4⁺ T cells in intestinal tissue;
the protecting integrity of intestinal barrier includes protecting intestinal tissue from damage, protecting mucus barrier, and protecting integrity of intestinal mucosal mechanical barrier.

Preferably, the product is a food, a healthcare food, or a nutrient supplement.

Preferably, the product includes a dairy product, and the dairy product includes milk powder, liquid milk, cheese, condensed milk, milk fat, or other dairy products.

Preferably, the product is a formula food.

Preferably, in the formula food, the nutritional component is a protein complex, and the protein complex is selected from a protein complex formed by lactoferrin and osteopontin via electrostatic interaction in a mass ratio of 1:10 to 1:0.05.

Preferably, the formula food is a formula milk powder, a formula food for special medical purpose, or a healthcare food.

Preferably, a mass ratio of lactoferrin to osteopontin is 1:5 to 1:0.1.

Preferably, sources of lactoferrin and osteopontin include cow milk, goat milk and their products, including but not limited to one or more slected from the group consisting of whole milk powder, skimmed milk powder, casein and whey protein powder.

Preferably, the enteritis includes *Escherichia coli* enteritis.

Preferably, the resisting enteritis includes reducing the number of serum cytokines IL-1β, IL-4, and IL-10, reducing the number of neutrophils in intestinal tissues, and reducing the number of CD4⁺ T cells in intestinal tissues.

Preferably, the protecting integrity of intestinal barrier includes protecting against intestinal tissue damage, protecting mucus barrier, and protecting integrity of intestinal mucosal mechanical barrier.

Preferably, a content of the lactoferrin in the formula food is 1-750 mg/100g, and a content of the osteopontin in the formula food is 20-500 mg/100g.

Preferably, a total protein content of the formula food is 10-20g/100g, a proportion of the whey protein in the total protein is 40-70%; a content of the fat is 15-30g/100g; and a content of the dietary fiber is 0.95-6.3g/100g.

Preferably, the formula food further comprises one or more selected from the group consisting of probiotics, lactose, DHA, ARA, nucleotides, calcium powder, and compound nutrients of vitamins and minerals.

Preferably, the formula food is an infant formula milk powder, which comprises by weight:

0-2000 parts of raw cow milk, 120-350 parts of lactose, 0-300 parts of whole milk powder, 0-325 parts of skimmed milk powder, 0-375 parts of desalted whey powder, 0-170 parts of whey protein powder, 0-80 parts of sunflower oil, 0-40 parts of corn oil, 0-80 parts of soybean oil, 0-30 parts of coconut oil, 0-85 parts of low-erucic acid rapeseed oil, 0-20 parts of flaxseed oil, 1,3-dioleyl-2-palmitoyl triglyceride 0-240 parts, α-lactalbumin powder 0-55 parts, 0-40 parts of β-casein powder, 0-5 parts of soybean phospholipid, 0-2 parts of anhydrous milk fat, 0-25 parts of fructo-oligosaccharide powder, 0-100 parts of galacto-oligosaccharide syrup, 2-5 parts of compound vitamin nutrient package, 0-3 parts of choline chloride nutrient package, 0-2 parts of mineral nutrition I package, 0-13 parts of mineral II nutrient package, 0-3 parts of magnesium chloride nutrient package, 0-5 parts of potassium chloride nutrient package, 0-20 parts of DHA, 0-25 parts of ARA, 0-0.2 parts of bifidobacterium, 0-8.5 parts of lactoferrin, and 0-1 part of nucleotide;

wherein, amounts of the raw cow milk, whole milk powder, and skimmed milk powder used are not all equal to 0; and amounts of the sunflower oil, corn oil, soybean oil, coconut oil, low-erucic acid rapeseed oil, flaxseed oil, and 1,3-dioleyl-2-palmitoyl triglyceride used are not all equal to 0flaxseed oil.

Preferably, the formula food is a formula food for special medical purpose.

Preferably, in the formula food for special medical purpose, a nutritional component is selected from a protein complex formed by lactoferrin and osteopontin via electrostatic interaction in a mass ratio of 1:10 to 1:0.1.

Preferably, the resisting enteritis and/or protection of integrity of intestinal barrier include preventing enteritis in premature/low birth weight infants and/or protecting the integrity of intestinal barrier of premature/low birth weight infants, preventing enteritis in lactose-intolerant infants and/or protecting the integrity of intestinal barrier of lactose-intolerant infants, preventing enteritis in patients with tumor and/or protecting the integrity of intestinal barrier of patients with tumor, preventing enteritis in patients with traumatic stress and/or protecting the integrity of intestinal barrier of patients with traumatic stress.

Preferably, a mass ratio of the lactoferrin and osteopontin is 1:5 to 1:0.1.

Preferably, the mass ratio of the lactoferrin and osteopontin is 1:5 to 1:0.2.

Preferably, the enteritis includes *Escherichia coli* enteritis.

Preferably, the resisting enteritis includes reducing the number of serum cytokines IL-1β, IL-4, and IL-10, reducing the number of neutrophils in intestinal tissue, and reducing the number of CD4⁺ T cells in intestinal tissue.

Preferably, the protecting integrity of intestinal barrier includes protecting against intestinal tissue damage, protecting mucus barrier, and protecting integrity of intestinal mucosal mechanical barrier.

Preferably, a content of the lactoferrin in the formula food for special medical purpose is 1-750 mg/100g.

Preferably, a content of the osteopontin in the formula food for special medical purpose is 20-600 mg/100g.

Preferably, the formula food for special medical purpose includes a dairy product, and the dairy product includes milk powder or liquid milk.

Preferably, the formula food for special medical purpose is a formula food for premature/low birth weight infant, and comprises raw materials of:
Whey protein powder 80-180 weight parts;
Lactose 0-580 weight parts;
Solid corn syrup 0-580 weight parts;
Sunflower oil 0-150 weight parts;
Corn oil 0-40 weight parts;
Soybean oil 20-80 weight parts;
OPO structured fat 0-140 weight parts;
Lactoferrin 0-7.5 weight parts;
Osteopontin 0-75 weight parts;
Compound nutrients comprising calcium powder, vitamins and minerals 7-50 weight parts;
DHA 2-15 weight parts;
ARA 3-22 weight parts;
Bifidobacterium 0.1-0.2 weight parts; and
Nucleotide 0.1-5 weight parts.

The formula food for special medical purpose is a formula food for lactose-intolerant infant, and comprises raw materials of:
Isolated whey protein powder 90-150 weight parts;
Lactose-free whole milk powder 0-500 weight parts;
Solid corn syrup 0-600 weight parts;
Sunflower oil 0-150 weight parts;
Corn oil 0-40 weight parts;
Soybean oil 20-80 weight parts;
OPO structured fat 0-140 weight parts;
Lactoferrin 0-7.5 weight parts;
Osteopontin 0-75 weight parts;
Compound nutrients comprising calcium powder, vitamins and minerals 7-50 weight parts;
DHA 2-15 weight parts;
ARA 3-22 weight parts; and
Nucleotide 0.1-5 weight parts.

The formula food for special medical purpose is a formula food suitable for patients with tumor, and comprises raw materials of:
Whey protein powder 90-200 weight parts;
Sodium caseinate 0-100 weight parts;
Casein 0-100 parts by weight;
Solid corn syrup 0-400 parts by weight;
Maltodextrin 0-400 parts by weight;
Sunflower oil 0-150 parts by weight;
Corn oil 0-40 parts by weight;
Soybean oil 20-80 parts by weight;
Medium chain triglycerides 0-100 parts by weight;
Fructo-oligosaccharides 0-50 parts by weight;
Lactoferrin 0-7.5 parts by weight;
Osteopontin 0-75 parts by weight;
Compound nutrients comprising calcium powder, vitamins and minerals 7-50 parts by weight;
DHA 5-80 parts by weight;
Tryptophan 0-10 parts by weight;
Arginine 0-10 parts by weight;
Glutamine 0-100 parts by weight; and
EPA 10-140 parts by weight;
The formula food for special medical purpose is a formula food suitable for patients with traumatic stress, and comprises raw materials of:
   Whey protein powder 90-200 parts by weight;
   Sodium caseinate 0-100 parts by weight;
   Casein 0-100 parts by weight;
   Solid corn syrup 0-400 parts by weight;
   Maltodextrin 0-400 parts by weight;
   Sunflower oil 0-150 parts by weight;
   Corn oil 0-40 parts by weight;
   Soybean oil 20-80 parts by weight;
   Medium chain triglycerides 0-100 parts by weight;
   Fructo-oligosaccharides 0-50 parts by weight;
   Lactoferrin 0-7.5 parts by weight;
   Osteopontin 0-75 parts by weight;
   Compound nutrients comprising calcium powder, vitamins and minerals 7-50 parts by weight;
   DHA 5-50 parts by weight;
   Tryptophan 0-10 parts by weight;
   Arginine 0-10 parts by weight;
   Glutamine 0-100 parts by weight; and
   EPA 10-100 parts by weight.

The present disclosure furher provides a product for resisting enteritis and/or protecting integrity of intestinal barrier, comprising a complex of lactoferrin and osteopontin.

Preferably, the product includes a dairy product, preferably, the dairy product includes milk powder, liquid milk, cheese, condensed milk, milk fat or other dairy products.

Preferably, an amount of the lactoferrin added to the product is 1-750 mg/100g product, and an amount of the lactoferrin added to the product is 20-200 mg/100g product.

Preferably, the lactoferrin and osteopontin are combined to each other through electrostatic interaction;
preferably, a mass ratio of the lactoferrin to osteopontin is 1:10-1:0.05, preferably 1:5-1:0.1.

Preferably, sources of the lactoferrin and osteopontin include cow milk, goat milk and their products, including but not limited to whole milk powder, skimmed milk powder, casein, whey protein powder.

Compared with the prior art, the composition provided by the present disclosure has a synergistic inhibitory effect on inflammation and high safety and is worthy of promotion and application. The experimental examples of the present disclosure have confirmed that the lactoferrin, osteopontin and calcium under a limited ratio of the present disclosure can alleviate the expression of proinflammatory factors at the mRNA level in RAW264.7 macrophages induced by LPS, and downregulate the expression of proteins related to the inflammatory pathway at the protein level, thereby alleviating the impact of the inflammatory response.

The present disclosure further provides use of a nutritional component in the manufacture of a product for resisting enteritis and/or protecting integrity of intestinal barrier, wherein the nutritional component comprises a complex of lactoferrin and osteopontin. The complex of lactoferrin and osteopontin provided by the present disclosure can synergistically increase serum anti-inflammatory factors IL-4 and IL-10 and reduce proinflammatory factors IL-1β; enhance intestinal epithelial tissue lymphocytes, inhibit activated CD4⁺ cells and neutrophils, enhance intestinal immunity, protect intestinal permeability, promote secretion of intestinal mucin and maintain the intestinal mucosal barrier.

### BRIEF DESCRIPTION OF DRAWINGS

FIG. 1 is a flow chart of LF-OPN-calcium ion treatment of macrophages in the experimental example of the present disclosure;
FIG. 2 is the effect of LF-OPN-calcium combination on cells under LPS in the experimental example of the present disclosure;
FIG. 3 is the effect of lactoferrin and osteopontin undigested on cell activity in the experimental example of the present disclosure;
FIG. 4 is the effect of lactoferrin and osteopontin digested on cell activity in the experimental example of the present disclosure;
FIG. 5 is the effect of LF-OPN-calcium combination on IL-1β mRNA level expression in the experimental example of the present disclosure;
FIG. 6 is the effect of LF-OPN-calcium combination on IL-6 mRNA level expression in the experimental example of the present disclosure;
FIG. 7 is the effect of LF-OPN-calcium combination on the expression of proteins related to inflammatory pathways in the experimental example of the present disclosure;
FIG. 8 is the effect of LF-OPN combination and LF-OPN-calcium combination on IL-1β in the experimental example of the present disclosure mRNA level expression;
FIG. 9 is a schematic diagram of the establishment of the enteritis mouse model;
FIG. 10a is the overall immune effect of the protein combination on enteritis mice (IL-1(3);
FIG. 10b is the overall immune effect of the protein combination on enteritis mice (IL-4);
FIG. 10c is the overall immune effect of the protein combination on enteritis mice (IL-10);
FIG. 11 is the local immune effect of the protein combination on the intestine (neutrophils);
FIG. 12 is the local immune effect of the protein combination on the intestine (CD4⁺T cells);
FIG. 13 is the protective effect of the protein combination on intestinal permeability;
FIG. 14 is a photo of the protective effect of the protein combination on intestinal tissue damage;
FIG. 15 is the test results of villus length;
FIG. 16 is the protective effect of the protein combination on the mucus barrier;
FIG. 17 is the protective effect of the protein combination on the integrity of the intestinal mucosal mechanical barrier.

### DETAILED DESCRIPTION

The present disclosure provides a composition, which is composed of lactoferrin (LF), osteopontin (OPN) and calcium salt, wherein the mass ratio of lactoferrin, osteopontin and calcium in calcium salt is 1: (0.05-5): (0.001-40).

Preferably, the mass ratio of lactoferrin, osteopontin and calcium in calcium salt is 1: (0.1-5): (0.001-40).

Preferably, the mass ratio of lactoferrin, osteopontin and calcium in calcium salt is 1: (0.15-5): (0.006-20).

Preferably, the mass ratio of lactoferrin, osteopontin and calcium in calcium salt is 1: (0.15-5): (0.01-10).

Preferably, the mass ratio of lactoferrin, osteopontin and calcium in calcium salt is 1: (0.15-5): (0.01-0.1).

In an embodiment of the present disclosure, the mass ratio of lactoferrin, osteopontin and calcium in calcium salt is 1: 0.2: 0.012.

In an embodiment of the present disclosure, the mass ratio of lactoferrin, osteopontin and calcium in calcium salt is 1: 1: 0.011.

In an embodiment of the present disclosure, the mass ratio of lactoferrin, osteopontin and calcium in calcium salt is 1: 1: 0.02.

In the present disclosure, the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

Milk mineral salt, also known as milk calcium, whey mineral concentrate, and whey calcium, is derived from cow milk. It is a milky white powder obtained by treating cow milk through membrane permeation, separation, concentration, and spray drying. Its main component is calcium phosphate. In addition, it also comprises abundant nutrients such as protein, lactose, zinc, phosphorus, sodium, potassium, and magnesium. The content of calcium is about 23% to 28%, and the calcium-phosphorus ratio is 2:1, which is more conducive to human absorption and utilization. As a new calcium source, it is widely used in various foods.

In the present disclosure, the lactoferrin is derived from cow milk, goat milk and their products, including but not limited to whole milk powder, skimmed milk powder, casein and whey protein powder, preferably whey protein powder.

The osteopontin is derived from cow milk, goat milk and their products, including but not limited to whole milk powder, skimmed milk powder, casein and whey protein powder, preferably whey protein powder.

In the composition of the present disclosure, the concentration of lactoferrin is 1 mg/ml, and the concentration of osteopontin is 1 mg/ml.

The composition provided by the present disclosure is administered orally or enterally; the composition can be used for premature infants, low birth weight infants, pregnant or lactating women.

The present disclosure also provides use of the above composition in the manufacture of an inflammatory factor expression inhibitor.

In the use of the composition, the concentration of lactoferrin is less than 5 mg/ml, preferably 1 mg/ml, the concentration of osteopontin is less than 5 mg/ml, preferably 1 mg/ml, and the calcium exists in the form of calcium ions, and the concentration of calcium ions is 0.0068-1.2 mg/ml.

In the present disclosure, the dosage form of the inflammatory factor expression inhibitor includes an oral or inhalation dosage form or other pharmaceutically acceptable dosage forms;
the oral dosage form includes one or more of tablets, capsules, powders, syrups, dry syrups, liquids or suspensions;
the inhalation dosage form includes one or more of powder sprays, aerosols or sprays.

The inflammatory factor is IL-1β and/or IL-6.

IL-1β and IL-6 are the most common pro-inflammatory factors. The experimental examples of the present disclosure confirms that in the lipopolysaccharide (LPS)-induced inflammation model, the composition, compared with LF and OPN monomer digests, can significantly inhibit the expression of inflammatory factor genes of macrophages, and has a synergistic effect.

The present disclosure also provides the use of the above composition in the manufacture of an anti-inflammation product.

In the use of the composition, the concentration of lactoferrin is less than 5 mg/ml, preferably 1 mg/ml, the concentration of osteopontin is less than 5 mg/ml, preferably 1 mg/ml, and the calcium exists in the form of calcium ions, and the concentration of calcium ions is 0.0068-1.2 mg/ml.

The anti-inflammation includes inhibiting the expression of inflammatory pathway proteins.

In the present disclosure, the inflammatory pathway proteins include one or more of membrane proteins, NF-κB pathway proteins, and STAT pathway proteins; the membrane proteins include but are not limited to TLR4; the NF-κB pathway proteins include but are not limited to p-p65; the STAT pathway proteins include but are not limited to p-STAT3.

The experimental example of the present disclosure confirms that after the digest of the composition provided by the present disclosure is used to treat RAW264.7 macrophages, the expression of membrane protein TLR4, key protein p-p65 of the NF-κB pathway, and p-STAT3 in the downstream STAT pathway is significantly inhibited.

The present disclosure also provides a food, comprising the composition.

In the present disclosure, the food is one or more of dairy products, cooking products, special foods for infants, special foods for pregnant women or new mothers, nutritional formula products or pet food products;
the dairy product is a liquid dairy product, formula milk powder, milk tablets or cheese.

The food of the present disclosure can be in the form of beverages, biscuits, cakes or pastry products.

The composition provided by the present disclosure has a synergistic inhibitory effect on inflammation and high safety, and is worthy of promotion and application. The experimental examples of the present disclosure confirm that the lactoferrin, osteopontin and calcium salt at a certain ratio of the present disclosure can alleviate the expression of pro-inflammatory factors in RAW264.7 macrophages induced by LPS at the mRNA level, and downregulate the expression of proteins related to the inflammatory pathway at the protein level, thereby alleviating the impact of inflammatory response.

The present disclosure also provides a dairy product and its use in the manufacture of an anti-inflammation product. The dairy product of the present disclosure comprises lactoferrin, osteopontin and calcium, has a synergistic inhibitory effect on inflammation and high safety, and is worthy of promotion and application.

Specifically, the present disclosure provides a dairy product, comprising lactoferrin, osteopontin and calcium salt;
the mass fraction of the lactoferrin in the dairy product is 0.006%-0.7%;
the mass fraction of the osteopontin in the dairy product is 0.01%-0.04%;
the mass fraction of the calcium in the calcium salt in the dairy product is 0.01%-1.5%.

In the present disclosure, the mass fraction of the lactoferrin in the dairy product is 0.03-0.6%;
the mass fraction of the osteopontin in the dairy product is 0.02%-0.04%;
the mass fraction of the calcium in the calcium salt in the dairy product is 0.3%-1.5%.

In the present disclosure, the mass ratio of the lactoferrin, osteopontin and calcium is 1:(0.1-5):(0.001-40); preferably 1:(0.15-5):(0.006-20), more preferably 1:(0.15-5):(0.01-10).

In the present disclosure, the mass ratio of the lactoferrin, osteopontin and calcium is 1:(0.1-5):(0.011-0.06).

In the present disclosure, the mass fraction of the lactoferrin in the dairy product is 0.03%-0.2%;
the mass fraction of the osteopontin in the dairy product is 0.022%-0.04%;
the mass fraction of the calcium in the calcium salt in the dairy product is 0.3%-0.5%.

In an embodiment of the present disclosure, the mass fraction of the lactoferrin in the dairy product is 0.035%;
the mass fraction of the osteopontin in the dairy product is 0.03%;
the mass fraction of the calcium in the calcium salt in the dairy products is 0.35%.

In an embodiment of the present disclosure, the mass fraction of the lactoferrin in the dairy product is 0.11%;
the mass fraction of the osteopontin in the dairy product is 0.022%;
the mass fraction of the calcium in the calcium salt in the dairy product is 0.45%.

In an embodiment of the present disclosure, the mass fraction of the lactoferrin in the dairy product is 0.008%;
the mass fraction of the osteopontin in the dairy product is 0.04%;
the mass fraction of the calcium in the calcium salt in the dairy product is 0.32%.

In the present disclosure, the lactoferrin is derived from one or more selected from the group consisting of cow milk or its products, goat milk or its products, camel milk or its products, preferably whey protein powder; in an embodiment of the present disclosure, the lactoferrin is derived from WPC80 whey protein powder and D90 desalted whey powder.

In the present disclosure, the osteopontin is derived from one or more selected from the group consisting of cow milk or its products, goat milk or its products, camel milk or its products, preferably whey protein powder; in an embodiment of the present disclosure, the osteopontin is derived from WPC80 whey protein powder and D90 desalted whey powder.

In the present disclosure, the calcium salt is one or more of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate; the main component of the milk mineral salt is calcium phosphate, and the milk mineral salt additionally comprises protein, lactose and zinc, phosphorus, sodium, potassium, magnesium, and other nutrients, the calcium content is about 23% to 28%, and the calcium-phosphorus ratio is 2:1; in an embodiment of the present disclosure, the calcium salt is calcium chloride.

In the present disclosure, in addition to the above components, the dairy product also comprises one or more of milk fat, vegetable oil, fish oil, dietary fiber, and probiotics;
the vegetable oil is one or more selected from the group consisting of sunflower oil, corn oil, coconut oil, rapeseed oil, flaxseed oil, peanut oil, and soybean oil;
the mass content of the vegetable oil in the dairy product is 0.1%-10%;
the dietary fiber is one or more selected from the group consisting of galacto-oligosaccharides, fructo-oligosaccharide, and breast milk oligosaccharides;
the mass content of the dietary fiber in the dairy product is 0.9%-7%.

Galacto-oligosaccharides (GOS) are milky white or slightly yellow powders refined by processes such as enzymatic synthesis and spray drying with whey as raw materials. GOS are functional oligosaccharides of breast milk. Galacto-oligosaccharides have a strong proliferation effect on the probiotics in the intestine, and have the same effect as plant fiber, which can promote gastrointestinal motility and improve intestinal function. Galacto-oligosaccharides are quite stable to heat and acid, and can meet the requirements of various food processing technologies.

Fructo-oligosaccharide (FOS) is a natural active substance with a sweetness of 0.3-0.6 times that of sucrose. Fructo-oligosaccharide not only maintains the pure sweetness of sucrose, but also tastes more refreshing than sucrose. It has health functions such as regulating intestinal flora, proliferating bifidobacteria, promoting calcium absorption, regulating blood lipids, immune regulation, and anti-caries.

Human milk oligosaccharides (HMOs) are carbohydrates composed of 3-10 monosaccharide units connected by glycoside chains. HMOs are complex mixed oligosaccharides present in human breast milk. HMOs can promote the growth of probiotics, inhibit the adhesion of harmful bacteria, protect the intestinal barrier, regulate intestinal cell proliferation and differentiation, regulate the body's immunity, and promote the development of cognitive function.

The probiotics are one or more selected from the group consisting of bifidobacteria, yeast, probiotic spore bacteria, *Clostridium butyricum,* and lactobacillus;
the mass content of the probiotics in dairy products is ≤0.02%.

In an embodiment of the present disclosure, the probiotics are bifidobacteria; the probiotics are preferably added to the dairy product in the form of probiotic powder. More preferably, based on 1000 parts by weight of the dairy product, the amount of bifidobacterium powder added is 0-0.2 parts by weight; more preferably 0.18-0.2 parts by weight. More preferably, each part by weight of bifidobacterium powder contains more than 3×10¹⁰ CFU of bifidobacteria.

Adding probiotics to dairy products can help lactose protein to decompose better, and the amino acid molecules of probiotics are more easily absorbed by the human body. For infants with incomplete intestinal function, it can better avoid the problem of indigestion.

In the present disclosure, the dairy product also preferably comprises cream, sugar, nucleotides and choline chloride;
the mass content of the lactose in the dairy product is 12% to 35%;
the mass content of the nucleotides in the dairy product is less than 0.07%;
the mass content of the choline chloride in the dairy product is less than 0.2%.

The dairy product of the present disclosure can be one or more of a dairy product for infants, a dairy product for pregnant women or new mothers, or a dairy product for pets; the dairy product of the present disclosure can be in the form of a beverage, biscuit, cake or pastry product.

The dairy product includes one or more of fermented milk, concentrated dairy products, milk powder, cheese, traditional dairy products, and milk-containing frozen drinks and milk-containing beverages.

The dairy product is preferably milk powder; further preferably infant formula milk powder; the total protein content in the infant formula milk powder is 10-20g/100g, the proportion of whey protein in the total protein is 40%-70%; the fat content is 15-30g/100g; the dietary fiber content is 0.95-6.3g/100g, and the carbohydrate content is 50-57g/100g; preferably, the linoleic acid content in the infant formula powder is 2700-4500mg/100g, and the α-linolenic acid content is 270-450mg/100g.

According to a specific embodiment of the present disclosure, in the infant formula powder of the present disclosure, the raw materials providing total protein include: raw milk, whole milk powder, skimmed milk powder, whey protein powder, desalted whey powder, and one or more of β-casein.

According to a specific embodiment of the present disclosure, in the infant formula powder of the present disclosure, the raw materials providing fat include vegetable oil or OPO structured fat in addition to the basic raw materials containing milk fat.

According to a specific embodiment of the present disclosure, in the infant formula powder of the present disclosure, the raw materials for providing carbohydrates include: lactose and basic raw materials containing lactose such as milk, whole milk powder and/or skimmed milk powder, etc., and lactose raw materials should be added to provide carbohydrates. That is, in the infant formula milk powder of the present disclosure, the raw materials for providing carbohydrates include raw material lactose in addition to the basic raw materials containing lactose, and the specific amount of lactose added is adjusted to make the carbohydrate content in the infant formula milk powder of the present disclosure 50-57g/100g.

According to a specific embodiment of the present disclosure, based on 1000 parts by weight of the infant formula milk powder of the present disclosure, its raw materials include: DHA 0-20 (preferably 8-17) parts by weight, ARA 0.1-25 parts by weight, lactoferrin 0.1-8.5 parts by weight, nucleotide 0.1-0.7 parts by weight.

According to a specific embodiment of the present disclosure, the raw materials of the infant formula milk powder of the present disclosure may further include compound nutrients comprising calcium powder, vitamins and minerals. The compound nutrients are a combination of nutrients that meet national standards, and different addition amounts are used according to different formulas. Preferably, based on 1000 parts by weight of infant formula milk powder, the compound nutrients comprising calcium powder, vitamins and minerals are 7-25 parts by weight. More preferably, the compound nutrients are added in the form of at least a compound vitamin package, calcium powder, and a mineral nutrient package. Specifically, the content of each component in the compound nutrients is as follows:
1) Compound vitamin nutrient package, the content of each component in each gram of compound vitamin nutrient package is as follows:
   Taurine: 140-340mg
   Vitamin A: 1700-5800µgRE
   Vitamin D: 25-70µg
   Vitamin B₁: 3000-6800µg
   Vitamin B₂: 3500-6900µg
   Vitamin B₆: 2400-4000µg
   Vitamin B₁₂: 8-20µg
   Vitamin Ki: 200-700µg
   Vitamin C: 155-700mg
   Vitamin E: 10-70mg α-TE
   Niacinamide: 10000-41550µg
   Folic acid: 500-920µg
   Biotin: 100-245µg
   Pantothenic acid: 7100-25230µg
   Inositol: 0-250mg
   L-carnitine: 0-60mg
2) Mineral I nutrient package, the content of each component in each gram of mineral I nutrient package is as follows:
   Iron: 40-110mg
   Zinc: 23-90mg
   Copper: 2600-4180µg
   Iodine: 500-995µg
   Selenium: 0-2 00µg
   Manganese: 0-579µg
3) Mineral II nutrient package, the content of each component in each gram of mineral II nutrient package is as follows:
   Calcium: 120-455mg
   Phosphorus: 0-150mg
4) Magnesium chloride nutrient package, each gram of magnesium chloride nutrient package contains:
   Magnesium: 80-170mg
5) Potassium chloride nutrient package, each gram of potassium chloride nutrient package contains:
   Potassium: 400-580mg;
6) Choline chloride nutrient package, each gram of choline chloride nutrient package contains:
   Choline: 300-950mg.

Preferably, based on 1000 parts by weight of infant formula milk powder, the amount of the compound vitamin nutrient package added is 2 to 4 parts by weight, the amount of the mineral II nutrient package added is 2 to 12 parts by weight, the amount of the mineral I nutrient package added is 0.5 to 3 parts by weight, the amount of the magnesium chloride nutrient package added is 0 to 2 parts by weight, the amount of the potassium chloride nutrient package added is 0 to 4.5 parts by weight, and the amount of the choline chloride nutrient added is 0 to 2 parts by weight, and the base material of each nutrient package is preferably lactose or sodium L-ascorbate.

Preferably, based on 1000 parts by weight of infant formula milk powder, the amount of bifidobacterium powder added is 0 to 0.2 parts by weight; more preferably 0.18 to 0.2 parts by weight. More preferably, each part by weight of bifidobacterium powder contains more than 3×10¹⁰ CFU of bifidobacteria.

In the infant formula milk powder of the present disclosure, the raw material phospholipids and/or anhydrous milk fat used are mainly used for the molding of powder particles during spray drying, and the phospholipids can be soybean phospholipid and/or lecithin. The amount of phospholipids and anhydrous milk fat used is small, but they also contribute to the fat content in the milk powder products.

Preferably, the infant formula milk powder comprises, in parts by weight, 0-2000 parts of raw cow milk, 120-350 parts of lactose, 0-300 parts of whole milk powder, 0-450 parts of skimmed milk powder, 0.01-375 parts of desalted whey powder, 0.01-170 parts of whey protein powder, 0-80 parts of sunflower oil, 0-40 parts of corn oil, 0-80 parts of soybean oil, 0-30 parts of coconut oil, 0-85 parts of low-erucic acid rapeseed oil, 0-20 parts of flaxseed oil, 1 ,3-dioleyl-2-palmitoyl triglyceride 0-240 parts, α-lactalbumin powder 0-55 parts, β-casein powder 0-40 parts, soybean phospholipid 0-5 parts, anhydrous milk fat 0-2 parts, fructo-oligosaccharide powder 0-25 parts, galacto-oligosaccharide syrup 0-100 parts, milk mineral salt 0.01-5 parts, vitamins 2-5 parts, choline chloride 0-3 parts, minerals 0-15 parts, magnesium chloride 0-3 parts, potassium chloride 0-5 parts, docosahexaenoic acid (DHA) 0-20 parts, eicosatetraenoic acid (ARA) 0-25 parts, bifidobacteria 0-0.2 parts, lactoferrin 0-8.5 parts, nucleotides 0-1 parts;
preferably, the infant formula milk powder of the present disclosure, in parts by weight, comprises:

| | |
|---|---|
| raw cow milk | 0-2000 parts by weight; |
| lactose | 125-350 parts by weight; |
| whole milk powder | 0-300 parts by weight of; |
| skimmed milk powder | 0-325 parts by weight of; |
| desalted whey powder | 0-375 parts by weight of; |
| whey protein powder | 0-170 parts by weight of; |
| sunflower oil | 0-80 parts by weight of; |
| corn oil | 0-40 parts by weight of; |
| soybean oil | 0-80 parts by weight of; |
| coconut oil | 0-30 parts by weight of; |
| low-erucic acid rapeseed oil | 0-85 parts by weight of; |
| flaxseed oil | 0-20 parts by weightflaxseed oil; |
| 1,3-dioleyl-2-palmitoylglycerol triglyceride | 0-240 parts by weight; |
| α-lactalbumin powder | 0-55 parts by weight; |
| β-casein powder | 0-40 parts by weight; |
| soy lecithin | 0-5 parts by weight; |
| anhydrous milk fat | 0-2 parts by weight; |
| fructo-oligosaccharide powder | 0-25 parts by weight; |
| galacto-oligosaccharide syrup | 0-100 parts by weight; |
| milk mineral salt | 0-5 parts; |
| compound nutrients comprising calcium powder, vitamins and minerals | 7-25 parts by weight; |
| DHA | 0-20 parts by weight; |
| ARA | 0-25 parts by weight; |
| bifidobacterium | 0-0.2 parts by weight; |
| lactoferrin | 0-8.5 parts by weight; |
| nucleotide | 0-1 parts by weight. |

It can be understood that in the infant formula milk powder of the present disclosure, the specific amount of each raw material should be adjusted and determined on the premise of meeting the requirements for the formula milk powder product indicators. In the infant formula milk powder of the present disclosure, the product performance indicators that are not described or listed in detail should be implemented in accordance with the national standards for infant formula foods and relevant standards and regulations.

In the infant formula milk powder of the present disclosure, each raw material is commercially available, and the selection of each raw material should meet the requirements of relevant standards. In addition, the compound nutrients can also be compounded in house. The present disclosure only uses "compound" for the convenience of expression, and it does not mean that the components in the compound must be mixed together before use. All raw materials should be added and used under the premise of meeting relevant regulations.

The present disclosure has no special restrictions on the preparation method of the dairy product, and conventional methods in the field can be used. When the product is milk powder, the preparation method of milk powder comprises: material formulation, homogenization, concentration and sterilization, spray drying, and dry mixing to obtain a finished product; the preparation method of milk powder preferably comprises: 1) coarse filtration of milk; 2) homogenization and sterilization of milk; 3) mixing milk and powder raw materials; 4) dissolving and oiling: putting oil into the oiling room, and mixing it with the product obtained in step 3) after the oil is melted; 5) dissolving calcium powder, vitamins, and minerals in pure water respectively, and adding them to the mixing tank in sequence to obtain a mixed liquid; 6) removing impurities in the mixed liquid; 7) homogenizing the liquid obtained in step 6) through a homogenizer, 8) cooling the product obtained in step 7); 9) concentrating and sterilizing the product obtained in step 8); 10) spray drying the concentrated milk to obtain milk powder; the preparation method of milk powder further comprises a post-processing packaging step, preferably comprising: screening powder, discharging powder, loading powder, packaging, boxing, sampling and storage.

The present disclosure also provides the use of the above-mentioned dairy products in the manufacture of anti-inflammation products.

In the present disclosure, the anti-inflammation includes inhibiting the expression of genes related to pro-inflammatory factors, and/or down-regulating the expression of proteins related to the inflammatory pathway;

The pro-inflammatory factors include IL-1β and/or IL-6, preferably IL-1β;

The proteins related to the inflammatory pathway include one or more of TLR4, p-p65, and p-STAT3.

The dairy products provided by the present disclosure contain lactoferrin, osteopontin, and calcium, which have a synergistic inhibitory effect on inflammation, and high safety, and are worthy of promotion and application, and play an important role in the healthy development of infants and young children. The experimental examples of the present disclosure have confirmed that the three components of lactoferrin, osteopontin, and calcium can alleviate the expression of pro-inflammatory factors in RAW264.7 macrophages induced by LPS at the mRNA level, and down-regulate the expression of proteins related to the inflammatory pathway at the protein level, thereby alleviating the impact of inflammatory response.

The present disclosure also provides a nutritional composition and its use in the manufacture of anti-inflammation products. The nutritional composition of the present disclosure contains lactoferrin, osteopontin and calcium, has a synergistic inhibitory effect on chronic inflammation that is easily produced in adults in diseased states such as patients with tumor and patients with traumatic stress, has high safety and is worthy of promotion and application.

Specifically, in the use of the nutritional composition provided by the present disclosure in the manufacture of anti-inflammation products:
the nutritional composition includes protein, fat, carbohydrate and calcium salt;
the protein includes lactoferrin and osteopontin;
in the nutritional composition, the total protein content is (9-50g)/100g, the fat content is (12-30g)/100g, the carbohydrate content is (30-60g)/100g, the lactoferrin content is (0.005-3g)/100g, the osteopontin content is (0.01-1g)/100g, and the calcium content is (0.1-2g)/100g.

In the present disclosure, the mass ratio of lactoferrin, osteopontin and calcium is 1: (0.1-5): (0.001-40); the mass ratio of lactoferrin, osteopontin and calcium is preferably 1: (0.1-5): (0.011-0.06), and more preferably 1: (0.1-5): (0.011-0.02).

In the present disclosure, the anti-inflammation includes inhibiting the expression of genes related to pro-inflammatory factors, and/or down-regulating the expression of proteins related to the inflammatory pathway;
the pro-inflammatory factors include IL-1β and/or IL-6, preferably IL-1β;
the proteins related to the inflammatory pathway include one or more of TLR4, p-p65, and p-STAT3.

In the present disclosure, the anti-inflammation product is preferably a food;
the anti-inflammation product is a formula food for special medical purpose; the formula food for special medical purpose refers to a formula food specially processed and prepared to meet the special needs of people with limited food intake, digestive and absorption disorders, metabolic disorders or specific disease states for nutrients or diets, including a formula food for special medical purposes for infants aged 0 to 12 months and a formula food for special medical purposes for people over 1 year old;
the anti-inflammation is preferably to resist chronic inflammation;
the patient who produces the chronic inflammation is preferably a patient with tumor or a patient with traumatic stress. Chronic inflammation is a long-term, slowly progressive inflammation that can last for months to many years. Sometimes people do not know that they are suffering from chronic inflammation. Chronic inflammation and related diseases are the main causes of death in humans. Stress can cause chronic systemic low-level inflammation in the body, and chronic low-level inflammation is the soil for various diseases, which in turn has a negative impact on human health. The occurrence and development of tumors are related to chronic inflammation. The microenvironment formed by chronic inflammation is crucial to the occurrence, development, and metastasis of tumors. At the same time, chronic inflammation is also the body's response to tumors. In general, inflammation has a promoting effect on tumors.

In the nutritional composition of the present disclosure, the total protein content is (15-35g)/100g, the fat content is (12-20g)/100g, the carbohydrate content is (32-50g)/100g, the lactoferrin content is (0.005-0.8g)/100g, the osteopontin content is (0.03-0.5g)/100g, and the calcium content is (0.1-1g)/100g.

In an embodiment of the present disclosure, the nutritional composition has a total protein content of 34g/100g, a fat content of 12g/100g, a carbohydrate content of 32g/100g, a lactoferrin content of 600mg/100g, an osteopontin content of 60mg/100g, and a calcium content of 300mg/100g.

In an embodiment of the present disclosure, the nutritional composition has a total protein content of 33g/100g, a fat content of 18.5g/100g, a carbohydrate content of 34g/100g, a lactoferrin content of 16mg/100g, an osteopontin content of 80mg/100g, and a calcium content of 640mg/100g.

In the present disclosure, the lactoferrin is derived from one or more selected from the group consisting of cow milk or its products, goat milk or its products, camel milk or its products, preferably whey protein powder.

In the present disclosure, the osteopontin is derived from one or more selected from the group consisting of cow milk or its products, goat milk or its products, camel milk or its products, preferably whey protein powder.

In the present disclosure, the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate. Milk mineral salt, also known as milk calcium, whey mineral concentrate, whey calcium, is derived from cow milk. It is a milky white powder obtained by treating cow milk through membrane permeation, separation, concentration and spray drying. Its main component is calcium phosphate. In addition, it also includes protein, lactose and zinc, phosphorus, sodium, potassium, magnesium and other nutrients. The calcium content is about 23% to 28%, and its calcium-phosphorus ratio is 2:1, which is more conducive to human absorption and utilization.

In the present disclosure, the raw materials for providing the protein include one or more of sodium caseinate, calcium caseinate, casein, hydrolyzed whey protein, hydrolyzed casein, hydrolyzed milk protein, hydrolyzed milk fat globule membrane protein, and isolated whey protein;
the raw materials for providing the fat include one or more of milk fat, vegetable oil, fish oil, and triglycerides; triglycerides are preferably medium-chain triglycerides;
the vegetable oil is one or more selected from the group consisting of sunflower oil, corn oil, coconut oil, low-erucic acid rapeseed oil, flaxseed oil, peanut oil, soybean oil, palm oil, and walnut oil; preferably sunflower oil, corn oil, and soybean oil; the addition of vegetable oil can not only provide fat components for the nutritional composition on the one hand, but also provide linoleic acid and α-linolenic acid the other hand; more preferably, based on 1000 parts by weight of the nutritional composition, the raw materials comprise 0-150 parts by weight of sunflower oil; 0-80 parts by weight of corn oil; 0-80 parts by weight of soybean oil; the addition of fish oil can not only provide fat components, but also provide unsaturated fatty acids; unsaturated fatty acids include docosahexaenoic acid (DHA), arachidonic acid (ARA) and eicosapentaenoic acid (EPA);
the raw materials for providing the carbohydrates include one or more of lactose, gelatinized starch, maltodextrin, solid corn syrup, glucose syrup, and dietary fiber; the carbohydrates in the nutritional composition of the present disclosure are partly derived from lactose and partly from non-lactose source materials, and the non-lactose source materials include but are not limited to gelatinized starch, maltodextrin, solid corn syrup, and glucose syrup; that is, in the nutritional composition of the present disclosure, the raw materials for providing carbohydrates include raw lactose and pre-hydrolyzed and gelatinized starch materials in addition to the basic raw materials containing lactose; preferably, based on 1000 parts by weight of the nutritional composition, the raw materials comprise: 0-580 parts by weight of lactose and 0-580 parts by weight of non-lactose materials; the above raw materials can adjust the specific addition amount of lactose within the above range so that the carbohydrate content in the nutritional composition of the present disclosure is 50-58g/100g; the dietary fiber is preferably fructo-oligosaccharide; fructo-oligosaccharide (FOS) is a natural active substance with a sweetness of 0.3-0.6 times that of sucrose. Fructo-oligosaccharide not only maintains the pure sweetness of sucrose, but also tastes fresher than sucrose. It has the health functions of regulating intestinal flora, proliferating bifidobacteria, promoting calcium absorption, regulating blood lipids, regulating immunity, and resisting dental caries;
the nutritional composition also includes one or more of amino acids and vitamins;
the amino acids include arginine and/or tryptophan;
in an embodiment of the present disclosure, based on 1000 parts by weight of the nutritional composition, the raw materials include: 8-50 parts by weight of DHA, 14-28 parts by weight of ARA; 10-140 parts by weight of EPA; 0-10 parts by weight of arginine; 0-10 parts by weight of tryptophan; 0-7.5 parts by weight of lactoferrin; 0-75 parts by weight of osteopontin; 7-50 parts by weight of a compound nutrient package containing calcium powder, vitamins and minerals.

In the nutritional composition of the present disclosure, the compound nutrient package is a combination of nutrients that meet national standards, and different addition amounts are used according to different formulas. Any one or any combination of the following compound nutrient package ingredients can be selectively used in the nutritional composition of the present disclosure if nutrients are added as needed. Preferably, the compound nutrient package at least includes compound vitamins, calcium powder, and mineral nutrient packages, and the content of each component in the compound nutrient package is as follows:
1) Compound vitamin nutrient package, each gram of compound vitamin nutrient package comprises:
   Taurine: 140-340mg
   Vitamin A: 1700-5800µgRE
   Vitamin D: 25-70µg
   Vitamin B1: 2000-6800µg
   Vitamin B2: 3000-6900µg
   Vitamin B6: 1700-4000µg
   Vitamin B12: 8-20µg
   Vitamin K1: 200-700µg
   Vitamin C: 0-700mg
   Vitamin E: 10-70mg α-TE
   Niacinamide: 10000-41550µg
   Folic acid: 350-920µg
   Biotin: 70-245µg
   Pantothenic acid: 7100-25230µg
   Inositol: 0-250mg
   L-carnitine: 0-60mg
2) Mineral I nutrient package, each gram of mineral I nutrient package comprises:
   Iron: 20-110mg
   Zinc: 23-90mg
   Copper: 2000-4180µg
   Iodine: 500-995µg
   Selenium: 0-200µg
   Manganese: 0-579µg
3) Mineral II nutrient package, each gram of mineral II nutrient package comprises:
   Sodium: 40-100mg
   Potassium: 200-500mg
4) Mineral III nutrient package, each gram of mineral III nutrient package comprises:
   Calcium: 200-500mg
   Phosphorus: 75-300mg
5) Compound magnesium chloride nutrient package, each gram of compound magnesium chloride nutrient package contains:
   Magnesium: 80-170mg
6) Choline chloride nutrient package, each gram of choline chloride nutrient package contains:
   Choline: 300-950 mg

The base material of the above-mentioned compound nutrient package is preferably lactose, maltodextrin, solid corn syrup or sodium L-ascorbate. Based on 1000 parts by weight of the nutritional composition, the addition amount of the compound nutrient package is 7 to 52 parts by weight, wherein the compound vitamin nutrient package is preferably 2 to 4 parts by weight, the mineral II nutrient package is preferably 2 to 20 parts by weight, the mineral III nutrient package is preferably 0.5 to 20 parts by weight, the mineral I nutrient package is preferably 0.5 to 6 parts by weight, magnesium chloride is 0 to 7 parts by weight, and choline chloride is 0 to 3 parts by weight. The base material of each nutrient package is preferably maltodextrin, lactose or sodium L-ascorbate.

The compound raw materials used for each nutrient provided in the nutrient package may interact with each other. For example, sulfate can accelerate the oxidation and destruction process of vitamins, reducing their utilization rate. Since sulfate appears in the form of ions in aqueous solution, it acts as an oxidant in the oxidation reaction to induce the oxidation of vitamins and destroy the structure of vitamins. Trace elements have different abilities in redox reactions, with copper, zinc and iron being the most active, followed by manganese and selenium. B vitamins and vitamin C are easily affected by copper ions, and vitamin B₂ is easily affected by iron ions.

**In** order to ensure the utilization efficiency of nutrients, the present disclosure selects stable nutrient dosage forms, for example: retinyl acetate is used as vitamin A. Retinol contains 1 hydroxyl group and 5 double bonds, which is very easy to oxidization, but the stability of retinol in the form of acetate will be greatly improved. Tocopherol acetate is used as vitamin E. Tocopherol is unstable, but tocopherol acetate has higher stability. Thiamine nitrate is used as vitamin B₁, which is more stable than thiamine hydrochloride. Sodium L-ascorbate is used as vitamin C.

The content of each component of the above-mentioned compound nutrient package refers to the amount of the nutrient substance added to strengthen the nutrient substance, and does not include the content of the nutrient components in other raw materials of the nutrient composition. According to a preferred specific embodiment of the present disclosure, the formula of the nutrient composition of the present disclosure comprises:
Whey protein powder 0-200 weight parts;
Sodium caseinate 0-100 weight parts;
Casein 0-100 weight parts;
Solid corn syrup 0-400 weight parts;
Maltodextrin 0-400 weight parts;
Resistant dextrin 0-50 weight parts;
Fructo-oligosaccharide 0-50 weight parts;
Sunflower oil 0-150 weight parts;
Corn oil 0-40 weight parts;
Soybean oil 20-80 weight parts;
Medium chain triglyceride 0-100 weight parts;
Tryptophan 0-10 weight parts;
Arginine 0-10 weight parts;
Lactoferrin 0.01-7.5 weight parts;
Osteopontin 0.75-37.5 parts by weight;
Compound nutrient package comprising calcium powder, vitamins and minerals 7-50 parts by weight;
DHA 5-80 parts by weight;
glutamine 0-100 parts by weight;
EPA 10-140 parts by weight.

It can be understood that the specific amount of each raw material in the nutritional composition of the present disclosure should be adjusted and determined on the premise of meeting the product indicator requirements of the nutritional composition.

In the nutritional composition of the present disclosure, each raw material can be commercially available, and the selection of each raw material should meet the relevant standard requirements, wherein the lactoferrin, osteopontin and calcium salt should meet the requirements of the present disclosure. In addition, the compound nutrient package can also be compounded by itself. The present disclosure only uses "compound" for the convenience of expression, and does not mean that the components in the compound must be mixed together before use. All raw materials should be added and used under the premise of meeting relevant regulations.

The present disclosure also provides a nutritional composition, comprising protein, fat, carbohydrate and calcium salt;
the protein includes lactoferrin and osteopontin;
in the nutritional composition, the total protein content is (9-50g)/100g, the fat content is (12-30g)/100g, the carbohydrate content is (30-60g)/100g, the lactoferrin content is (0.005-3g)/100g, the osteopontin content is (0.01-1g)/100g, and the calcium content is (0.1-2g)/100g.

In the present disclosure, the nutritional composition specifically comprises 0-200 parts by weight of whey protein powder; 0-100 parts by weight of sodium caseinate; 0-100 parts by weight of casein; 0-400 parts by weight of solid corn syrup; 0-400 parts by weight of maltodextrin; 0-50 parts by weight of resistant dextrin; 0-50 parts by weight of fructo-oligosaccharide; 0-150 parts by weight of sunflower oil; 0-40 parts by weight of corn oil; 20-80 parts by weight of soybean oil; 0-100 parts by weight of medium-chain triglycerides; 0-10 parts by weight of tryptophan; 0-10 parts by weight of arginine; 0.01-7.5 parts by weight of lactoferrin; 0.75-37.5 parts by weight of osteopontin; 0.0075-300 parts by weight of calcium salt; 7-50 parts by weight of a compound nutrient package comprising calcium powder, vitamins and minerals; 5-80 parts by weight of DHA; 0-100 parts by weight of glutamine; and 10-140 parts by weight of EPA.

The present disclosure has no special restrictions on the preparation method of the nutritional composition, and conventional methods in the art can be used. The preparation method of the nutritional composition comprises: material formulation, homogenization, concentration and sterilization, spray drying, and dry mixing to obtain a finished product;
the preparation method preferably comprises:
1) Addition of powder: the powder raw materials are added to the powder mixing tank uniformly through an air delivery system after being measured according to the formula for storage;
2) Vacuum suction of powder: various powder raw materials in the powder mixing tank are sucked into a vacuum mixing tank through a vacuum system;
3) Melting oil: putting the oil specified in the formula into an oil melting room according to the formula requirements;
4) Storage of mixed oil: the mixed oil is stored in an oil storage tank under heat preservation;
5) Weighing: the mixed oil is pumped into the mixing tank through the oil pump according to the formula requirements;
6) Dissolution and addition of nutrient: the compound nutrient package is dissolved with water and added into the mixing tank;
7) Dissolution and addition of lactoferrin, osteopontin and calcium salt: the osteopontin and calcium salt are dissolved according to the steps in step 6, and then added to the mixing tank;
8) Filtration: the mixed liquid is filtered through a filter to remove physical impurities in the raw materials;
9) Homogenization: the filtered liquid is homogenized by a homogenizer;
10) Cooling and storage: the homogenized liquid enters a plate heat exchanger for cooling;
11) Concentration and sterilization: double-effect concentration is used during production; the discharge concentration is 48%-52% dry matter;
12) Storage, preheating, filtration, and spray drying of concentrated milk: the concentrated milk is temporarily stored in the concentrated milk balance tank; after the material is preheated, filtered and dried, the fine powder obtained is agglomerated at the top of the tower;
13) Drying and cooling by fluidized bed: the powder coming out of the drying tower is dried twice in the fluidized bed (primary) and cooled in the fluidized bed (secondary);
14) Packaging: the staff of the powder making workshop weighs and seals the DHA, ARA and lactoferrin according to the formula requirements;
15) Dry mixing: the weighed DHA, EPA, ARA, lactoferrin and milk powder are mixed in a dry mixer; if the protein content is high, some casein, whey protein powder and sodium caseinate can be mixed and added evenly through dry mixing;
16) Powder screening: a vibrating screen is used to make the particle size of the milk powder uniform, and the powder residue is discarded;
17) Powder discharging: a sterilized powder collection box is used to collect the powder and transport it from the powder discharging room to the powdering room;
18) Powder loading: the milk powder was added into the powder storage tank of large and small packaging machines according to the packaging requirements;
19) Packaging: 800g of the milk powder is packaged with nitrogen flushed by automatic packaging machine800g of the milk powder is packaged with nitrogen flushed by automatic packaging machine, the oxygen content during the nitrogen filling is less than 1%; 900g of milk powder is packaged by iron cans automatically with nitrogen flushed, and the oxygen content is less than 5%;
20) Packing: the packaged small bags are put into the carton, the powder spoon is also added, and the carton is sealed with a carton sealing machine;
21) Inspection of finished products: sampling inspection of packaged products according to the inspection plan is conducted;
22) Storage: products that have passed the inspection are stored in a warehouse, and are required to be stored at room temperature and under humidity ≤65%.

The nutritional composition provided by the present disclosure comprises lactoferrin, osteopontin and calcium, which have a synergistic inhibitory effect on inflammation, especially for chronic inflammation that is easily produced in adult in diseased states such as patients with tumor and patients with traumatic stress, and has high safety and is worthy of promotion and application. The experimental examples of the present disclosure have confirmed that the three components of lactoferrin, osteopontin and calcium can alleviate the expression of pro-inflammatory factors in RAW264.7 macrophages induced by LPS at the mRNA level, and downregulate the expression of proteins related to the inflammatory pathway at the protein level, thereby alleviating the impact of inflammatory response.

The present disclosure provides use of a nutritional component in the manufacture of a product for resisting enteritis and/or protecting integrity of intestinal barrier, wherein the nutritional component includes a complex of lactoferrin and osteopontin.

The mass ratio of the lactoferrin to osteopontin is 1:10 to 1:0.05, preferably 1:10, 1:5, 1:1, 1:0.2, 1:0.1, 1:0.05, or any value between 1:10 and 1:0.05, more preferably any value between 1:5 and 1:0.1.

Lactoferrin (LF) is an iron-binding lactose protein. LF is beneficial to intestinal health in the following aspects, including (a) enhancing the permeability of intestinal epithelial monolayer cells; (b) facilitating microbial antagonism, preventing the colonization and proliferation of intestinal pathogens; (c) enhancing the growth and maturation of intestinal monolayer cell components and intestinal nerve fibers; (d) balancing anti-inflammatory and pro-inflammatory responses to maintain intestinal homeostasis. Studies have shown that insufficient levels of osteopontin (OPN) in infant formula milk powder may partially explain the developmental differences between infants fed with formula milk powder or breast milk. In the premature pig model test, the OPN-fed group had a higher ratio of villi to crypts in the small intestine tissue, and higher monocyte and lymphocyte counts than the control group, and could cause slight improvements in intestinal structure and systemic immunity. In the present disclosure, the lactoferrin and osteopontin bind to each other through electrostatic interaction, thereby achieving a synergistic effect of resistingresisting enteritis and/or protecting the integrity of the intestinal barrier.

In the present disclosure, the sources of lactoferrin and osteopontin include cow milk, goat milk and their products, including but not limited to whole milk powder, skimmed milk powder, casein, and whey protein powder.

In some specific embodiments of the present disclosure, the nutritional components may also include minerals, vitamins, probiotics and prebiotics.

In the present disclosure, the nutritional components have the effect of resisting enteritis and/or protecting the integrity of the intestinal barrier.

In some specific embodiments of the present disclosure, the enteritis includes *Escherichia coli* enteritis.

The resisting enteritis includes reducing the number of serum cytokines IL-1β, IL-4, and IL-10, reducing the number of neutrophils in intestinal tissue, and reducing the number of CD4⁺T cells in intestinal tissue.

The protecting integrity of intestinal barrier includes protecting against intestinal tissue damage, protecting mucus barrier and protecting integrity of intestinal mucosal mechanical barrier.

In the present disclosure, the product is a food, a healthcare food or a nutrient supplement.

The product includes a dairy product. The dairy product includes milk powder, liquid milk, cheese, condensed milk, milk fat or other dairy products.

The amount of lactoferrin added to the product is 1 to 750 mg/100g product, preferably 1, 5, 10, 50, 100, 200, 300, 400, 500, 600, 700, 750, or any value between 1 and 750 mg/100g product, and the amount of lactoferrin added to the product is 20 to 200 mg/100g product, preferably 20, 40, 60, 80, 100, 120, 140, 180, 200, or any value between 20 and 200 mg/100g product.

The present disclosure also provides a product for resisting enteritis and/or protecting integrity of intestinal barrier, including a complex of lactoferrin and osteopontin.

The product includes dairy products, preferably, the dairy products include milk powder, liquid milk, cheese, condensed milk, milk fat or other dairy products;

The amount of the lactoferrin added to the product is 1-750 mg/100g product, preferably 1, 5, 10, 50, 100, 200, 300, 400, 500, 600, 700, 750, or any value between 1-750 mg/100g product, and the amount of lactoferrin added to the product is 20-200 mg/100g product, preferably 20, 40, 60, 80, 100, 120, 140, 180, 200, or any value between 20-200 mg/100g product.

The complex of the lactoferrin and osteopontin provided by the present disclosure can synergistically increase serum anti-inflammatory factors IL-4 and IL-10 and reduce pro-inflammatory factors IL-1β; enhance lymphocytes of intestinal epithelial tissue, inhibit activated CD4⁺ cells and neutrophils, enhance intestinal immunity; protect intestinal permeability, promote secretion of intestinal mucin and maintain intestinal mucosal barrier.

The present disclosure provides use of a protein complex in the manufacture of a formula food with resisting enteritis, wherein the protein complex is selected from a protein complex formed by lactoferrin and osteopontin via electrostatic interaction in a mass ratio of 1:10 to 1:0.05.

The present disclosure also provides use of a protein complex in the manufacture of a formula food with the function of protecting the integrity of the intestinal barrier, wherein the protein complex is selected from a protein complex formed by lactoferrin and osteopontin via electrostatic interaction in a mass ratio of 1:10 to 1:0.05.

Among them, lactoferrin (LF) is an iron-binding lactose protein. LF is beneficial to intestinal health in the following aspects, including (a) enhancing the permeability of intestinal epithelial monolayer cells; (b) facilitating microbial antagonism, preventing the colonization and proliferation of intestinal pathogens; (c) enhancing the growth and maturation of intestinal monolayer cell components and intestinal nerve fibers; (d) balancing anti-inflammatory and pro-inflammatory responses to maintain intestinal homeostasis. Studies have shown that insufficient levels of osteopontin (OPN) in infant formula milk powder may partially explain the developmental differences between infants fed with formula milk powder or breast milk. In a premature pig model experiment, the OPN-fed group had a higher ratio of villi to crypts in the small intestine tissue, and higher monocyte and lymphocyte counts than the control group, and could cause slight improvements in intestinal structure and systemic immunity. In the present disclosure, the lactoferrin and osteopontin bind to each other through electrostatic interaction, thereby achieving a synergistic effect of resisting enteritis and/or protecting the integrity of the intestinal barrier.

The formula food is a formula milk powder, formula food for special medical purpose or healthcare food; preferably, the formula food is preferably infant formula milk powder.

In the above use, the mass ratio of the lactoferrin to osteopontin is preferably 1:5-1:0.1, and more preferably 1:5, 1:4, 1:3, 1:2, 1:1, 1:0.5, 1:0.2, 1:0.1, or any value between 1:5-1:0.1.

The content of lactoferrin in the formula food is 1-750 mg/100g, preferably 1, 5, 10, 50, 100, 200, 300, 400, 500, 600, 700, 750, or any value between 1-750 mg/100g, and the content of osteopontin in the formula food is 20-500 mg/100g, preferably 20, 40, 60, 80, 100, 200, 300, 400, 500, or any value between 20-500 mg/100g.

The sources of lactoferrin and osteopontin include cow milk, goat milk and their products, including but not limited to one or more of whole milk powder, skimmed milk powder, casein and whey protein powder.

The enteritis includes *Escherichia coli* enteritis. In some specific embodiments of the present disclosure, the resisting enteritis includes reducing the number of serum cytokines IL-1β, IL-4, and IL-10, reducing the number of neutrophils in intestinal tissues, and reducing the number of CD4⁺T cells in intestinal tissues.

The protecting integrity of intestinal barrier includes protecting against intestinal tissue damage, protecting mucus barrier, and protecting integrity of intestinal mucosal mechanical barrier.

In the formula food for resisting enteritis and/or protecting integrity of intestinal barrier provided by the present disclosure, the total protein content is 10-20g/100g, the proportion of whey protein in the total protein is 40-70%; the fat content is 15-30g/100g; the dietary fiber content is 0.95-6.3g/100g.

According to a specific embodiment of the present disclosure, the total protein content in the formula food of the present disclosure is 10-20g/100g, preferably 10, 12, 14, 16, 18, 20, or any value between 10-20g/100g. The raw materials providing total protein include: raw milk (cow/sheep), whole milk powder (cow/sheep), skimmed milk powder (cow/sheep), whey protein powder (cow/sheep), desalted whey powder (cow/sheep), and one or more of β-casein.

According to a specific embodiment of the present disclosure, the fat content in the formula food of the present disclosure is 15-30g/100g, preferably 15, 20, 25, 30, or any value between 15-30g/100g. The raw materials providing fat include vegetable oil or OPO structured fat in addition to the basic raw materials containing milk fat. Preferably, the linoleic acid content in the formula food is 2700-4500mg/100g, and the α-linolenic acid content is 270-450mg/100g.

According to a specific embodiment of the present disclosure, in the formula food comprising the complex of the present disclosure, the dietary fiber content is 0.95-6.3g/100g, preferably 0.95, 1.0, 1.5, 2.0, 2.5, 3.0, 3.5, 4.0, 4.5, 5.0, 5.5, 6.0, 6.3, or any value between 0.95-6.3g/100g. In the present disclosure, the dietary fiber includes one or more of galacto-oligosaccharides, fructo-oligosaccharides, and breast milk oligosaccharides.

According to a specific embodiment of the present disclosure, in the formula food comprisintg the complex of the present disclosure, part of the carbohydrates come from basic raw materials containing lactose such as milk, whole milk powder and/or skimmed milk powder, and raw material lactose should be additionally added to provide carbohydrates. That is, in the formula food of the present disclosure, the raw materials for providing carbohydrates include raw material lactose in addition to the basic raw materials containing lactose. Preferably, based on 1000 parts by weight of the formula food containing the complex, the raw materials comprise: 125 to 325 parts by weight of lactose, preferably 125, 150, 175, 200, 225, 250, 275, 300, 325, or any value between 125 and 325 parts by weight. The specific amount of lactose added can be adjusted within the range so that the carbohydrate content of the formula food containing the complex of the present disclosure is 50 to 57 g/100 g, preferably 50, 51, 52, 53, 54, 55, 56, 57, or any value between 50 and 57 g/100 g.

In some specific embodiments of the present disclosure, the nutritional components may also include minerals, vitamins, probiotics and prebiotics.

According to a specific embodiment of the present disclosure, in the formula food containing the complex provided by the present disclosure, the probiotic is bifidobacterium. Preferably, based on 1000 parts by weight of the formula food containing the complex, the amount of bifidobacterium added is 0 to 0.2 parts by weight, preferably 0, 0.05, 0.1, 0.15, 0.2, or any value between 0 and 0.2 parts by weight, and further preferably any value between 0.18 and 0.2 parts by weight. More preferably, each part by weight of bifidobacterium powder contains more than 3×10¹⁰ CFU of bifidobacterium.

According to a specific embodiment of the present disclosure, the raw materials of the formula food of the present disclosure may also include one or more combinations of appropriate DHA, ARA, nucleotides, and lactoferrin. Preferably, based on 1000 parts by weight of the formula food of the present disclosure, its raw materials include: 0-20 parts by weight of DHA, preferably 8-17 parts by weight; 0-25 parts by weight of ARA, preferably 10-20 parts by weight; 0-8.5 parts by weight of lactoferrin, preferably 2-6 parts by weight; 0-1 parts by weight of nucleotides, preferably 0.3-0.7 parts by weight. Wherein, the lactoferrin is lactoferrin added additionally in the dry process when the amount of lactoferrin contained in the semi-finished product produced by the wet process is insufficient, and is added in the dry mixing step of step 16) in the following preparation method.

According to a specific embodiment of the present disclosure, the raw materials of the formula food of the present disclosure may also include compound nutrients comprising calcium powder, vitamins and minerals. The compound nutrients are a combination of nutrients that meet national standards, and different addition amounts are used according to different formulas. Preferably, based on 1000 parts by weight of the formula food, the raw materials comprise: 7 to 25 parts by weight of compound nutrients comprising calcium powder, vitamins and minerals, preferably 7, 10, 12, 15, 18, 20, 22, 25, or any value between 7 and 25 parts by weight. More preferably, the compound nutrients are added in the form of at least a compound vitamin package, calcium powder, and a mineral nutrient package. Specifically, the compound nutrients are preferably added in the form of the following nutrient packages:
1) Compound vitamin nutrient package, the content of each component in each gram of the compound vitamin nutrient package is as follows:
   Taurine: 140-340mg
   Vitamin A: 1700-5800µgRE
   Vitamin D: 25-70µg
   Vitamin B1: 3000-6800µg
   Vitamin B2: 3500-6900µg
   Vitamin B6: 2400-4000µg
   Vitamin B12: 8-20µg
   Vitamin K1: 200-700µg
   Vitamin C: 155-700mg
   Vitamin E: 10-70mg α-TE
   Niacinamide: 10000-41550µg
   Folic acid: 500-920µg
   Biotin: 100-245µg
   Pantothenic acid: 7100-25230µg
   Inositol: 0-250mg
   L-carnitine: 0-60mg
2) Mineral II nutrient package, the content of each component in each gram of the mineral II nutrient package is as follows:
   Calcium: 120-455mg
   Phosphorus: 0-150mg
3) Mineral I nutrient package, the content of each component in each gram of the mineral I nutrient package is as follows:
   Iron: 40 -110mg
   Zinc: 23-90mg
   Copper: 2600-4180µg
   Iodine: 500-995µg
   Selenium: 0-200µg
   Manganese: 0-579µg
4) Compound magnesium chloride nutrient package, each gram of compound magnesium chloride nutrient package contains:
   Magnesium: 80-170mg
5) Compound potassium chloride nutrient package, each gram of compound potassium chloride nutrient package contains:
   Potassium: 400-580mg;
6) Choline chloride nutrient package, each gram of choline chloride nutrient package contains:
   Choline: 300-950mg.

Preferably, based on 1000 parts by weight of the formula food, the addition amount of the compound vitamin nutrient package is 2 to 4 parts by weight, preferably 2, 2.5, 3, 3.5, 4, or any value between 2 to 4 parts by weight. The addition amount of the mineral II nutrient package is 2 to 12 parts by weight, preferably 2, 4, 6, 8, 10, 12, or any value between 2 to 12 parts by weight. The addition amount of the mineral I nutrient package is 0.5 to 3 parts by weight, preferably 0.5, 1.0, 1.5, 2.0, 2.5, 3.0, or any value between 0.5 to 3 parts by weight, and the addition amount of the compound magnesium chloride nutrient package is 0 to 2 parts by weight, preferably 0, 0.5, 1.0, 1.5, 2.0, or any value between 0 to 2 parts by weight. The amount of the compound potassium chloride nutrient package added is 0-4.5 parts by weight, preferably 0, 0.5, 1.0, 1.5, 2.0, 2.5, 3.0, 3.5, 4.0, 4.5, or any value between 0-4.5 parts by weight. The amount of choline chloride added is 0-2 parts by weight, preferably 0, 0.5, 1.0, 1.5, 2.0, or any value between 0-2 parts by weight. The base material of each nutrient package is preferably lactose or sodium L-ascorbate.

In the formula food containing a complex provided by the present disclosure, the raw materials phospholipids and/or anhydrous milk fat are mainly used for the molding of powder particles during spray drying, and the phospholipids can be soybean phospholipid and/or lecithin. The amount of phospholipids and/or anhydrous milk fat is small, but it also contributes to the fat content in milk powder products.

In some specific embodiments of the present disclosure, the formula food provided by the present disclosure is an infant formula milk powder for resisting enteritis and/or protecting integrity of intestinal barrier, comprising the following raw materials in parts by weight:
Raw milk 0-2000 parts by weight;
Lactose 125-350 parts by weight;
Whole milk powder 0-300 parts by weight;
Skimmed milk powder 0-325 parts by weight;
Desalted whey powder 0-375 parts by weight;
Whey protein powder 0-170 parts by weight;
Sunflower oil 0-80 parts by weight;
Corn oil 0-40 parts by weight;
Soybean oil 0-80 parts by weight;
Coconut oil 0-30 parts by weight;
Low-erucic acid rapeseed oil 0-85 parts by weight;
Flaxseed oil 0-20 parts by weight;
1,3-dioleyl-2-palmitoyl triglyceride 0-240 parts by weight;
α-lactalbumin powder 0-55 parts by weight;
β-casein powder 0-40 parts by weight;
Soybean phospholipid 0-5 parts by weight;
Anhydrous milk fat 0-2 parts by weight;
Fructo-oligosaccharide powder 0-25 parts by weight;
Galacto-oligosaccharide syrup 0-100 parts by weight;
Compound nutrients comprising calcium powder, vitamins and minerals 7-25 parts by weight;
DHA 0-20 parts by weight;
ARA 0-25 parts by weight;
Bifidobacterium 0-0.2 parts by weight;
Lactoferrin 0-8.5 parts by weight;
Nucleotide 0-1 parts by weight.

Among them, the amount of three raw materials of raw milk, whole milk powder and skimmed milk powder are not all 0; the amount of seven raw materials of sunflower oil, corn oil, soybean oil, coconut oil, low-erucic acid rapeseed oil, flaxseed oil, and 1,3-dioleyl-2-palmitoyl triglyceride are not all 0.

In the present disclosure, lactoferrin and osteopontin are derived from the lactoferrin and osteopontin contained in the raw materials in the above formula, and when the lactoferrin content contained in the raw materials is insufficient, lactoferrin can be additionally added as a formula raw material.

In the infant formula milk powder containing a complex of the present disclosure, the specific amount of each raw material should be adjusted and determined on the premise of meeting the requirements of product indicator of the formula milk powder. In the infant formula milk powder containing a complex of the present disclosure, the product performance indicators that are not described or listed in detail should be implemented in accordance with the provisions of the national standards for infant formula foods and the relevant standards and regulations.

In the infant formula milk powder containing a complex of the present disclosure, each raw material is commercially available, and the selection of each raw material should meet the requirements of relevant standards, and the complex should also meet the requirements of the present disclosure. In addition, the compound nutrients can also be compounded in house. The term "compound" is used in the present disclosure only for the convenience of expression, and does not mean that the components in the compound must be mixed together before application. All raw materials should be added and used under the premise of meeting relevant regulations.

In the present disclosure, the product is a food, a healthcare food or a nutrient supplement.

The product includes a dairy product. The dairy product includes milk powder, liquid milk, cheese, condensed milk, milk fat or other dairy products.

On the other hand, the present disclosure also provides a method of preparing the infant formula milk powder containing the complex of lactoferrin and osteopontin. The process flow of the preparation mainly comprises: material formulation, homogenization, concentration and sterilization, spray drying, and dry mixing to obtain the finished product. Specifically, the method of preparing the infant formula milk powder containing the complex of lactoferrin and osteopontin of the present disclosure comprises:
1) Coarse filtration of milk: after the milk is coarsely filtered and degassed in the balance cylinder, it is preheated by a plate heat exchanger and separated from impurities by a separator.
2) Homogenization and sterilization of milk: after removing impurities, part of the raw milk enters the homogenizer for homogenization, and the other part is not homogenized. After the homogenization, the two parts are mixed and enter the sterilization system for sterilization.
3) Addition of powder: various powder raw materials are added to a powder mixing tank for storage through an air delivery system after being measured according to the formula.
4) Vacuum suction of powder: various powder raw materials in the powder mixing tank are sucked into a vacuum mixing tank through a vacuum system.
5) Melting oil: according to the formula requirements, the oil specified in the formula is placed in the oil melting room. The temperature of the oil melting room should be maintained at 50-90°C. After the oil is melted, it is pumped into the storage tank of mixed oil through the oil pump and flow meter according to the formula ratio requirements.
6) Storage of mixed oil: the mixed oil is stored in the oil storage tank at a temperature of 40-50°C, and the storage time is less than 12 hours to prevent oxidation of fat.
7) Weighing: according to the formula requirements, the mixed oil is pumped into the mixing tank through the oil pump.
8) Dissolution and addition of nutrient: calcium powder, minerals and vitamins are added separately, dissolved with 100-200kg of pure water separately, and then pumped into the wet mixing tank. After each one is pumped, 100kg of pure water is used to rinse the addition tank and pipeline.
9) Filtration: the mixed liquid is filtered through a filter to remove physical impurities that may be brought into the raw materials.
10) Homogenization: the mixed liquid is homogenized by a homogenizer, with a first-level homogenization pressure of 105±5bar, and a second-level homogenization pressure of 32±3bar. The fat globules are mechanically treated to disperse into uniform fat globules.
11) Cooling and storage: the homogenized liquid enters the plate heat exchanger for cooling: it is cooled to below 20°C and temporarily stored in a pre-storage tank, and then enters the next process within 6 hours, and an agitator is turned on according to the set requirements.
12) Concentration and sterilization: double-effect concentration is used during production, the sterilization temperature is ≥83°C, and the sterilization time is 25 seconds. The discharge concentration is 48%-52% dry matter.
13) Storage of concentrated milk, preheating, filtration, and spray drying: the concentrated milk is temporarily stored in the concentrated milk balance tank. It is preheated to 60-70°C by the scraper preheater. After preheating, the material is filtered through a filter with a pore size of 1mm, and then pumped by a high-pressure pump into a drying tower for spray drying. The fine powder is agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
14) Fluidized bed drying and cooling: the powder coming out of the drying tower is dried twice in a fluidized bed (first level) and cooled to 25-30°C in a fluidized bed (secondary level). At the same time, the phospholipids are mixed with the carrier and heated to 60-65°C, and evenly dispersed on the surface of the powder under the action of compressed air, so that the powder particles agglomerate to increase their particle size and solubility.
15) Packaging: the staff of the powder making workshop weighs and seals DHA, ARA, lactoferrin and bifidobacteria according to the formula.
16) Dry mixing: the weighed DHA, ARA, lactoferrin, bifidobacteria and milk powder are mixed in a dry mixer.
17) Powder screening: a vibrating screen is used to make the particle size of the milk powder uniform, and the powder residue is discarded.
18) Powder discharging: a sterilized powder collection box is used to collect the powder and transport it from a powder discharging room to a powder loading room.
19) Powder loading: the milk powder is poured into a powder storage tank on large and small packaging machines according to the packaging requirements.
20) Packaging: 800g of the milk powder is packaged with nitrogen flushed by automatic packaging machine, and the oxygen content is less than 1% when nitrogen is flushed. 900g of milk powder is packaged by iron cans automatically with nitrogen flushed, and the oxygen content is less than 5%.
21) Packing: the packaged small bags are put into a carton and a powder spoon is added at the same time, and the carton is sealed with a carton sealing machine.
22) Inspection of finished products: sampling inspection is carried out on packaged products according to the inspection plan.
23) Storage: products that have passed the inspection are stored in a warehouse, and are required to be stored at room temperature with a humidity of ≤65%.

The present disclosure provides use of a protein complex in the manufacture of a formula food for resisting enteritis and/or protecting integrity of intestinal barrier. The formula food of the present disclosure can enhance human intestinal immunity, protect intestinal permeability, promote intestinal mucin secretion, maintain intestinal mucosal barrier, and thus protect a healthy intestine.

The present disclosure provides use of a nutrient component in the manufacture of a formula food for special medical purpose for resisting enteritis and/or protecting integrity of intestinal barrier. The nutrient component is selected from a protein complex formed by lactoferrin and osteopontin via electrostatic interaction in a mass ratio of 1:10 to 1:0.1.

Lactoferrin (LF) is an iron-binding lactose protein. LF is beneficial to intestinal health in the following aspects, including (a) enhancing the permeability of intestinal epithelial monolayer cells; (b) facilitating microbial antagonism, preventing the colonization and proliferation of intestinal pathogens; (c) enhancing the growth and maturation of intestinal monolayer cell components and intestinal nerve fibers; (d) balancing anti-inflammatory and pro-inflammatory reactions to maintain intestinal homeostasis. Studies have shown that insufficient levels of osteopontin (OPN) in infant formula milk powder may partially explain the developmental differences between infants fed with formula milk powder or breast milk. In a premature pig model experiment, the OPN-fed group had a higher ratio of villi to crypts in the small intestine tissue, and higher monocyte and lymphocyte counts than the control group, and could cause slight improvements in intestinal structure and systemic immunity. In the present disclosure, the lactoferrin and osteopontin bind to each other through electrostatic interaction, thereby achieving a synergistic effect in resisting enteritis and/or protection of integrity of intestinal barrier.

The mass ratio of lactoferrin to osteopontin is preferably 1:5-1:0.1, and further preferably 1:5, 1:4, 1:3, 1:2, 1:1, 1:0.5, 1:0.2, 1:0.1, or any value between 1:5-1:0.1.

The content of lactoferrin in the formula food is 1-750 mg/100g, preferably 1, 5, 10, 50, 100, 200, 300, 400, 500, 600, 700, 750, or any value between 1-750 mg/100g.

The content of osteopontin in the formula food is 20-600 mg/100g, preferably 20, 40, 60, 80, 100, 200, 300, 400, 500, 600, or any value between 20-600 mg/100g.

The enteritis includes *Escherichia coli* enteritis. In some specific embodiments of the present disclosure, the resisting enteritis includes reducing the number of serum cytokines IL-1β, IL-4, IL-10, reducing the number of neutrophils in intestinal tissue and reducing the number of CD4⁺ T cells in intestinal tissue.

The protecting integrity of intestinal barrier includes protecting intestinal tissue from damage, protecting mucus barrier and protecting integrity of intestinal mucosal mechanical barrier.

In the present disclosure, the resisting enteritis and/or protection of integrity of intestinal barrier include preventing enteritis in premature/low birth weight infants and/or protecting the integrity of intestinal barrier of premature/low birth weight infants, preventing enteritis in lactose-intolerant infants and/or protecting the integrity of intestinal barrier of lactose-intolerant infants, preventing enteritis in patients with tumor and/or protecting the integrity of intestinal barrier of patients with tumor, preventing enteritis in patients with traumatic stress and/or protecting the integrity of intestinal barrier of patients with traumatic stress.

In the present disclosure, the formula food for special medical purpose includes a dairy product, and the dairy product includes milk powder or liquid milk.

The content of lactoferrin in the formula food for special medical purpose is 1-750 mg/100g, preferably 1, 5, 10, 50, 100, 200, 300, 400, 500, 600, 700, 750, or any value between 1-750 mg/100g.

The content of osteopontin in the formula food is 20-600 mg/100g, preferably 20, 40, 60, 80, 100, 200, 300, 400, 500, 600, or any value between 20-600 mg/100g.

The formula food for special medical purpose of the present disclosure also comprises fat. The raw material for providing fat also includes vegetable oil in addition to the basic raw material containing milk fat. The vegetable oil may include but is not limited to one or more selected from the group consisting of sunflower oil, corn oil, soybean oil, low-erucic acid rapeseed oil, coconut oil, palm oil, and walnut oil, preferably including sunflower oil, corn oil and soybean oil. The addition of these vegetable oils provides fat components for the product on the one hand, and linoleic acid on the other hand, and can also provide α-linolenic acid. In addition, the raw material for providing fat can also selectively include raw material OPO structured lipid added to provide 1,3-dioleyl-2-palmitoyl triglyceride. Since the purity of the OPO structured fat raw materials currently sold on the market is different, that is, the content of the active ingredient 1,3-dioleyl-2-palmityl triglyceride is different, usually around 40%-70%, in the present disclosure, in order to distinguish the active ingredient 1,3-dioleyl-2-palmityl triglyceride and its raw materials, the term "1,3-dioleyl-2-palmityl triglyceride" is used when describing the active ingredient, and the commonly known "OPO structured fat" is used when describing the food raw materials providing the active ingredient 1,3-dioleyl-2-palmityl triglyceride. The specific addition amount of OPO structured fat can be converted according to the content requirements of 1,3-dioleyl-2-palmityl triglyceride and the purity of the OPO structured fat raw material in the product of the present disclosure. More preferably, based on 1000 parts by weight of the formula food for special medical purpose, the raw materials include but are not limited to: 0-150 parts by weight of sunflower oil; 0-40 parts by weight of corn oil; 0-80 parts by weight of soybean oil; and 0-140 parts by weight of OPO structured fat.

The formula food for special medical purpose of the present disclosure also includes carbohydrates, wherein a part of the carbohydrates is derived from lactose, a part is derived from non-lactose source materials, including but not limited to pregelatinized starch, maltodextrin, solid corn syrup, glucose syrup, and a part is derived from modified starch emulsifiers. That is, in the formula food of the present disclosure, the raw materials for providing carbohydrates include but are not limited to raw material lactose, emulsifiers and pre-hydrolyzed or gelatinized starch materials in addition to the basic raw materials containing lactose. Preferably, based on 1000 parts by weight of the formula food for special medical purpose, the raw materials include but are not limited to: 0-580 parts by weight of lactose, 0-580 parts by weight of non-lactose materials. The specific addition amount of lactose can be adjusted within the range so that the carbohydrate content of the present disclosure is 50-58g/100g.

The formula food for special medical purpose of the present disclosure also includes but is not limited to one or more of appropriate DHA, ARA, EPA, nucleotides, arginine, tryptophan, lactoferrin, etc., and also includes compound nutrients comprising calcium powder, vitamins and minerals. Preferably, based on 1000 parts by weight of the formula food for special medical purpose, the raw materials include: 8 to 50 parts by weight of DHA, 14 to 28 parts by weight of ARA, 10 to 140 parts by weight of EPA, 0 to 10 parts by weight of arginine, 0 to 10 parts by weight of tryptophan, 0 to 7.5 parts by weight of lactoferrin, 0 to 75 parts by weight of osteopontin, and 7 to 50 parts by weight of compound nutrients comprising calcium powder, vitamins and minerals.

Among them, the compound nutrients are a combination of nutrients that meet national standards, and different addition amounts are used according to different formulas. The formula food of the present disclosure can selectively adopt any one or any combination of the following compound nutrient ingredients if nutrients are added as needed. Preferably, the compound nutrients include at least compound vitamins, calcium powder, and mineral nutrient packages, and the amount used of each component is as follows:
1) Compound vitamins, each gram of compound vitamins comprises:
   Taurine: 140-340 mg
   Vitamin A: 1700-5800 µgRE
   Vitamin D: 25-70 µg
   Vitamin B1: 2000-6800 µg
   Vitamin B2: 3000-6900 µg
   Vitamin B6: 1700-4000 µg
   Vitamin B12: 8-20 µg
   Vitamin K1: 200-700 µg
   Vitamin C: 0-700 mg
   Vitamin E: 10-70 mg α-TE
   Niacinamide: 10000-41550 µg
   Folic acid: 350-920 µg
   Biotin: 70-245 ng
   Pantothenic acid: 7100-25230 µg
   Inositol: 0-250 mg
   L-carnitine: 0-60 mg
2) Mineral II, each gram of mineral II comprises:
   Sodium: 40-100 mg
   Potassium: 200-500 mg
2) Mineral III, each gram of mineral III comprises:
   Calcium: 200-500 mg
   Phosphorus: 75-300 mg
3) Mineral I, each gram of mineral I comprises:
   Iron: 20-110mg
   Zinc: 23-90mg
   Copper: 2000-4180 µg
   Iodine: 500-995 µg
   Selenium: 0-200 µg
   Manganese: 0-579 µg
4) Compound magnesium chloride package, each gram of magnesium chloride package comprises:
   Magnesium: 80-170mg
5) Choline chloride package, each gram of choline chloride package comprises:
   Choline: 300-950 mg

The base material of the above compound nutrients is preferably lactose, solid corn syrup or sodium L-ascorbate. Based on 1000 parts by weight of the formula food for special medical purpose, the added amount of compound nutrients is 7 to 52 parts by weight, wherein the compound nutrients include: preferably 2 to 4 parts by weight of compound vitamin nutrient package, preferably 2 to 20 parts by weight of mineral II nutrient package, preferably 0.5 to 20 parts by weight of mineral III nutrient package, preferably 0.5 to 6 parts by weight of mineral I nutrient package, 0 to 7 parts by weight of magnesium chloride, and 0 to 3 parts by weight of choline chloride, and the base material of each nutrient package is preferably lactose or sodium L-ascorbate.

The compound raw materials used for each nutrient provided in the nutrient package may interact with each other. For example, sulfate can accelerate the oxidation and destruction process of vitamins, reducing their utilization rate. Since sulfate appears in the form of ions in aqueous solution, it acts as an oxidant in the oxidation reaction to induce the oxidation of vitamins and destroy the structure of vitamins. Trace elements have different abilities in redox reactions, with copper, zinc and iron being the most active, followed by manganese and selenium. B vitamins and vitamin C are easily affected by copper ions, and vitamin B2 is easily affected by iron ions.

**In** order to ensure the utilization efficiency of nutrients, the present disclosure selects a stable nutrient dosage form, for example: retinyl acetate is used as vitamin A. Retinol contains 1 hydroxyl group and 5 double bonds, which is very easy to oxidization, but the stability of retinol in the form of acetate will be greatly improved. Tocopherol acetate is used as vitamin E. Tocopherol is unstable, but tocopherol acetate has higher stability. Thiamine nitrate is used as vitamin B₁, which is more stable than thiamine hydrochloride among the existing forms of thiamine. Sodium L-ascorbate is used as vitamin C.

The content of each component of the above-mentioned compound nutrients refers to the addition amount to strengthen the nutrient substance, and does not include the nutrient component content of other raw materials in the formula food for special medical purpose.

In the present disclosure, the formula food for special medical purpose includes a dairy product, and the dairy product includes milk powder or liquid milk.

In some specific embodiments of the present disclosure, the formula food for special medical purpose is a formula food for premature/low birth weight infant, and the raw materials comprise:
Whey protein powder 80-180 weight parts;
Lactose 0-580 weight parts;
Solid corn syrup 0-580 weight parts;
Sunflower oil 0-150 weight parts;
Corn oil 0-40 weight parts;
Soybean oil 20-80 weight parts;
OPO structured fat 0-140 weight parts;
Lactoferrin 0-7.5 weight parts;
Osteopontin 0-75 weight parts;
Compound nutrients comprising calcium powder, vitamins and minerals 7-50 weight parts;
DHA 2-15 weight parts;
ARA 3-22 weight parts;
Nucleotide 0.1-5 weight parts;
Bifidobacterium 0.1-0.2 weight parts.

In the formula food for premature/low birth weight infants provided by the present disclosure, the specific amount of each raw material should be adjusted and determined on the premise of meeting the requirements for the product indicator of the formula food. In the formula food for premature/low birth weight infants of the present disclosure, the product performance indicators that are not described or listed in detail should be implemented in accordance with the national standards for infant formula food or modulated milk powder and the provisions of relevant standards and regulations.

In the formula food for special medical purpose provided by the present disclosure, each raw material is commercially available, and the selection of each raw material should meet the requirements of relevant standards. In addition, the compound nutrients can also be compounded in house. The present disclosure only uses "compound" for the convenience of expression, and it does not mean that the components in the compound must be mixed together before application. All raw materials should be added and used under the premise of meeting relevant regulations.

The present disclosure also provides a method of preparing the formula food for premature/low birth weight infants, and the process flow of the preparation mainly comprises: material formulation, homogenization, concentration and sterilization, spray drying, and dry mixing to obtain the finished product. Specifically, the method of preparing a formula food for special medical purpose containing nutritional components that can protect the integrity of the intestinal mucosal barrier of the present disclosure comprises:
1) Addition of powder: various powder raw materials are added to a powder mixing tank through an air delivery system after being measured according to the formula for storage.
2) Vacuum suction of powder: various powder raw materials in the powder mixing tank are sucked into a vacuum mixing tank through a vacuum system.
3) Melting oil: the oil specified in the formula is put into an oil melting room according to the formula requirements, and the temperature of the oil melting room should be maintained at 50-90°C. After the oil is melted, it is pumped into the storage tank of mixed oil through an oil pump and a flow meter according to the formula ratio requirements.
4) Storage of mixed oil: the mixed oil is stored in an oil storage tank at a temperature of 40-50°C, and the storage time is less than 12 hours to prevent fat oxidation.
5) Weighing: the mixed oil is pumped into the mixing tank through the oil pump according to the formula requirements.
6) Dissolution and addition of nutrient: calcium powder, minerals, and vitamins are added separately, and dissolved with 100-200kg of pure water separately, and then they are pumped into the wet mixing tank. After each one is pumped, 100kg of pure water is used to rinse the addition tank and pipeline.
7) Filtration: the mixed liquid is filtered through a filter to remove physical impurities that may be brought into the raw materials.
8) Homogenization: the mixed liquid is homogenized by a homogenizer, with a first-level homogenization pressure of 105±5 bar and a second-level pressure of 32±3 bar. The fat globules are mechanically treated to disperse into uniform fat globules.
9) Cooling and storage: the homogenized liquid enters a plate heat exchanger for cooling: it is cooled to below 20°C and temporarily stored in a pre-storage tank. It enters the next process within 6 hours, and an agitator is turned on according to the set requirements.
10) Concentration and sterilization: double-effect concentration is used during production, the sterilization temperature is ≥83°C, and the sterilization time is 25 seconds. The discharge concentration is 48%-52% dry matter.
11) Storage of liquid, preheating, filtration, and spray drying: the concentrated liquid is temporarily stored in a liquid balance tank. It is preheated to 60-70°C by a scraper preheater. After preheating, the material is filtered through a filter with a pore size of 1mm, and then pumped by a high-pressure pump into a drying tower for spray drying. The fine powder is agglomerated at the top of the tower or in the fluidized bed as required. Inlet air temperature: 165-180°C, exhaust air temperature 75-90°C, high-pressure pump pressure 160-210 bar, tower negative pressure -4 to -2 mbar.
12) Fluidized bed drying and cooling: the powder coming out of the drying tower is dried twice in a fluidized bed (first level) and cooled to 25-30°C in a fluidized bed (second level).
13) Packaging: the staff of the powder making workshop weighs and seals the DHA, ARA, osteopontin, lactoferrin, and bifidobacteria according to the formula requirements.
14) Dry mixing: the weighed DHA, ARA, osteopontin, lactoferrin, bifidobacteria and powdered materials are mixed in a dry mixer. If the protein content is high, some casein, whey protein powder and sodium caseinate can be mixed and added evenly by dry mixing to obtain powdered formula food.
15) Powder screening: the particle size of the powdered formula food is uniformed by vibrating screen, and the powder residue is discarded.
16) Powder discharging: the powder is collected in a sterilized powder collecting box and transported from a powder discharging room to a powder loading room.
17) Powder loading: the powdered formula food is loaded into a powder storage tank of large and small packaging machines according to the packaging requirements.
18) Packaging: 800g of the milk powder is packaged with nitrogen flushed by automatic packaging machine, And the oxygen content is less than 1% when nitrogen is flushed. 900g of milk powder is packaged by iron cans automatically with nitrogen flushed, and the oxygen content is less than 5%.
19) Packing: the packaged small bags are put into a carton and a powder spoon is added at the same time, and the carton is sedaled with a carton sealing machine.
20) Inspection of finished products: sampling inspection is carried out on packaged products according to the inspection plan.
21) Storage: products that have passed the inspection are stored in a warehouse, and are required to be stored at room temperature with a humidity of ≤65%.

In some specific embodiments of the present disclosure, the formula food for special medical purpose is a formula food for lactose-intolerant infant, and the raw materials comprise:
Isolated whey protein powder 90-150 weight parts;
Lactose-free whole milk powder 0-500 weight parts;
Solid corn syrup 0-600 weight parts;
Sunflower oil 0-150 weight parts;
Corn oil 0-40 weight parts;
Soybean oil 20-80 weight parts;
OPO structured fat 0-140 weight parts;
Lactoferrin 0-7.5 weight parts;
Osteopontin 0-75 weight parts;
Compound nutrients comprising calcium powder, vitamins and minerals 7-50 weight parts;
DHA 2-15 weight parts;
ARA 3-22 weight parts; and
Nucleotides 0.1-5 weight parts.

The formula food for lactose-intolerant infant provided by the present disclosure is a lactose-free formula food, and the specific amount of each raw material should be adjusted and determined on the premise of meeting the product indicator requirements of the formula food. In the lactose-free formula food of the present disclosure, the product performance indicators that are not described or listed in detail shall be implemented in accordance with the national standards for infant formula food or modulated milk powder and the provisions of relevant standards and regulations.

In the lactose-free formula food of the present disclosure, all raw materials can be commercially available, and the selection of each raw material should meet the requirements of relevant standards. In addition, the compound nutrients can also be compounded in house. The term "compound" is used in the present disclosure only for the convenience of expression, and does not mean that the components in the compound must be mixed together before use. All raw materials should be added and used under the premise of meeting relevant regulations.

On the other hand, the present disclosure also provides a preparation method of the above-mentioned lactose-intolerant infant formula food, and the process flow of the preparation mainly comprises: material formulation, homogenization, concentration and sterilization, spray drying, and dry mixing to obtain the finished product. Specifically, the method of preparing the low-allergy lactose-free formula food of the present disclosure comprises: preparing the lactose-free formula food by a wet or dry production process.

Specifically, it comprises the following steps:
1) Addition of powder: various powder raw materials are added to a powder mixing tank for storage through an air delivery system after being measured according to the formula.
2) Vacuum suction of powder: various powder raw materials in the powder mixing tank are sucked into a vacuum mixing tank through a vacuum system.
3) Melting oil: the oil specified in the formula is put into an oil melting room according to the formula requirements. The temperature of the oil melting room should be maintained at 50-90°C. After the oil is melted, it is pumped into a storage tank of mixed oil through an oil pump and a flow meter according to the formula ratio requirements.
4) Storage of mixed oil: the mixed oil is stored in an oil storage tank at a temperature of 40-50°C. The storage time is less than 12 hours to prevent fat oxidation.
5) Weighing: the mixed oil is pumped into a mixing tank through an oil pump according to the formula requirements.
6) Dissolution and addition of nutrients: calcium powder, minerals, and vitamins are added separately, dissolved with100-200kg of pure water separately, and pumped into a wet mixing tank. After each one is pumped, 100kg of pure water is used to rinse an addition tank and pipeline.
7) Filtration: the mixed liquid is filtered through a filter to remove physical impurities that may be brought into the raw materials.
8) Homogenization: the mixed liquid is homogenized by a homogenizer with a first-level homogenization pressure of 105±5 bar, and a second-level pressure of 32±3 bar. The fat globules are mechanically treated to disperse into uniform fat globules.
9) Cooling and storage: the homogenized liquid enters a plate heat exchanger for cooling: it is cooled to below 20°C, temporarily stored in a pre-storage tank, and enters the next process within 6 hours. An agitator is turned on according to the set requirements.
10) Concentration and sterilization: double-effect concentration is used during production, the sterilization temperature is ≥83°C, and the sterilization time is 25 seconds. The discharge concentration is 48%-52% dry matter.
11) Liquid storage, preheating, filtration, and spray drying: the concentrated liquid is temporarily stored in a liquid balance tank. It is preheated to 60-70°C by a scraper preheater. After preheating, the material is filtered through a filter with a pore size of 1mm, and then pumped by a high-pressure pump into a drying tower for spray drying. The fine powder is agglomerated at the top of the tower or in the fluidized bed as required. Inlet air temperature: 165-180°C, exhaust air temperature 75-90°C, high pressure pump pressure 160-210 bar, tower negative pressure -4 to -2 mbar.
12) Fluidized bed drying and cooling: the powder coming out of the drying tower is dried in a fluidized bed (first level) for the second time, and then cooled to 25-30°C in a fluidized bed (secondary level).
13) Packaging: the staff of the powder making workshop weighs and seals DHA, ARA, lactoferrin, and 2'-fucosyllactose according to the formula requirements.
14) Dry mixing: mixing the weighed DHA, ARA, lactoferrin, osteopontin and powdered materials in a dry mixer. If the protein content is high, some casein, whey protein powder, and sodium caseinate can be mixed and added evenly by dry mixing to obtain powdered formula food.
15) Powder screening: the particle size of powdered formula food is uniformed by vibrating screen, and the powder residue is discarded.
16) Powder discharging: a sterilized powder collecting box is used to collect the powder and transport it from a powder discharging room to a powder loading room.
17) Powder loading: the powdered formula food is put into a powder storage tank of large and small packaging machines according to the packaging requirements.
18) Packaging: 800g of the milk powder is packaged with nitrogen flushed by automatic packaging machine. The oxygen content is less than 1% when nitrogen is flushed. 900g of milk powder is packaged by iron cans automatically with nitrogen flushed, and the oxygen content is less than 5%.
19) Packing: the packaged small bags are put into a carton and a powder spoon is added at the same time, and the carton is sealed with a carton sealing machine.
20) Inspection of finished products: sampling inspection of packaged products according to the inspection plan is conducted.
21) Storage in warehouse: products that have passed the inspection are stored in a warehouse, and are required to be stored at room temperature with a humidity of ≤65%.

In some specific embodiments of the present disclosure, the formula food for special medical purpose is a formula food suitable for patients with tumor, and the raw materials comprise:
Whey protein powder 90-200 parts by weight;
Sodium caseinate 0-100 parts by weight;
Casein 0-100 parts by weight;
Solid corn syrup 0-400 parts by weight;
Maltodextrin 0-400 parts by weight;
Sunflower oil 0-150 parts by weight;
Corn oil 0-40 parts by weight;
Soybean oil 20-80 parts by weight;
Medium chain triglycerides 0-100 parts by weight;
Fructo-oligosaccharides 0-50 parts by weight;
Lactoferrin 0-7.5 parts by weight;
Osteopontin 0-75 parts by weight;
Compound nutrients comprising calcium powder, vitamins and minerals 7-50 parts by weight;
DHA 5-80 parts by weight;
Tryptophan 0-10 parts by weight;
Arginine 0-10 parts by weight;
Glutamine 0-100 parts by weight; and
EPA 10-140 parts by weight.

In the formula food suitable for patients with tumor provided by the present disclosure, the specific amount of each raw material should be adjusted and determined on the premise of meeting the requirements of the formula food product indicator.

In the formula food suitable for patients with tumor provided by the present disclosure, each raw material can be commercially available, and the selection of each raw material should meet the requirements of relevant standards, wherein lactoferrin and osteopontin should meet the requirements of the present disclosure. In addition, the compound nutrients can also be compounded in house. The present disclosure only uses "compound" for the convenience of expression, and does not mean that the components in the compound must be mixed together before use. All raw materials should be added and used under the premise of meeting relevant regulations.

The present disclosure also provides a method of preparing a formula food suitable for patients with tumor, and the process flow of the preparation mainly comprises: batching, homogenization, concentration and sterilization, spray drying, and dry mixing to obtain a finished product. Specifically, the method of preparing a formula food for patients with tumor containing lactoferrin and osteopontin compounded in a certain proportion of the present disclosure comprises:
1) Addition of powder: various powder raw materials are added to a powder mixing tank through an air delivery system after being measured according to the formula for storage.
2) Vacuum suction of powder: various powder raw materials in the powder mixing tank are sucked into a vacuum mixing tank through a vacuum system.
3) Melting oil: the oil specified in the formula is added into an oil melting room according to the formula requirements, and the temperature of the oil melting room should be maintained at 50-90°C. After the oil is melted, it is pumped into a mixed oil tank through the oil pump and flow meter according to the formula ratio requirements.
4) Storage of mixed oil: the mixed oil is stored in an oil storage tank at a temperature of 40-50°C, and the storage time is less than 12 hours to prevent fat oxidation.
5) Weighing: the mixed oil is pumped into the mixing tank through the oil pump according to the formula requirements.
6) Dissolution and addition of nutrient: calcium powder, minerals, and vitamins are added separately, dissolved with 100-200kg of pure separately, and then pumped into the wet mixing tank. After each one is pumped, 100kg of pure water is used to rinse the addition tank and pipeline.
7) Filtration: the mixed liquid is filtered through a filter to remove physical impurities that may be brought into the raw materials.
8) Homogenization: the mixed liquid is homogenized by a homogenizer with a first-level homogenization pressure of 105±5bar and a second-level pressure of 32±3bar. The fat globules are mechanically treated to disperse into uniform fat globules.
9) Cooling and storage: the homogenized liquid enters a plate heat exchanger for cooling: it is cooled to below 20°C and temporarily stored in a pre-storage tank. It enters the next process within 6 hours, and an agitator is turned on according to the set requirements.
10) Concentration and sterilization: double-effect concentration is used during production, the sterilization temperature is ≥83°C, and the sterilization time is 25 seconds. The discharge concentration is 48%-52% dry matter.
11) Liquid storage, preheating, filtration, and spray drying: the concentrated liquid is temporarily stored in a liquid balance tank. It is preheated to 60-70°C by a scraper preheater. After preheating, the material is filtered through a filter with a pore size of 1mm, and then pumped by a high-pressure pump into a drying tower for spray drying. The fine powder is agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
12) Fluidized bed drying and cooling: the powder coming out of the drying tower is dried twice in a fluidized bed (first level) and cooled to 25-30°C in a fluidized bed (second level).
13) Packaging: the staff of the powder making workshop weighs and seals the DHA, ARA, and lactoferrin in bags according to the formula requirements.
14) Dry mixing: the weighed DHA, EPA, ARA, lactoferrin, osteopontin and powdered materials are mixed in a dry mixer. If the protein content is high, some casein, whey protein powder and sodium caseinate can be mixed and added evenly by dry mixing to obtain powdered formula food.
15) Powder screening: the particle size of powdered formula food is uniformed by vibrating screen, and the powder residue is discarded.
16) Powder discharging: the powder is collected in a sterilized powder collecting box and transported from a powder discharging room to a powder loading room.
17) Powder loading: the powdered formula food is poured into a powder storage tank of large and small packaging machines according to the packaging requirements.
18) Packaging: 800g of the milk powder is packaged with nitrogen flushed by automatic packaging machine. The oxygen content is less than 1% when nitrogen is flushed. 900g of milk powder is packaged by iron cans automatically with nitrogen flushed, and the oxygen content is less than 5%.
19) Packing: the packaged small bags are put into a carton and a powder spoon is added at the same time, and the carton is sealed with a carton sealing machine.
20) Inspection of finished products: sampling inspection is carried out on the packaged products according to the inspection plan.
21) Storage: products that have passed the inspection are stored in a warehouse, and are required to be stored at room temperature with a humidity of ≤65%.

In some specific embodiments of the present disclosure, the formula food for special medical purpose is a formula food suitable for patients with traumatic stress, and the raw materials comprise:
whey protein powder 90-200 parts by weight;
sodium caseinate 0-100 parts by weight;
casein 0-100 parts by weight;
solid corn syrup 0-400 parts by weight;
maltodextrin 0-400 parts by weight;
sunflower oil 0-150 parts by weight;
corn oil 0-40 parts by weight;
soybean oil 20-80 parts by weight;
medium chain triglycerides 0-100 parts by weight;
fructo-oligosaccharide 0-50 parts by weight;
lactoferrin 0-7.5 parts by weight;
osteopontin 0-75 parts by weight;
compound nutrients comprising calcium powder, vitamins and minerals 7-50 parts by weight;
DHA 5-50 parts by weight;
tryptophan 0-10 parts by weight;
arginine 0-10 parts by weight;
glutamine 0-100 parts by weight; and
EPA 10-100 parts by weight.

In the formula food for patients with traumatic stress provided by the present disclosure, the specific amount of each raw material should be adjusted and determined on the premise of meeting the requirements for the product indicators of the formula food.

In the formula food for patients with traumatic stress provided by the present disclosure, each raw material can be commercially available, and the selection of each raw material should meet the requirements of relevant standards, wherein lactoferrin and osteopontin should meet the requirements of the present disclosure. In addition, the compound nutrients can also be compounded in house. The present disclosure uses "compound" only for the convenience of expression, and does not mean that the components in the compound must be mixed together before use. All raw materials should be added and used under the premise of meeting relevant laws and regulations.

The present disclosure also provides a method of preparing a formula food for patients with traumatic stress, and the process flow of the preparation mainly comprises: material formulation, homogenization, concentrated sterilization, spray drying, and dry mixing to obtain a finished product. Specifically, the method of preparing a formula food for patients with traumatic stress of the present disclosure comprises:
1) Addition of powder: various powder raw materials are added to a powder mixing tank uniformly through an air delivery system after being measured according to the formula for storage.
2) Vacuum suction of powder: various powder raw materials in the powder mixing tank are sucked into a vacuum mixing tank through a vacuum system.
3) Melting oil: putting the oil specified in the formula into the oil melting room according to the formula requirements. The temperature of the oil melting room should be maintained at 50-90°C. After the oil is melted, it is pumped into a mixed oil tank through the oil pump and flow meter according to the formula ratio requirements.
4) Storage of mixed oil: The mixed oil is stored in an oil storage tank at a temperature of 40-50°C. The storage time is less than 12 hours to prevent fat oxidation.
5) Weighing: the mixed oil is pumped into the mixing tank through the oil pump according to the formula requirements.
6) Dissolution and addition of nutrients: calcium powder, minerals, and vitamins are added separately, dissolved with 100-200kg of pure water separately, and pumped into a wet mixing tank. After each one is pumped, 100kg of pure water is used to rinse the addition tank and pipeline.
7) Dissolution and addition of lactoferrin, osteopontin and calcium salt: osteopontin and calcium salt are dissolved according to the partial mixed liquid in step 6, and added to the mixing tank to obtain a mixed liquid containing lactoferrin and osteopontin.
8) Filtration: the mixed liquid is filtered through a filter to remove physical impurities that may be brought into the raw materials.
9) Homogenization: the mixed liquid is homogenized by a homogenizer with a first-level homogenization pressure of 105±5bar, and a second-level pressure of 32±3bar. The fat globules are mechanically treated to disperse into uniform fat globules.
10) Cooling and storage: the homogenized liquid enters a plate heat exchanger for cooling: it is cooled to below 20°C, and temporarily stored in a pre-storage tank. It enters the next process within 6 hours, and an agitator is turned on according to the set requirements.
11) Concentration and sterilization: double-effect concentration is used during production, the sterilization temperature is ≥83°C, and the sterilization time is 25 seconds. The discharge concentration is 48%-52% dry matter.
12) Liquid storage, preheating, filtration, and spray drying: the concentrated liquid is temporarily stored in a liquid balance tank. It is preheated to 60-70°C by a scraper preheater. After preheating, the material is filtered through a filter with a pore size of 1mm, and then pumped by a high-pressure pump into a drying tower for spray drying. The fine powder is agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
13) Fluidized bed drying and cooling: the powder coming out of the drying tower is dried twice in a fluidized bed (first level) and cooled to 25-30°C in a fluidized bed (secondary level). At the same time, lactoferrin, osteopontin and calcium salt are mixed with the carrier and heated to 60-65°C. Under the action of compressed air, they are evenly dispersed on the surface of the powder, so that the powder particles agglomerate to increase their particle size and solubility.
14) Packaging: the staff of the powder making workshop weighs and seals DHA, ARA and lactoferrin in bags according to the formula requirements.
15) Dry mixing: the weighed DHA, EPA, ARA, lactoferrin and powdered materials are mixed in a dry mixer. If the protein content is high, some casein, whey protein powder and sodium caseinate can be mixed and added evenly by dry mixing to obtain powdered formula food.
16) Powder screening: the particle size of powdered formula food is uniformed by vibrating screen, and the powder residue is discarded.
17) Powder discharging: the powder is collected in a sterilized powder collecting box and transported from a powder discharging room to a powder loading room.
18) Powder loading: the powdered formula food is poured into a powder storage tank of large and small packaging machines according to the packaging requirements.
19) Packaging: 800g of the milk powder is packaged with nitrogen flushed by automatic packaging machine. The oxygen content is less than 1% when nitrogen is flushed. 900g of milk powder is packaged by iron cans automatically with nitrogen flushed, and the oxygen content is less than 5%.
20) Packing: the packaged small bags are put into a carton and a powder spoon is added at the same time, and the carton is sealed with a carton sealing machine.
21) Inspection of finished products: sampling inspection is carried out on the packaged products according to the inspection plan.
22) Storage: products that have passed the inspection are stored in a warehouse, and are required to be stored at room temperature with a humidity of ≤65%.

The nutritional components provided by the present disclosure can be prepared as a formula food for special medical purpose for premature/low birth weight infants, lactose-intolerant infants, patients with tumor, and patients with traumatic stress. They can synergistically increase serum anti-inflammatory factors IL-4 and IL-10 and reduce pro-inflammatory factors IL-1β; enhance intestinal epithelial tissue lymphocytes, inhibit activated CD4⁺ cells and neutrophils, and enhance intestinal immunity; protect intestinal permeability, promote secretion of intestinal mucin and maintain intestinal mucosal barrier.

In order to further understand the present disclosure, the composition for resisting enteritis and/or protecting integrity of intestinal barrier provided by the present disclosure and its use are described in conjunction with the following examples. The protection scope of the present disclosure is not limited by the following examples.

### Example 1

The composition of this example consisted of lactoferrin, osteopontin and calcium chloride, wherein the mass ratio of lactoferrin, osteopontin, and calcium in calcium chloride was 1:5:0.06.

### Example 2

The composition of this example consisted of lactoferrin, osteopontin and calcium chloride, wherein the mass ratio of the mass ratio of lactoferrin, osteopontin, and calcium in calcium chloride was 1:1:0.02.

### Example 3

The composition of this example consisted of lactoferrin, osteopontin and calcium chloride, wherein the mass ratio of the mass ratio of lactoferrin, osteopontin, and calcium in calcium chloride was 1:0.15:0.0115.

### Example 4

The composition of this example consisted of lactoferrin, osteopontin and calcium chloride, wherein the mass ratio of the mass ratio of lactoferrin, osteopontin, and calcium in calcium chloride was 1:0.1:0.011.

### Example 5

The composition of this example consisted of lactoferrin, osteopontin and calcium chloride, wherein the mass ratio of the mass ratio of lactoferrin, osteopontin, and calcium in calcium chloride was 1:0.1:0.001.

### Example 6

The composition of this example consisted of lactoferrin, osteopontin and calcium chloride, wherein the mass ratio of the mass ratio of lactoferrin, osteopontin, and calcium in calcium chloride was 1:0.1:40.

### Experimental example

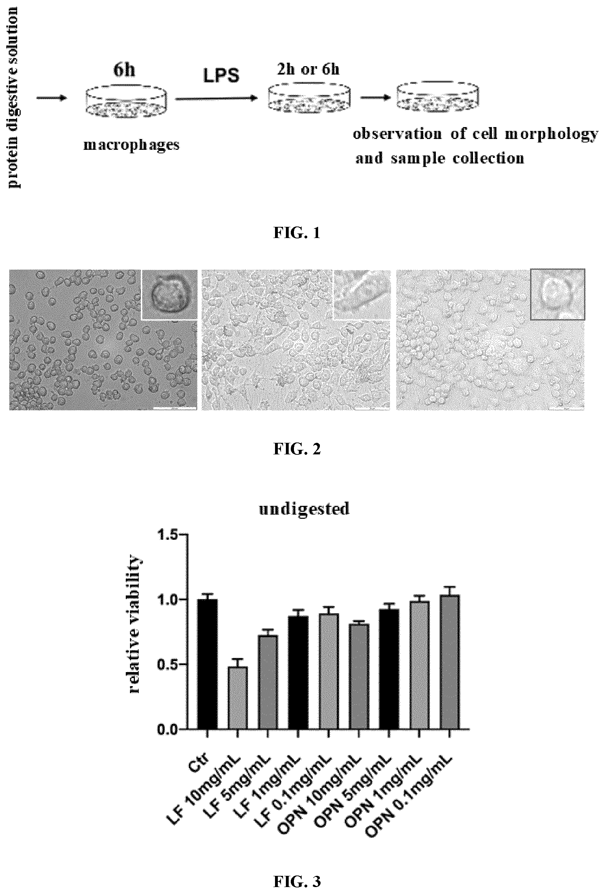

1. Materials and reagents
   RAW264.7 macrophages, calcium chloride, LPS, lactoferrin, osteopontin, PBS buffer, porcine trypsin, porcine pepsin, DMEM medium (Gibco, 11995065), the purity of calcium chloride ≥99%, lactoferrin from Hilmar1000, and osteopontin from Lacprodan^{®} OPN-10.
2. *In vitro* digestion of LF-OPN-calcium
   (1) Experimental groups were set up, and the composition and proportion of the raw materials were as follows:
      Group 1: consisting of lactoferrin, osteopontin and calcium chloride, wherein the mass ratio of lactoferrin, osteopontin, and calcium in calcium chloride was 1:5:0.06;
      Group 2: consisting of lactoferrin, osteopontin and calcium chloride, wherein the mass ratio of lactoferrin, osteopontin, and calcium in calcium chloride was 1:1:0.02;
      Group 3: consisting of lactoferrin, osteopontin and calcium chloride, wherein the mass ratio of lactoferrin, osteopontin, and calcium in calcium chloride was 1:0.15:0.0115;
      Group 4: consisting of lactoferrin, osteopontin and calcium chloride, wherein the mass ratio of lactoferrin, osteopontin, and calcium in calcium chloride was 1:0.1:0.011;
      Group 5: consisting of lactoferrin, osteopontin and calcium chloride, wherein the mass ratio of lactoferrin, osteopontin, and calcium in calcium chloride was 1:3:0.04.
      Group 6: consisting of lactoferrin, osteopontin and calcium chloride, wherein the mass ratio of lactoferrin, osteopontin, and calcium in calcium chloride was 1:0.02:0.012.
      Group 7: consisting of lactoferrin and osteopontin, wherein the mass ratio of lactoferrin and osteopontin is 1:0.2.
   (2) LF, OPN and calcium were successively dissolved in the in vitro digestion systems simulating gastric digestive juice and intestinal digestive juice in the proportions of step (1), and the digested samples were sterilized by a 0.22 µm filter membrane before treating the cells.
3. LF-OPN-calcium ion treatment of macrophages
   2 × 10⁵ RAW264.7 cells/well were inoculated into a 6-well culture plate, with 3 replicates for each treatment group. The inoculated cells were cultured at 37°C and 5% CO₂. After 24 hours, the serum-free and antibiotic-free DMEM medium containing LF, OPN and calcium ions (monomers or combinations after *in vitro* digestion) was replaced for 6 hours of pretreatment. LPS was then added to the culture medium to a concentration of 1 µg/ml. 2 hours after the LPS treatment, the cells were collected for RNA extraction, and 6 hours after LPS treatment, the cells were collected for protein extraction. The experimental process is shown in FIG. 1.
4. Detection indicators
   (1) Cell morphology

RAW264.7 cells were seeded in a 12-well cell culture plates, and after 24 hours, the serum-free and antibiotic-free DMEM medium containing LF, OPN, and calcium was replaced. After 6 hours of protein incubation, LPS was added to a concentration of 1µg/ml for 6 hours of treatment. Using an inverted microscope, the RAW264.7 cells treated at different time points were photographed to record the cell morphology and characterize the degree of differentiation.

The experimental results are shown in FIG. 2. In FIG. 2, left: blank control group; middle: LPS group; right: lactoferrin-osteopontin-calcium combination + LPS group. After 6 hours of LPS treatment, the shape of RAW264.7 cells changed from round when undifferentiated to elongated; however, when the treatment of active protein-calcium combination was performed for 6 hours in advance, the cell morphology after 6 hours of LPS treatment was rounder than that of LPS treatment alone, which was closer to the cell morphology when undifferentiated.

### (2) Cell viability

The cytotoxicity of active protein to RAW264.7 was determined by CCK8 experiment. The cells were cultured in 96-well plates. After 24 hours of inoculation, the cells were replaced with serum-free and antibiotic-free DMEM medium containing LF-OPN-calcium digests for 12 hours of treatment, and then replaced with serum-free and antibiotic-free DMEM medium containing CCK8 for 1 hour of treatment. The absorbance was measured at a wavelength of 450 nm using a microplate reader.

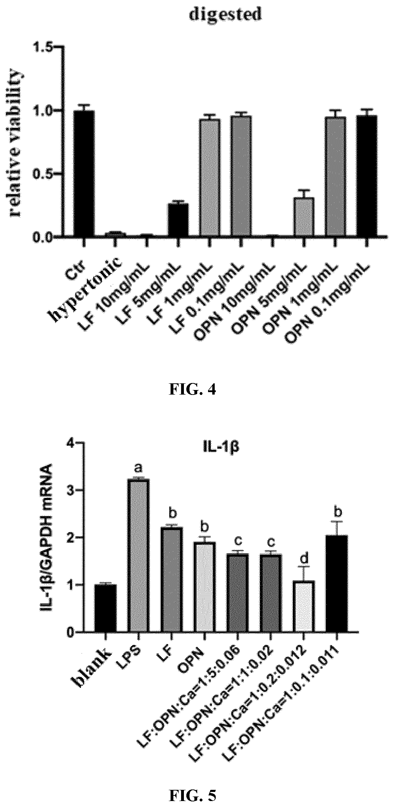

The experimental results are shown in FIGs. 3 and 4. When the concentrations of LF and OPN were higher than 5 mg/ml, the cell viability was significantly affected. When the concentrations were lower than 1 mg/ml, the cell toxicity was lower. Therefore, in this experiment, the concentrations of LF and OPN monomers or mixturewere all 1 mg/ml.

### (3) Expression of inflammatory factors

RAW264.7 cells were inoculated in 12-well cell culture plates. After 24 hours, the cells were replaced with serum-free and antibiotic-free DMEM medium containing LF-OPN-calcium. After 6 hours, LPS was added to a concentration of 1 µg/ml. After 2 hours, RNA was collected, and the expression levels of inflammatory factors such as IL-6 and IL-1β in mRNA were quantitatively analyzed by RT-qPCR.

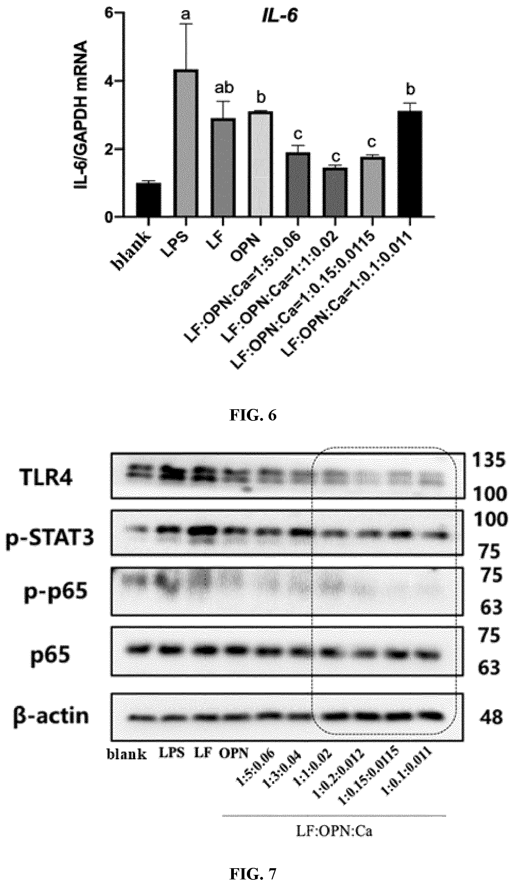

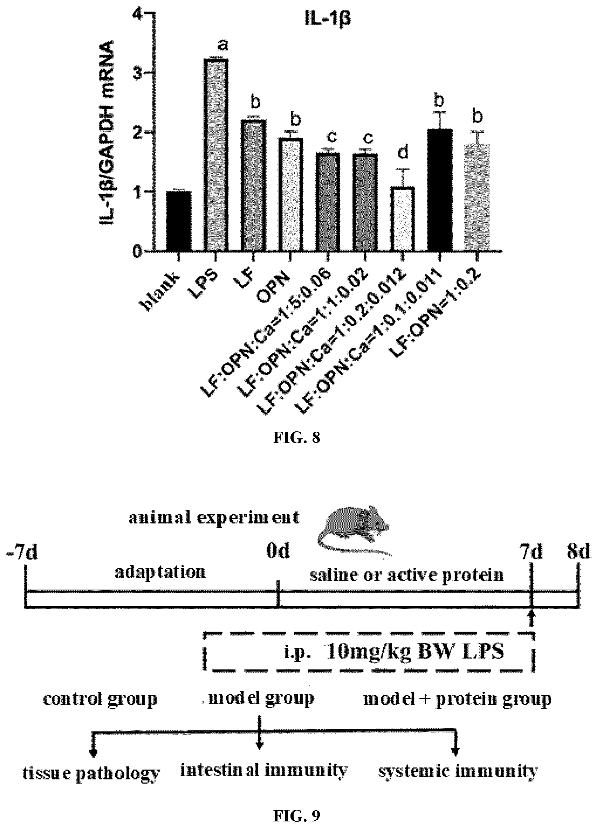

IL-1β and IL-6 are the most common pro-inflammatory factors. The experimental results are shown in FIGs. 5 and 6. It can be seen that the LF and OPN monomer digests can both inhibit the expression of inflammatory factor genes, and the combination has a synergistic effect. In the mixed digest, when the ratio is LF:OPN:Ca=1:(0.1-5):(0.011-0.06), it is better than LF and OPN monomers, and there is a statistical difference. From FIG. 8, it can be seen that compared with the experimental group of lactoferrin and osteopontin (LF:OPN=1:0.2), the lactoferrin, osteopontin and calcium group (LF:OPN:Ca=1:0.2:0.012) has a better anti-inflammatory effect, and there is a statistical difference.

### (4) Expression of proteins related to inflammatory pathways

RAW264.7 cells were inoculated in 12-well cell culture plates. After 24 hours, the serum-free and antibiotics-free DMEM medium containing LF, OPN, and calcium ions was replaced. After 6 hours, LPS was added to a concentration of 1µg/ml. After another 6 hours, protein samples were collected and the expression of proteins related to TLR4 and NF-κB pathways was analyzed by Western blot.

After treating RAW264.7 with LPS, LF and OPN monomer digests and composite digests of different ratios for six hours, the expression of membrane protein TLR4, key protein p-p65 (p65) of NF-κB pathway, and p-STAT3 in the downstream STAT pathway was detected. The results are shown in FIG. 7. It can be seen that the combination of LF:OPN:Ca=1:(0.1-5):(0.011-0.06), especially LF:OPN:Ca=1:(0.1-5):(0.011-0.02), can better inhibit the expression of inflammatory pathway proteins compared with LF or OPN monomers.

The raw materials in the compound vitamin nutrient package, mineral I nutrient package, mineral II nutrient package, magnesium chloride nutrient package, potassium chloride nutrient package and choline chloride nutrient package in the following product examples were all purchased from Beijing Jinkangpu Food Technology Co., Ltd.; the material composition of the compound vitamin nutrient package is: sodium L-ascorbate, lactose, taurine, dl-α-tocopherol acetate raw material (the mass content of dl-α-tocopherol acetate in the dl-α-tocopherol acetate raw material is 50%), pyridoxine hydrochloride raw material (the mass content of pyridoxine hydrochloride in the pyridoxine hydrochloride raw material is 5%), cholecalciferol raw material (the mass content of cholecalciferol in the cholecalciferol raw material is 0.25%), retinyl acetate raw material (the mass content of retinyl acetate in the retinyl acetate raw material is 11.5%), niacinamide, D-calcium pantothenate, D-biotin raw material (the mass content of D-biotin in the D-biotin raw material is 1%), phylloquinonep raw material (the mass content of phylloquinone in the phylloquinone raw material is 5%), riboflavin raw material (the mass content of riboflavin in the riboflavin raw material is 10%), folic acid raw material (the mass content of folic acid in the folic acid raw material is 10%), thiamine nitrate and cyanocobalamin raw material (the mass content of cyanocobalamin in the cyanocobalamin raw material is 1%);
the material composition of the mineral I nutrient package is: potassium iodide raw material (the mass content of potassium iodide in the potassium iodide raw material is 0.5%), copper sulfate raw material (the mass content of copper sulfate in the copper sulfate raw material is 5%), ferrous sulfate raw material (the mass content of ferrous sulfate in the ferrous sulfate raw material is 79.2%), zinc sulfate, sodium selenite raw material (the mass content of sodium selenite in the sodium selenite raw material is 0.5%), manganese sulfate raw material (the mass content of manganese sulfate in the manganese sulfate raw material is 5%) and lactose; and
the material composition of the mineral II nutrient package is: tricalcium phosphate, sodium chloride, calcium carbonate and lactose.

### Product Example 1

This example provides a powdered infant formula milk powder, wherein the total protein content of the powdered formula milk powder was 10.5 g/100g, the fat content was 28 g/100g; the carbohydrate content was 55g/100g, the lactoferrin content was 35mg/100g, the osteopontin content was 30mg/100g, and the calcium content in the calcium salt was 350mg/100g. The mass ratio of lactoferrin, osteopontin and the calcium in the calcium salt was 1:0.86:10.

This example was realized by compounding the following raw materials in parts by weight, and the raw material composition comprised:
1300 parts of raw milk, 320 parts of desalted whey powder (D90), 225 parts of edible vegetable blending oil, 210 parts of lactose, 22 parts of galacto-oligosaccharide, 25 parts of α-lactalbumin powder, 11 parts of fructo-oligosaccharide, 8 parts of casein, 5 parts of whey protein powder (WPC80), 4 parts of phospholipids, 22 parts of compound nutrient packages, wherein the compound nutrients include about 4 parts of compound vitamin nutrient package, about 1 part of choline chloride nutrient package, about 12 parts of mineral II nutrient package (wherein the calcium content in each gram of mineral II nutrient package was 290mg), about 1 part of mineral I nutrient package, about 2 parts of magnesium chloride nutrient package and about 2 parts of potassium chloride nutrient package, and the base material of each nutrient package included lactose, 14 parts of DHA, 17 parts of ARA, 0.1 parts of bifidobacterium powder, about 0.7 parts of nucleotides, and about 0.3 parts of lactoferrin.

The specific preparation process of the infant formula milk powder in this example was as follows:
1) Coarse filtration of milk: after the raw milk was coarsely filtered and degassed in the balance cylinder, it was preheated by a plate heat exchanger and then separated from impurities by a separator.
2) Homogenization and sterilization of milk: after impurities were removed, part of the raw milk entered the homogenizer for homogenization, and the other part was not homogenized. After the homogenization was completed, the two parts were mixed and entered the sterilization system for sterilization.
3) Addition of powder: various powder raw materials (except DHA, ARA and bifidobacterium powder) were added to a powder mixing tank through an air delivery system after being measured according to the formula, and were sucked into a vacuum mixing tank through a vacuum system;
4) Melting oil: the oil specified in the formula was put into an oil melting room according to the formula requirements, and the temperature of the oil melting room was maintained at 60°C. After the oil was melted, it was pumped into a mixed oil storage tank, and the mixed oil was pumped into a mixing tank through an oil pump according to the formula requirements;
5) Dissolution and addition of nutrient: calcium powder, vitamins, minerals and other nutrient packages were dissolved in pure water respectively, and then added to the mixing tank in sequence to obtain a mixed liquid.
6) Filtration: the mixed liquid was filtered through a filter to remove physical impurities brought by the raw materials.
7) Homogenization: the filtered liquid was homogenized by a homogenizer, and the fat globules in the liquid were mechanically treated so that the fat globules were dispersed into uniform fat globules to obtain a homogenized liquid.
8) Cooling and storage: the homogenized liquid entered a plate heat exchanger for cooling: it was cooled to below 20°C, temporarily stored in a pre-storage tank, and entered the next process within 6 hours, and an agitator was turned on according to the set requirements.
9) Concentration and sterilization: double-effect concentration was used during production, the sterilization temperature was ≥ 83°C, and the sterilization time was 25 seconds. The discharge concentration was 50% dry matter.
10) Storage, preheating, filtration and spray drying of concentrated milk: the concentrated milk was temporarily stored in the concentrated milk balance tank. It was preheated to 60°C by a scraper preheater. After preheated, the material was filtered through a filter with a pore size of 1mm, and then pumped with a high-pressure pump into a drying tower for spray drying. The fine powder was agglomerated at the top of the tower or in the fluidized bed as required. Inlet temperature: 180°C, exhaust temperature 86°C, high-pressure pump pressure 200bar, tower negative pressure about -4mba.
11) Fluidized bed drying and cooling: the powder coming out of the drying tower was dried again in a fluidized bed (primary) and cooled to 30°C in a fluidized bed (secondary) to obtain the main ingredient of milk powder.
12) Packaging: according to the formula requirements, the DHA, ARA and bifidobacterium powder were weighed and sealed in the bags for packaging.
13) Dry mixing: the weighed DHA, ARA and bifidobacterium powder were mixed with the main ingredient of milk powder in the dry mixer.
14) Powder screening: a vibrating screen was used to make the particle size of the milk powder uniform, and the powder residue was discarded.
15) Powder discharging: a sterilized powder collection box was used to collect the powder, which was then transported from a powder discharging room to a powder loading room.
16) Powder loading: the milk powder was poured into the powder storage tank of large and small packaging machines according to the packaging requirements.
17) Packaging: 400g of milk powder was packaged with nitrogen flushed by automatic packaging machine. The oxygen content was less than 1% when nitrogen was flushed.
18) Packing: the packaged small bags were put into a carton and a powder spoon was added at the same time, and the carton was sealed with a carton sealing machine.
19) Inspection of finished products: sampling inspection was carried out on the packaged products according to the inspection plan.
20) Storage: products that had passed the inspection were stored in a warehouse, and were required to be stored at room temperature with a humidity of ≤ 65%.

### Product Example 2

This example provides a powdered infant formula milk powder, wherein the total protein content of the powdered formula milk powder was 11 g/100g, the fat content was 27 g/100g; the carbohydrate content was 54g/100g; the lactoferrin content was 110 mg/100g, the osteopontin content was 22 mg/100g, and the calcium content in the calcium salt was 450 mg/100g. The mass ratio of lactoferrin, osteopontin and calcium in the calcium salt was 1:0.2:4.

This example was realized by compounding the following raw materials in parts by weight, and the raw material composition comprised:
1200 parts of raw milk, 355 parts of desalted whey powder (D90), 225 parts of edible vegetable blending oil, 170 parts of lactose, 40 parts of galacto-oligosaccharide, 25 parts of α-lactalbumin powder, 10 parts of fructo-oligosaccharide, 17 parts of casein, 4 parts of phospholipids, 22 parts of compound nutrient packages, wherein the compound nutrients comprised about 4 parts of compound vitamin nutrient package, about 1 part of choline chloride nutrient package, about 12 parts of mineral II nutrient package (wherein the calcium content of in each gram of mineral II nutrient package was 375mg), about 1 part of mineral I nutrient package, about 2 parts of magnesium chloride nutrient package and about 2 parts of potassium chloride nutrient package, and the base material of each nutrient package included lactose, 12 parts of DHA, 14 parts of ARA, 0.1 parts of bifidobacterium powder, about 0.6 parts of nucleotides, and 1.3 parts of lactoferrin.

The specific preparation process of the infant formula milk powder in this example was consistent with that in product example 1.

### Product example 3

This example provides a powdered infant formula milk powder, wherein the total protein content of the powdered formula milk powder was 13.5 g/100g, the fat content was 22 g/100g; the carbohydrate content was 55g/100g, the lactoferrin was 8 mg/100g, the osteopontin was 40 mg/100g, and the calcium in the calcium salt was 320 mg/100g, and the mass ratio of the lactoferrin, osteopontin and the calcium in the calcium salt was 1:5:40.

This example was realized by compounding the following raw materials in parts by weight, and the raw material composition comprised:
1150 parts of raw milk, 200 parts of skimmed milk powder, 175 parts of desalted whey powder (D90), 170 parts of edible vegetable blending oil, 220 parts of lactose, 70 parts of galacto-oligosaccharide, 4 parts of α-lactalbumin powder, 11 parts of fructo-oligosaccharide, 1 part of casein, 8 parts of whey protein powder (WPC80), 4 parts of phospholipids, 17 parts of compound nutrient packages, wherein the compound nutrients included about 3 parts of compound vitamin nutrient packages, about 2 parts of choline chloride nutrient packages, 8 parts of mineral II nutrient packages (wherein the calcium content in each gram of mineral II nutrient package was 400mg), about 1 part of mineral nutrient package, about 1 part of magnesium chloride nutrient package and about 2 parts of potassium chloride nutrient package, and the base material of each nutrient package included lactose, 11 parts of DHA, 13 parts of ARA, 0.1 parts of bifidobacterium powder, about 0.7 parts of nucleotides, and about 0.03 parts of lactoferrin.

The specific preparation process of the infant formula milk powder in this example was consistent with that in product example 1.

### Product comparative example

This example provides a powdered infant formula milk powder, the total protein content of the powdered formula milk powder was 13.5 g/100g, the fat content was 21 g/100g; the carbohydrate content was 55g/100g, the lactoferrin content was 5 mg/100g, the osteopontin content was 30 mg/100g, and the content of calcium in calcium salt was 240mg/100g, and the mass ratio of lactoferrin, osteopontin and calcium in calcium salt was 1:6:48.

This example was realized by compounding the following raw materials in parts by weight, and the raw material composition comprised:
1400 parts of raw milk, 190 parts of skimmed milk powder, 155 parts of desalted whey powder (D90), 155 parts of edible vegetable blending oil, 235 parts of lactose, 64 parts of galacto-oligosaccharide, 4 parts of α-lactalbumin powder, 16 parts of fructo-oligosaccharide, 1 part of casein, 8 parts of whey protein powder (WPC80), 4 parts of phospholipids, 17 parts of compound nutrient packages, wherein the compound nutrients comprised about 3 parts of compound vitamin nutrient packages, about 2 parts of choline chloride nutrient packages, 8 parts of mineral II nutrient packages (wherein the calcium content of each gram of mineral II nutrient package was 300mg), about 1 part of mineral nutrient package, about 1 part of magnesium chloride nutrient package and about 2 parts of potassium chloride nutrient package, and the base material of each nutrient package included lactose, 11 parts of DHA, 13 parts of ARA, 0.1 parts of bifidobacterium powder, and about 0.7 parts of nucleotides.

The specific preparation process of the infant formula milk powder in this example was consistent with that in product example 1.

The nutritional composition content in the nutritional package of the following product example was as follows:
1) Compound vitamin nutrient package, each gram of compound vitamin nutrient package contained:
   Taurine: 140-340mg
   Vitamin A: 1700-5800µgRE
   Vitamin D: 25-70µg
   Vitamin B₁: 2000-6800µg
   Vitamin B₂: 3000-6900µg
   Vitamin B₆: 1700-4000µg
   Vitamin B₁₂: 8-20µg
   Vitamin K₁: 200-700µg
   Vitamin C: 0-700mg
   Vitamin E: 10-70mg α-TE
   Niacinamide: 10000-41550µg
   Folic acid: 350-920µg
   Biotin: 70-245µg
   Pantothenic acid: 7100-25230µg
   Inositol: 0-250mg
   L-carnitine: 0-60mg.
2) Mineral I nutrient package, each gram of mineral I nutrient package contained:
   Iron: 20-110mg
   Zinc: 23-90mg
   Copper: 2000-4180µg
   Iodine: 500-995µg
   Selenium: 0-200µg
   Manganese: 0-579µg
3) Mineral II nutrient package, each gram of mineral II nutrient package contained:
   Sodium: 40-100mg
   Potassium: 200-500mg
4) Mineral III nutrient package, each gram of mineral III nutrient package contained:
   Calcium: 200-500mg
   Phosphorus: 75-300mg.

The calcium salts in mineral III nutrient package were calcium carbonate, calcium hydrogen phosphate and tricalcium phosphate.

Product Example 4 - formula powder for patients with tumor (preparation of 1000 kg):
70 kg of casein, 70 kg of whey protein powder, 200 kg of sodium caseinate, 320 kg of maltodextrin, 60 kg of corn oil, 40 kg of soybean oil, 20 kg of medium chain triglycerides, 30 kg of fructo-oligosaccharide, 6 kg of lactoferrin, 0.6 kg of osteopontin, 3 kg of calcium salt, 1 kg of glutamine, 20 kg of compound nutrient package, 50 kg of DHA, 80 kg of EPA, 0.5 kg of arginine, 0.5 kg of tryptophan, and the rest was maltodextrin.

Among them, each gram of compound nutrient package comprised about 6.0 parts by weight of compound vitamin nutrient package, about 2.0 parts by weight of choline chloride nutrient package, about 10 parts by weight of mineral III nutrient package, 20 parts by weight of mineral II nutrient package, about 6 parts by weight of mineral I nutrient package, and about 7.0 parts by weight of compound magnesium chloride nutrient package, and the base material of each nutrient package was maltodextrin.

The specific preparation process of the formula powder containing lactoferrin, osteopontin and calcium salt was as follows:
1) Addition of powder: the powder base materials were uniformly added to a powder mixing tank through an air delivery system after being measured according to the formula for storage.
2) Vacuum suction of powder: the various powder materials in the powder mixing tank were sucked into a vacuum mixing tank through a vacuum system.
3) Melting oil: the oil specified in the formula was put into an oil melting room according to the formula requirements. The temperature of the oil melting room should be maintained at 50-90°C. After the oil was melted, it was pumped into the mixed oil storage tank through an oil pump and a flow meter according to the formula ratio requirements.
4) Storage of mixed oil: the mixed oil was stored in an oil storage tank at a temperature of 40-50°C, and the storage time was less than 12 hours to prevent fat oxidation.
5) Weighing: the mixed oil was pumped into a mixing tank through an oil pump according to the formula requirements.
6) Dissolution and addition of nutrients: the compound nutrient packages containing calcium powder, minerals, and vitamins were dissolved separately with 100-200kg of pure water, and then pumped into a wet mixing tank. After each one was pumped, the addition tank and pipeline were rinsed with 100kg of pure water.
7) Dissolusion and addition of lactoferrin, osteopontin and calcium salt: lactoferrin, osteopontin and calcium salt were dissolved according to the steps in step 6, and added to the mixing tank.
8) Filtration: the mixed liquid was filtered through a filter to remove physical impurities that may be brought into the raw materials.
9) Homogenization: the filtered liquid was homogenized by a homogenizer with a first-level homogenization pressure of 105±5bar and a second-level pressure of 32±3bar. The fat globules in the liquid were dispersed into uniform fat globules.
10) Cooling and storage: the homogenized liquid entered a plate heat exchanger for cooling: it was cooled to below 20°C, temporarily stored in a pre-storage tank, and entered the next process within 6 hours. An agitator was turned on according to the set requirements.
11) Concentration and sterilization: double-effect concentration was used during production, the sterilization temperature was ≥ 83°C, and the sterilization time was 25 seconds. The discharge concentration was 48%-52% dry matter.
12) Storage, preheating, filtration, and spray drying of concentrated milk: the concentrated milk was temporarily stored in a concentrated milk balance tank, preheated to 60-70°C by a scraper preheater, the preheated material was filtered through a filter with a pore size of 1mm, and then pumped by a high-pressure pump into a drying tower for spray drying. The fine powder was agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
13) Fluidized bed drying and cooling: the powder coming out of the drying tower was dried twice by a fluidized bed (first level), and then cooled to 25-30°C by a fluidized bed (secondary level).
14) Packaging: the staff of the powder making workshop weighed DHA, ARA and lactoferrin according to the formula requirements and sealed them in the bags for packaging.
15) Dry mixing: the weighed DHA, EPA, ARA, lactoferrin and milk powder were mixed evenly in the dry mixer.
16) Powder screening: a vibrating screen was used to make the particle size of the milk powder uniform, and the powder residue was discarded.
17) Powder discharging: a sterilized powder collection box was used to collect the powder, which was transported from a powder discharging room to a powder loading room.
18) Powder loading: the milk powder was poured into a powder storage tank of large and small packaging machines according to the packaging requirements.
19) Packaging: 800g of the milk powder was packaged with nitrogen flushed by an automatic packaging machine, and the oxygen content was less than 1% when the nitrogen was flushed.
20) Packing: the packaged small bags was put into a carton and then a powder spoon was added at the same time, and the carton was sealed with a box sealing machine.
21) Inspection of finished products: sampling inspection was carried out on the packaged products according to the inspection plan.
22) Storage: products that had passed the inspection were stored in a warehouse at room temperature with a humidity ≤ 65%.

In the product of this example, the total protein content was 34g/100g, the fat content was 12g/100g, the carbohydrate content was 32g/100g, the lactoferrin content was 600mg/100g, the osteopontin content was 60mg/100g, the calcium content was 300mg/100g, and the mass ratio of lactoferrin, osteopontin and calcium was 1:0.1:0.5.

Product Example 5-formula powder for patients with traumatic stress (preparation of 1000 kg):
60 kg of sodium caseinate, 70 kg of casein, 200 kg of whey protein powder, 340 kg of maltodextrin, 120 kg of corn oil, 30 kg of soybean oil, 35 kg of medium chain triglycerides, 30 kg of fructo-oligosaccharide, 0.16 kg of lactoferrin, 0.8 kg of osteopontin, 6 kg of calcium salt, 1 kg of glutamine, 25 kg of compound nutrient package, 30 kg of DHA, 60 kg of EPA, 1 kg of arginine, 1 kg of tryptophan, and the rest was maltodextrin.

Among them, each gram of the compound nutrient package comprised about 6.0 parts by weight of the compound vitamin nutrient package, about 2.0 parts by weight of the choline chloride nutrient package, about 20 parts by weight of the mineral III nutrient package, 20 parts by weight of the mineral II nutrient package, about 6 parts by weight of the mineral I nutrient package, and about 7.0 parts by weight of the compound magnesium chloride nutrient package, and the base material of each nutrient package was maltodextrin.

The product preparation process was as shown in product example 4.

In the product of this example, the total protein content was 33g/100g, the fat content was 18.5g/100g, the carbohydrate content was 34g/100g, the lactoferrin content was 16mg/100g, the osteopontin content was 80mg/100g, the calcium content was 640mg/100g, and the mass ratio of lactoferrin, osteopontin and calcium was 1:5:40.

### Experimental example

### 1. Materials and methods

1.1 Experimental animals: 4-week-old male SPF grade C57BL/6J mice.

1.2 Group design: the animal test groups were designed based on the results of the *in vitro* test as shown in Table 1:

**Table 1 Animal test group design**

| Number of groups | Group | Ratio |
|---|---|---|
| Control group | Normal saline | 10mg/kg BW |
| 1 | OPN group | 300mg/kg BW |
| 2 | LF group | 300mg/kg BW |
| 3 | LF:OPN group 1 | 1:10 and dosage was 300mg/kg BW |
| 4 | LF:OPN group 2 | 1:5 and dosage was 300mg/kg BW |
| 5 | LF:OPN group 3 | 1:1 and dosage was 300mg/kg BW |
| 6 | LF:OPN group 4 | 1:0.2 and dosage was 300mg/kg BW |

### 1.3 Experimental design

120 4-week-old male SPF mice (C57BL/6J) were raised in a mouse house at 22-25°C, with a 12-h light/dark cycle per day, and free access to water and food, and the formal experiment began after 1 week of adaptation. Before the start of the experiment, the mice were randomly divided into 8 groups according to body weight.

### 2 Experimental methods

### 2.1 Establishment and treatment of enteritis mouse model

After a one-week adaptation period, the mice were given 300 mg/kg BW/d of lactoferrin, osteopontin, or a combination of the two proteins by gavage for 7 consecutive days, and lipopolysaccharide (LPS) was injected intraperitoneally on the seventh day to establish the model, that is, LPS was injected intraperitoneally at 10 mg/kg BW (body weight), and the control group was injected intraperitoneally with 10 mg/kg BW of normal saline. The mice were scrificed on the 8^{th} day and biological samples were collected, as shown in FIG. 9.

FIG. 9 is a schematic diagram of the establishment of enteritis mouse model. In FIG. 9, the control group was the saline group, the model group was injected with LPS only, and the model + protein group was injected with LPS and different ratios of LF:OPN.

### 2.2 Sample collection and preservation

Serum collection and preservation: blood was collected from the orbital vein, placed in a water bath at 37°C until serum precipitated, and centrifuged at 3000r/min for 10 min, and the supernatant was packaged, frozen in liquid nitrogen and stored in a -80°C refrigerator.

Intestinal sample collection and preservation: the small intestine was cut open and intestinal tissue samples of about 1×1 cm² were taken from the same position of the duodenum, jejunum and ileum, and the middle of the colon, respectively. The contents attached to the intestinal wall were gently washed off in saline, and the intestine was spread on filter paper, and immersed in 4% paraformaldehyde fixative for making tissue sections. The fixative containing intestinal tissue was stored in a 4°C refrigerator. The sampling sites were: 4 cm behind the pylorus for the duodenum, 10 cm behind the duodenum-colon ligament for the jejunum, and 10 cm behind the junction of the jejunum and ileum (ileocecal ligament) for the ileum.

### 2.3 Tissue sections and histopathological evaluation

### Tissue section preparation:

(1) Tissue dehydration, transparency and wax immersion: the colon was removed from 70% ethanol and immersed in 80% ethanol for 40 minutes; 95% ethanol for 60 minutes; anhydrous ethanol I for 30 minutes; anhydrous ethanol II for 30 minutes; xylene I for 5 minutes; xylene II for 5 minutes; wax solution I for 60 minutes; wax solution II for 60 minutes sequecially.
(2) Embedding: the wax solution was poured into an embedding box, the tissue block was placed therein, and the wax holder was installed.
(3) Sectioning: a manual slicer was used to cut the wax block into continuous 4 µm wax strips, then the wax strips were put in a water bath at 43°C so that the wax strips adhered to the surface of microscope slide.
(4) Baking: the temperature was kept at 37°C overnight, and baking was performed at 62°C for 60 minutes.

Intestinal histological examination (hematoxylin-eosin staining):
(1) Dewaxing: the slices were immersed in xylene I for 10 minutes; xylene II for 10 minutes; anhydrous ethanol for 8 minutes; 95% ethanol for 5 minutes; 70% ethanol for 5 minutes; 50% ethanol for 5 minutes; and distilled water for 5 minutes successively.
(2) Staining: the slices were stained with hematoxylin staining solution for 1 minute; washed with distilled water 3 times, 2 minutes each time; differentiated with 1% hydrochloric acid alcohol solution for 5 seconds; washed with distilled water 3 times, 5 minutes each time; stained with eosin staining solution for 1 minute; and washed with distilled water 3 times, 2 minutes each time.
(3) Dehydration, transparency, and sealing: the slices were immersed in 70% ethanol for 2 seconds; 95% ethanol for 5 seconds; anhydrous ethanol for 5 minutes; xylene I for 5 minutes; and xylene II for 5 minutes successively; 1 drop of neutral balsam was dripped on the microscope slide, and the microscope slide was covered with a cover glass, and dried overnight. Observation was performed and photos were taken under a microscope.

### 2.4 Mucin secretion detection

(1) Dewaxing: the tissue sections were immersed in xylene I for 10 min; xylene II for 10 min; anhydrous ethanol for 8 min; 95% ethanol for 2 min; 70% ethanol for 2 min; and distilled water for 2 min successively;
(2) 3% acetic acid solution was added for incubation at room temperature for 3 min;
(3) the acetic acid solution was shaken off, and Alcian blue solution (pH 2.5) was added for incubation at 37°C for 15 min;
(4) the sections were placed in 3% acetic acid solution and rinsed for 10 sec;
(5) the setions were washed with distilled water 3 times, each washing time being 2 min, 1 min, and 1 min respectively;
(6) the sections were stained with nuclear fast red dye solution for 5 min, then washed with distilled water 3 times, each washing time being 2 min, 1 min, and 1 min respectively;
(7) the sections were dehydrated and sealed.

### 2.5 Intestinal goblet cell detection

Intestinal goblet cell detection:
(1) the intestinal tissue sections were dewaxed to water;
(2) the intestinal tissue sections were treated with 0.5% periodic acid solution for 10 min;
(3) the intestinal tissue sections were rinsed with running water for 5 min, and then washed with distilled water twice;
(4) the intestinal tissue sections were stained with Schiff reagent in the dark for 30 min;
(5) the intestinal tissue sections were rinsed with running water for 10 min;
(6) the nuclei were counterstained with hematoxylin for 30 s, which were then differentiated with 1% hydrochloric acid aqueous solution for 3 s, and blued with ammonia water;
(7) the intestinal tissue sections were dehydrated with anhydrous ethanol;
(8) the intestinal tissue sections were treated n-butanol for 30 s;
(9) the intestinal tissue sections were cleared with xylene and mounted with neutral balsam.

After PAS staining, observation was performed under a light microscope, and it could be found that red glycogen-stained cells were goblet cells. Six fields of view were randomly selected under a high-power microscope to count the number of goblet cells for comparison.

### 2.6 Immunohistochemistry

After the OCT embedded frozen tissue were sectioned, they were incubated in 3% BSA at room temperature for 1 h, and then incubated with fluorescent primary antibodies (primary antibodies for F4/80, Gr1, CD11b, CD4, CD8a, and CD31) at 4°C overnight, followed by incubation with fluorescent secondary antibodies for 1 h. After the sections were rinsed three times with PBS, the cell nuclei were stained with Hoechst33528, and then observed and photographed under a fluorescence microscope.

### 2.7 Intestinal permeability test

The D-lactic acid content in serum and diamine oxidase activity were measured by enzyme-linked immunosorbent assay according to the kit instructions of assay kits purchased from Nanjing Jiancheng Bioengineering Institute.

### 2.8 ELISA detection of immunoglobulins and inflammatory factors

The ELISA kit used in this test was different from the traditional 96-well plate coated with antibody and enzyme-labeled reading kit. Instead, it used microspheres-coated antibodies, and the fluorescence signal intensity was detected by flow cytometry and the test results were obtained after analysis by dedicated software. The specific experimental steps were as follows:
(1) the standard was gradiently diluted, 25 µL of standard/sample was added, 25 µL of Assay Buffer and 25 µL of mixed microspheres were added to a 96-well plate, with three replicates for each sample, and the well plate was placed on a shaker, and mixed at 800 rpm for 2 h at room temperature;
(2) centrifugation was performed, the supernatant was removed, and 200 µL of wash buffer was added to wash the precipitate twice;
(3) 25 µL of detection antibody was added to each well, and mixed at 800 rpm for 1 h at room temperature on a shaker;
(4) 25 µL of PE fluorescent dye was added to each well, and mixed at 800 rpm for 0.5 h at room temperature;
(5) centrifugation was performed, the supernatant was removed, 200 µL of wash buffer was added to wash the precipitate twice, and the precipitate was resuspend with 150 µL of wash buffer;
(6) a flow cytometer was used to detect the resuspended microspheres with the PE channel as the horizontal axis and the APC channel as the vertical axis;
(7) a dedicated software was used to draw standard curves for each indicator based on the flow cytometry results, and the sample concentration was calculated based on the standard curve.

### Cytokines detected: IL-1β, IL-4, IL-10

### 3 Detection of tight junction proteins

The intestinal tissue ground with liquid nitrogen was lysed in RIPA solution (10 mmol/L Tris-HCl, pH 7.4; 150 nmol/L NaCl; 10 mmol/L EDTA; 1% NP-40; 0.1% SDS) (containing protease and phosphatase inhibitors) and centrifuged at 13800 g for 15 minutes. The protein content of the supernatant was quantified by the BCA method. The protein was separated by SDS-PAGE electrophoresis (12% separation gel and 5% stacking gel) with a sample load of 25 µg (electrophoresis conditions were 65 V, 40 minutes; 115 V, 80 minutes). After the electrophoresis was completed, the membrane was transferred by wet transfer under constant current conditions (conditions were 200 mA, 50 minutes). Then, it was blocked with 5% skimmed milk powder or 5% albumin at room temperature for 1 hour. The membrane was incubated with the primary antibody (1:2000 dilution) at 4°C overnight, and washed three times with TBST buffer with 10 minutes each time. Then the membrane was incubated with HRP-labeled secondary antibody (1:5000 dilution) at room temperature for 1 hour. The membrane was washed three times with TBST washing buffer with 10 minutes each time. The membrane was reacted with enhanced ECL chemiluminescence reagent and exposed and developed by the ImageQuant LAS 4000 mini system.

### 3.0 Data statistical analysis

The data are expressed as "mean ± standard error". The ANOVA model of SAS 9.0 was used for one-way or two-way analysis of variance, and Duncan's method was used for multiple comparisons. P < 0.05 indicated a significant difference. The same letters in the figures indicated P > 0.05, that is, there was no statistically significant difference.

### Experimental results:

### Experimental example 1: overall immune effect of protein combination on enteritis mice - serum cytokines IL-1β, IL-4, IL-10

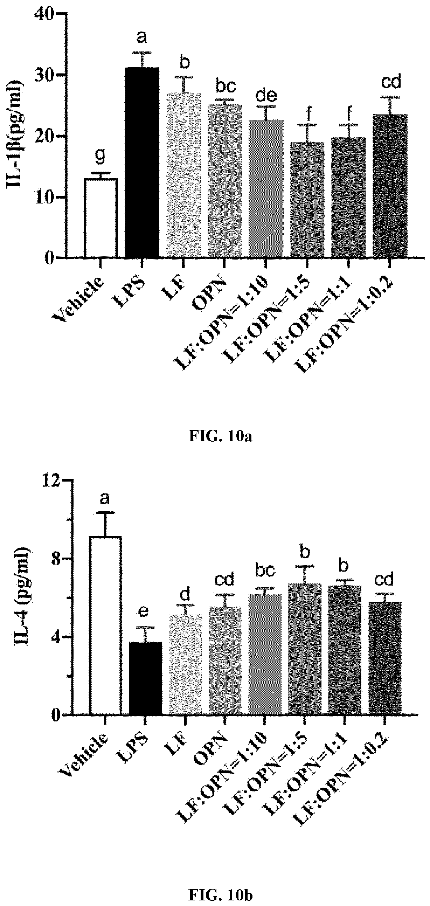

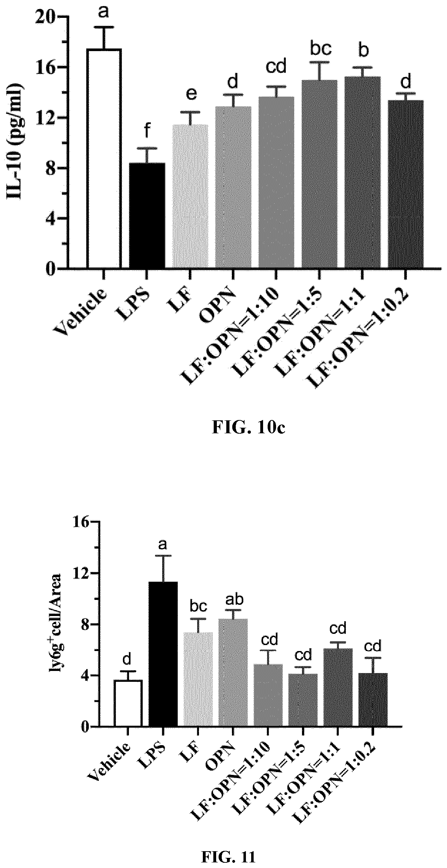

See FIG. 10a-FIG. 10b, which show the overall immune effect of protein combination on enteritis mice. FIG. 10a is the overall immune effect of protein combination on enteritis mice (IL-1β); FIG. 10b is the overall immune effect of protein combination on enteritis mice (IL-4); FIG. 10c is the overall immune effect of protein combination on enteritis mice (IL-10). In FIG. 10a-10b, vehicle is the saline group, LPS is the lipopolysaccharide group, LF is the lactoferrin group, OPN is the osteopontin group, "LF:OPN=1:10" represents the compound group with a lactoferrin and osteopontin mass ratio of 1:10, "LF:OPN=1:5" represents the compound group with a lactoferrin and osteopontin mass ratio of 1:5; "LF:OPN=1:1" represents the compound group with a lactoferrin and osteopontin mass ratio of 1:1; "LF:OPN=1:0.2" represents the compound group with a lactoferrin and osteopontin mass ratio of 1:0.2.

The experiment first selected four groups of ratios of lactoferrin and osteopontin; the results in FIG. 10a show that both LF and OPN monomers reduced the increase of the pro-inflammatory factor IL-1β caused by LPS, but the combination at the ratio of LF:OPN=1:10-1:0:2 had a better anti-inflammatory effect on IL-1β than that of monomers; in addition, LF and OPN monomers could increase the levels of anti-inflammatory factors IL-4 (FIG. 10b) and IL-10 (FIG. 10c), but the combination of LF and OPN produced a higher level of anti-inflammatory factors. Similarly, the combination at the ratio of LF:OPN=1:10-1:0:2 had a better promoting effect on anti-inflammatory factors than that of monomers. Therefore, the compound ratio of LF and OPN in the range of 1:10-1:0.2 had a certain effect of enhancing the body's immunity.

### Experimental Example 2: effect of protein combination on intestinal local immunity - neutrophils

See FIG. 11, which shows the effect of protein combination on intestinal local immunity (neutrophils). Based on Experimental Example 1, the ratios of LF:OPN were preferably 1:10, 1:1, and 1:0.2, and the immune effect of protein combination on specific damaged parts, namely the intestine, was further explored. In metabolic inflammation, the migration of neutrophils to the inflammatory site can promote macrophage infiltration of adipose tissue to cause local and systemic inflammation. However, when the protein combination was at a ratio of LF:OPN=1:10 to 1:0.2, the number of neutrophils in intestinal tissue was significantly reduced compared with the use of the monomer, which was beneficial to reducing the intestinal inflammatory response.

### Experimental Example 3: effect of protein combination on intestinal local immunity - CD4⁺ T cells

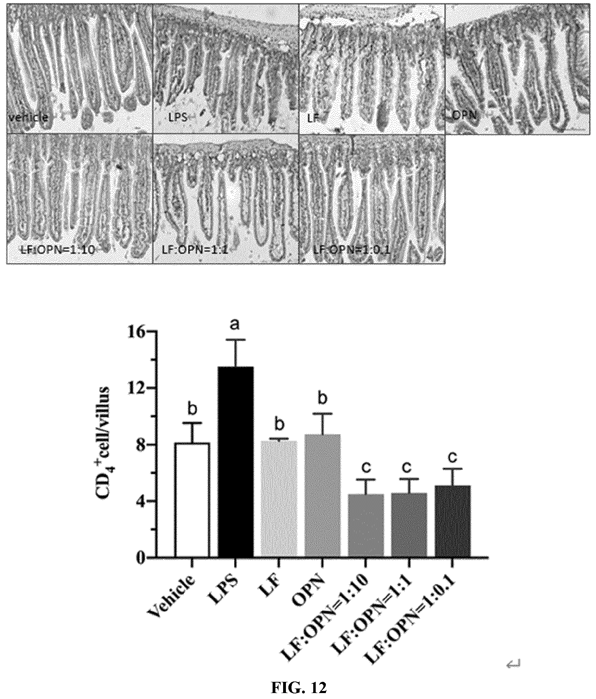

See FIG. 12, which shows the effect of protein combination on intestinal local immunity (CD4⁺ T cells). The upper pannel of FIG. 12 is a cell staining picture, and the lower pannel is a CD4⁺ count of the cell staining picture. According to previous reports, there is a group of overactivated CD4⁺ T cells in the lesions of inflammatory bowel disease, which act as pathogenic factors of enteritis. The results of Experimental Example 3 showed that when the protein combination was at a ratio of LF:OPN=1:10-1:0.2, it significantly reduced the CD4⁺ T cells in the intestinal tissue compared with the use of the monomers, which helped to reduce intestinal inflammation.

### Experimental Example 4: protective effect of protein combination on intestinal permeability

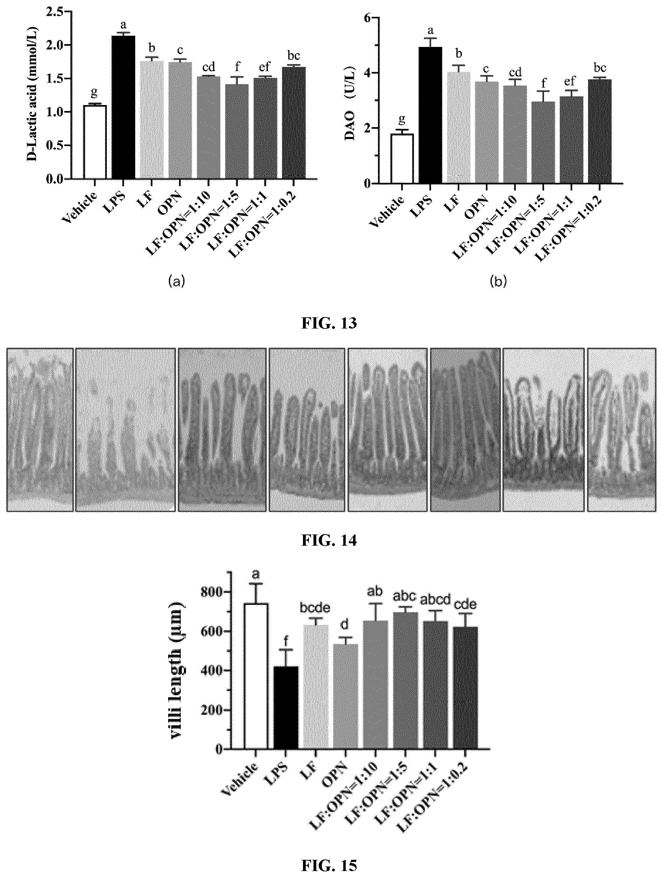

See FIG. 13, which shows the protective effect of protein combination on intestinal permeability. D-lactic acid and DAO (diamine oxidase) only exist in the intestine under normal physiological conditions. When the intestinal barrier is destroyed and the intestinal permeability increases, the two are released into the blood. The determination of DAO activity and D-lactic acid content in serum can directly reflect intestinal permeability. The results showed that LF and OPN monomers had a certain protective effect on the intestinal permeability of enteritis mice, but when the compound ratio of LF and OPN was 1:10-1:0.2, it had a synergistic effect of reducing intestinal permeability.

### Experimental Example 5: protective effect of protein combination on intestinal tissue damage

See FIGs. 14 and 15. FIG. 14 is a photo of the protective effect of protein combination against intestinal tissue damage. In FIG. 14, the groups from left to right are: vehicle, LPS, LF, OPN, LF:OPN=1:10, 1:5, 1:1, 1:0.2. FIG. 15 is the test result of villus length. Intestinal damage in LPS-modeled enteritis mice (second from the left in the figure) is manifested in shortened and sparse intestinal villi. The mixed protein (LF:OPN=1:10-1:0.2) treatment group had a better reduction in intestinal damage than that of the monomer, which promoted the increase of villus length.

### Experimental Example 6: protective effect of protein combination on mucus barrier

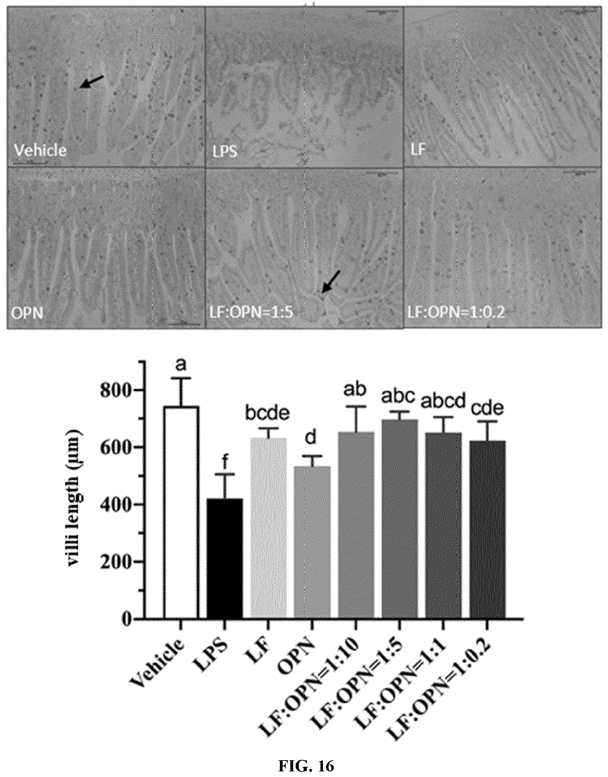

See FIG. 16, which shows the protective effect of protein combination on mucus barrier. The upper pannel of FIG. 16 is the AB cell staining picture, and the lower pannel is the count of the AB cell staining picture. Goblet cells are mucus-secreting cells distributed between mucosal columnar epithelial cells. They secrete mucin to form a mucosal barrier to protect epithelial cells. The intestinal mucus barrier can prevent intraluminal bacteria from contacting the epithelium, play an anti-infection role, and regulate the balance between intestinal immunity and external stimulation.

Based on Experimental Example 4, the protein combinations at ratios of LF:OPN=1:5 and 1:0.2 were selected. The results showed that the protein combination LF:OPN=1:5 could stimulate the secretion of mucin, and its effect was better than the monomers.

### Experimental Example 7: protective effect of protein combination on the integrity of the intestinal mucosal mechanical barrier

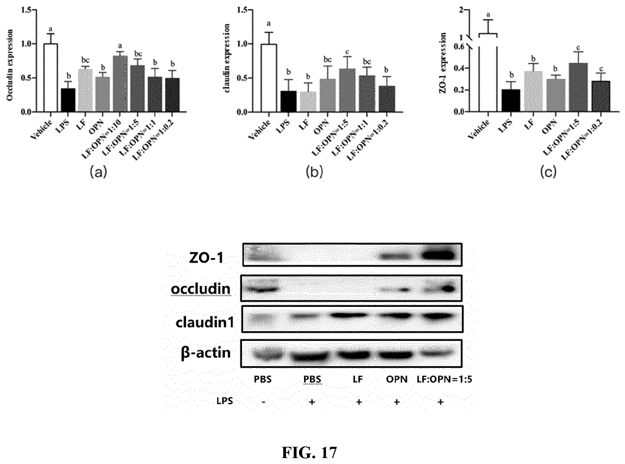

See FIG. 17, which shows the protective effect of protein combination on the integrity of the intestinal mucosal mechanical barrier. Based on Experimental Examples 4, 5, and 6, the effect of the protein combination on the expression of different intestinal junction proteins was further explored. The results showed that the combination at the compounding ratio LF:OPN=1:10-1:0.0.2 was better than the monomers, which significantly increased the expression of intestinal junction proteins occludin, claudin1, and zo-1, and was beneficial to maintaining the integrity of the intestinal mucosa.

In the following product examples:
Compound vitamin nutrient package: sodium L-ascorbate, lactose, taurine, dl-α-tocopherol acetate (50%), pyridoxine hydrochloride (5%), cholecalciferol (0.25%), retinyl acetate (11.5%), nicotinamide, D-calcium pantothenate, D-biotin (1%), phylloquinone (5 %), riboflavin (10%), folic acid (10%), thiamine nitrate, cyanocobalamin (1%);
Choline chloride nutrient package: choline chloride;
Mineral II nutrient package: tricalcium phosphate, sodium chloride, calcium carbonate, lactose;
Mineral I nutrient package: potassium iodide (0.5%), copper sulfate (5%), ferrous sulfate (79.2%), zinc sulfate, sodium selenite (0.5%), manganese sulfate (5%), lactose;
Magnesium chloride nutrient package: magnesium chloride;
Potassium chloride nutrient package: potassium chloride.

### Product Example 6

This product example provides an infant formula milk powder containing a complex of lactoferrin and osteopontin that has resisting enteritis and/or protection of integrity of intestinal barrier. The total protein content of the formula was 13.0 g/100g, the fat content was 23.0 g/100g; the carbohydrate content was 52g/100g, the lactoferrin content was 24 mg/100g, and the osteopontin content was 120 mg/100g. The mass ratio of the lactoferrin to the osteopontin was 1:5.

This product example was realized by compounding the following raw materials in parts by weight, and the raw material composition comprised:
1950 parts of raw milk, 250 parts of desalted whey powder (D90), 204 parts of lactose, 155 parts of edible vegetable blending oil, 85 parts of galacto-oligosaccharide, 65 parts of whey protein powder (WPC80), 5 parts of fructo-oligosaccharide, 2 parts of α-lactalbumin powder, 4 parts of phospholipids, 18 parts of compound nutrients, wherein the compound nutrients comprised about 3 parts of compound vitamin nutrient package, about 2 parts of choline chloride nutrient package, about 9 parts of mineral II nutrient package, about 1 part of mineral I nutrient package, about 1 part of magnesium chloride nutrient package and about 2 parts of potassium chloride nutrient package, and the base material of each nutrient package was lactose, 10 parts of DHA, 12 parts of ARA, 0.2 parts of bifidobacteria, about 0.7 parts of nucleotides, and about 0.4 parts of lactoferrin.

The specific preparation process of infant formula milk powder in this product example was as follows:
1) Coarse filtration of milk: after the milk was coarsely filtered and degassed in the balance cylinder, it was preheated in a plate heat exchanger and separated from impurities by a separator.
2) Homogenization and sterilization of milk: after impurities were removed, part of the raw milk entered the homogenizer for homogenization, and the other part was not homogenized. The two parts were mixed after homogenization and entered the sterilization system for sterilization.
3) Addition of powder: various powder raw materials were added to the powder mixing tank through an air delivery system after measured according to the formula, and were sucked into a vacuum mixing tank through a vacuum system;
4) Melting oil: according to the formula requirements, the oil specified in the formula was placed in an oil melting room, and the temperature of the oil melting room was maintained at 50-90°C. After the oil was melted, it was pumped into a mixed oil storage tank, and the mixed oil was pumped into the mixing tank through the oil pump according to the formula requirements;
5) Dissolution and addition of nutrient: calcium powder, vitamins, minerals and other nutrient packages were dissolved separately with pure water, and then added to the mixing tank in sequence to obtain a mixed liquid.
6) Filtration: the mixed liquid was filtered through a filter to remove physical impurities that might be brought into the raw materials.
7) Homogenization: the mixed liquid was homogenized by a homogenizer, and the fat globules were mechanically treated to disperse into uniform fat globules.
8) Cooling and storage: the homogenized liquid entered a plate heat exchanger for cooling: it was cooled to below 20°C, temporarily stored in a pre-storage tank, and entered the next process within 6 hours. An agitator was turned on according to the set requirements.
9) Concentration and sterilization: double-effect concentration was used during production, the sterilization temperature was ≥ 83°C, and the sterilization time was 25 seconds. The discharge concentration was 50% dry matter.
10) Storage, preheating, filtration, and spray drying of concentrated milk: the concentrated milk was temporarily stored in a concentrated milk balance tank. It was preheated to 60°C by a scraper preheater. After preheated, the material was filtered through a filter with a pore size of 1mm, and then pumped by a high-pressure pump into a drying tower for spray drying. The fine powder was agglomerated at the top of the tower or in the fluidized bed as required. Inlet air temperature: 180°C, exhaust air temperature 86°C, high pressure pump pressure 200bar, tower negative pressure -4mba.
11) Fluidized bed drying and cooling: the powder coming out of the drying tower was dried twice in a fluidized bed (first level), and then cooled to 30°C in a fluidized bed (second level) to obtain the main ingredient of milk powder.
12) Packaging: according to the formula requirements, DHA, ARA or bifidobacteria was weighed and sealed in bags.
13) Dry mixing: the weighed DHA, ARA or bifidobacteria was mixed with the main ingredient of milk powder in the dry mixer.
14) Powder screening: a vibrating screen was used to make the particle size of the milk powder uniform, and the powder residue was discarded.
15) Powder discharging: a sterilized powder collection box was used to collect the powder, which was then transported from a powder discharging room to a powder loading room.
16) Powder loading: the milk powder was poured into the powder storage tank of large and small packaging machines according to the packaging requirements.
17) Packaging: 400g of the milk powder was packaged with nitrogen flushed by an automatic packaging machine. The oxygen content was less than 1% when nitrogen was flushed. 900g of milk powder was packaged by iron cans automatically with nitrogen flushed, and the oxygen content was less than 5%.
18) Packing: the packaged small bags were put into a carton and a powder spoon was added at the same time, and the carton was sealed with a carton sealing machine.
19) Inspection of finished products: sampling inspection was carried out on the packaged products according to the inspection plan.
20) Storage: products that had passed the inspection were stored in a warehouse, and were required to be stored at room temperature and humidity ≤65%.

### Product Example 7

This product example provides an infant formula milk powder containing a complex of lactoferrin and osteopontin that has resisting enteritis and/or protection of the integrity of intestinal barrier. The total protein content of the formula was 11.5 g/100g, the fat content was 27.5 g/100g; the carbohydrate content was 53g/100g, the lactoferrin content was 6.8 mg/100g, and the osteopontin content was 20.4 mg/100g. The mass ratio of lactoferrin to osteopontin was 1:3.

This product example was realized by compounding the following raw materials in parts by weight, and the raw material composition comprised:
1300 parts of raw cow milk, 350 parts of desalted whey powder (D90), 213 parts of edible vegetable blending oil, 157 parts of lactose, 40 parts of galacto-oligosaccharide, 32 parts of α-lactalbumin powder, 22 parts of fructo-oligosaccharide, 16 parts of casein, 4 parts of phospholipids, 24 parts of compound nutrients, wherein the compound nutrients comprised about 3.5 parts of compound vitamin nutrient package, about 1.5 parts of choline chloride nutrient package, about 12 parts of mineral II nutrient package, about 1 part of mineral I nutrient package, about 2 parts of magnesium chloride nutrient package and about 4 parts of potassium chloride nutrient package, and the base material of each nutrient package included lactose, 12 parts of DHA, 14 parts of ARA, 0.2 parts of bifidobacteria, and about 0.7 parts of nucleotides.

The specific preparation process of the infant formula milk powder of this product example was consistent with that of Product Example 6.

### Product Example 8

This product example provides an infant formula milk powder containing a complex of lactoferrin and osteopontin that has resisting enteritis and/or protection of the integrity of intestinal barrier. The total protein content of the formula was 10.5 g/100g, the fat content was 28.0 g/100g; the carbohydrate content was 54g/100g, the lactoferrin content was 211.5mg/100g, and the osteopontin content was 21.2mg/100g. The mass ratio of lactoferrin and osteopontin was 1:0.1.

This product example was realized by compounding the following raw materials in parts by weight, and the raw material composition comprised:
1000 parts of raw cow milk, 375 parts of desalted whey powder (D90), 230 parts of edible vegetable blending oil, 155 parts of lactose, 40 parts of galacto-oligosaccharide, 30 parts of α-lactalbumin powder, 20 parts of fructo-oligosaccharide, 17 parts of casein, 4 parts of phospholipids, 23 parts of compound nutrients, wherein the compound nutrients comprised about 2.5 parts of compound vitamin nutrient package, about 1.5 parts of choline chloride nutrient package, about 12 parts of mineral II nutrient package, about 1 part of mineral I nutrient package, about 2 parts of magnesium chloride nutrient package and about 4 parts of potassium chloride nutrient package, and the base material of each nutrient package included lactose, 12 parts of DHA, 14 parts of ARA, 0.2 parts of bifidobacteria, about 0.7 parts of nucleotides, and about 2.4 parts of lactoferrin.

The specific preparation process of the infant formula milk powder of this product example was consistent with that of Product Example 6.

**In** the following product examples, the compound vitamin nutrient package comprised: retinyl acetate, cholecalciferol, dl-α-tocopherol acetate, phylloquinone, thiamine nitrate, riboflavin, pyridoxine hydrochloride, cyanocobalamin, niacinamide, folic acid, D-calcium pantothenate, sodium L-ascorbate, D-biotin, inositol, taurine.

Choline chloride nutrient package comprised: choline chloride.

Calcium powder nutrient package comprised: calcium carbonate, calcium hydrogen phosphate, tricalcium phosphate.

Sodium potassium nutrient package comprised: sodium citrate, potassium citrate, potassium chloride.

Mineral nutrient package comprised: ferrous sulfate, zinc sulfate, copper sulfate, sodium selenite, potassium iodide, manganese sulfate.

Magnesium chloride nutrient package comprised: magnesium chloride.

Product Example 9: the raw materials for the preparation of formula food containing nutritional components for premature/low birth weight infant (preparation of 1000 kg) comprised:
80 kg of hydrolyzed whey protein powder, 115 kg of lactose, 460 kg of solid corn syrup, 130 kg of high oleic sunflower oil, 40 kg of corn oil, 70 kg of soybean oil, 90 kg of OPO structured fat, 5 kg of lactoferrin, 0.5 kg of osteopontin, 38 kg of compound nutrients, 9 kg of DHA, 18 kg of ARA, 0.1 kg of bifidobacteria, and 0.65 kg of nucleotides.

The compound nutrients package comprised about 3.0 kg of compound vitamin nutrient package, about 2.0 kg of choline chloride nutrient package, about 12 kg of calcium powder nutrient package, 16 kg of sodium potassium nutrient package, about 2 kg of mineral nutrient package, and about 3.0 kg of magnesium chloride nutrient package. The base material of each nutrient package was solid corn syrup.

The specific preparation process of the formula food containing nutritional components for premature/low birth weight infant was as follows:
1) Addition of powder: various powder raw materials were added to a powder mixing tank through an air delivery system after being measured according to the formula for storage.
2) Vacuum suction of powder: various powder raw materials in the powder mixing tank were sucked into a vacuum mixing tank through a vacuum system.
3) Melting oil: the oil specified in the formula was put into an oil melting room according to the formula requirements. The temperature of the oil melting room should be maintained at 50-90°C. After the oil was melted, it was pumped into the storage tank of mixed oil through the oil pump and flow meter according to the formula ratio requirements.
4) Storage of mixed oil: the mixed oil was stored in an oil storage tank at a temperature of 40-50°C, and the storage time was less than 12 hours to prevent fat oxidation.
5) Weighing: the mixed oil was pumped into a mixing tank through an oil pump according to the formula requirements.
6) Dissolution and addition of nutrient: calcium powder, minerals, and vitamins were added separately, dissolved with 100-200kg of pure water separately, and then pumped into a wet mixing tank. After each one was pumped, 100kg of pure water was used to rinse the addition tank and pipeline.
7) Filtration: the mixed liquid was filtered through a filter to remove physical impurities that might be brought into the raw materials.
8) Homogenization: the mixed liquid was homogenized by a homogenizer with a first-level homogenization pressure of 105±5bar and a second-level pressure of 32±3bar. The fat globules were mechanically treated to disperse into uniform fat globules.
9) Cooling and storage: the homogenized liquid entered a plate heat exchanger for cooling: it was cooled to below 20°C and temporarily stored in a pre-storage tank. It entered the next process within 6 hours, and an agitator was turned on according to the set requirements.
10) Concentration and sterilization: double-effect concentration was used during production, the sterilization temperature was ≥ 83°C, and the sterilization time was 25 seconds. The discharge concentration was 48%-52% dry matter.
11) Storage, preheating, filtration, and spray drying of liquid: the concentrated liquid was temporarily stored in a liquid balance tank, and preheated to 60-70°C by a scraper preheater. After preheated, the material was filtered through a filter with a pore size of 1mm, and then pumped into a drying tower for spray drying with a high-pressure pump. The fine powder was agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
12) Fluidized bed drying and cooling: the powder coming out of the drying tower was then dried twice in a fluidized bed (first level), and then cooled to 25-30°C in a fluidized bed (secondary level) to obtain a powdered material.
13) Packaging: the staff of the powder making workshop weighed DHA, ARA, osteopontin, lactoferrin, and bifidobacteria and sealed them into bags according to the formula requirements.
14) Dry mixing: the weighed DHA, ARA, osteopontin, lactoferrin, bifidobacteria and powdered materials were mixed in a dry mixer. If the protein content was high, some casein, whey protein powder and sodium caseinate could be mixed and added evenly by dry mixing to obtain powdered formula food.
15) Powder screening: the particle size of powdered formula food was uniformed by a vibrating screen, and the powder residue was discarded.
16) Powder discharging: a sterilized powder collecting box was used to collect the powder, which was then transported from a powder discharging room to a powder loading room.
17) Powder loading: the powdered formula food was poured into a powder storage tank of large and small packaging machines according to the packaging requirements.
18) Packaging: 800g of the milk powder was packaged with nitrogen flushed by automatic packaging machine. The oxygen content was less than 1% when nitrogen was flushed. 900g of milk powder was packaged by iron cans automatically with nitrogen flushed, and the oxygen content was less than 5%.
19) Packing: the packaged small bags were put into a carton and a powder spoon was added at the same time, and the carton was sealed with a carton sealing machine.
20) Inspection of finished products: sampling inspection was carried out on packaged products according to the inspection plan.
21) Storage: products that had passed the inspection were stored in a warehouse, and were required to be stored at room temperature with a humidity of ≤ 65%.

The protein content of this product was 6.4g/100g, the fat content was 33g/100g, and the carbohydrate content was 57.5g/100g. The lactoferrin content was 500mg/100g, and the osteopontin content was 50mg/100g.

Product Example 10: the raw materials for the preparation of lactose-free formula food containing nutritional components (preparation of 1000 kg) comprised:

90 kg of isolated whey protein, 100 kg of lactose-free whole milk powder, 458 kg of solid corn syrup, 120 kg of high-oleic sunflower oil, 40 kg of corn oil, 50 kg of soybean oil, 80 kg of OPO structured fat, 0.1 kg of breast milk oligosaccharide LNnT, 38 kg of compound nutrients, 9 kg of DHA, 18 kg of ARA, 0.65 kg of nucleotides, 7.5 kg of lactoferrin, and 27.5 kg of osteopontin.

Among them, the compound nutrients comprised about 3.0 kg of compound vitamin nutrient package, about 2.0 kg of choline chloride nutrient package, about 12 kg of calcium powder nutrient package, 16 kg of sodium potassium nutrient package, about 2 kg of mineral nutrient package, and about 3.0 kg of magnesium chloride nutrient package. The base material of each nutrient package was solid corn syrup.

The specific preparation process of lactose-free formula food containing nutritional components was as follows:
1) Addition of powder: various powder raw materials were added to a powder mixing tank through an air delivery system after being measured according to the formula and stored.
2) Vacuum suction of powder: various powder raw materials in the powder mixing tank were sucked into a vacuum mixing tank through a vacuum system.
3) Melting oil: the oil specified in the formula was put into an oil melting room according to the formula requirements. The temperature of the oil melting room should be maintained at 50-90°C. After the oil was melted, it was pumped into a mixed oil storage tank through an oil pump and a flow meter according to the formula ratio requirements.
4) Storage of mixed oil: the mixed oil was stored in an oil storage tank at a temperature of 40-50°C, and the storage time was less than 12 hours to prevent fat oxidation.
5) Weighing: the mixed oil was pumped into a mixing tank through an oil pump according to the formula requirements.
6) Dissolution and addition of nutrients: calcium powder, minerals, and vitamins were added separately, dissolved with 100-200kg of pure separately, and pumped into the wet mixing tank. After each one was pumped, 100kg of pure water was used to rinse the addition tank and pipeline.
7) Filtration: the mixed liquid was filtered through a filter to remove physical impurities that may be brought into the raw materials.
8) Homogenization: the mixed liquid was homogenized by a homogenizer with a first-level homogenization pressure of 105±5bar and a second-level pressure of 32±3bar. The fat globules were mechanically treated to disperse into uniform fat globules.
9) Cooling and storage: the homogenized liquid entered a plate heat exchanger for cooling: it was cooled to below 20°C and temporarily stored in a pre-storage tank. It entered the next process within 6 hours, and an agitator was turned on according to the set requirements.
10) Concentration and sterilization: double-effect concentration was used during production, the sterilization temperature was ≥ 83°C, and the sterilization time was 25 seconds. The discharge concentration was 48%-52% dry matter.
11) Storage, preheating, filtration, and spray drying of liquid: the concentrated liquid was temporarily stored in a liquid balance tank. It was preheated to 60-70°C by the scraper preheater. After preheated, the material was filtered through a filter with a pore size of 1mm, and then pumped by a high-pressure pump into a drying tower for spray drying. The fine powder was agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
12) Fluidized bed drying and cooling: the powder coming out of the drying tower was dried twice in a fluidized bed (first level) and cooled to 25-30°C in a fluidized bed (second level).
13) Packaging: the staff of powder making workshop weighed the DHA, ARA, lactoferrin and osteopontin and sealed them into bags according to the formula requirements.
14) Dry mixing: the weighed DHA, ARA, lactoferrin, osteopontin and powdered materials were mixed in a dry mixer. If the protein content was high, some casein, whey protein powder and sodium caseinate could be mixed and added evenly by dry mixing to obtain powdered formula food.
15) Powder screening: the particle size of powdered formula food was uniformed by vibrating screen, and the powder residue was discarded.
16) Powder discharging: a sterilized powder collecting box was used to collect the powder, which was then transported from a powder discharging room to a powder loading room.
17) Powder loading: the powdered formula food was poured into a powder storage tank of large and small packaging machines according to the packaging requirements.
18) Packaging: 800g of the milk powder was packaged with nitrogen flushed by automatic packaging machine. The oxygen content was less than 1% when nitrogen was flushed. 900g of milk powder was packaged by iron cans automatically with nitrogen flushed, and the oxygen content was less than 5%.
19) Packing: the packaged small bags were put into a carton and a powder spoon was added at the same time, and the carton was sealed with a carton sealing machine.
20) Inspection of finished products: sampling inspection was carried out on packaged products according to the inspection plan.
21) Storage: products that had passed the inspection were stored in a warehouse, and were required to be stored at room temperature with a humidity of ≤ 65%.

The protein content of this product was 9.0g/100g, the fat content was 29g/100g, and the carbohydrate content wass 45.8g/100g. The lactoferrin content was 750mg/100g, and the osteopontin content was 2750mg/100g.

Product Example 11: the raw materials for the preparation of a formula food for patients with tumor containing nutritional components that can protect the intestinal mucosal barrier integrity (preparation of 1000 kg) comprised:
60 kg of sodium caseinate, 60 kg of casein, 200 kg of whey protein powder, 340 kg of maltodextrin, 40 kg of corn oil, 50 kg of soybean oil, 30 kg of medium-chain triglycerides, 30 kg of fructo-oligosaccharide, 6 kg of lactoferrin, 30 kg of osteopontin, 1 kg of glutamine, 38 kg of compound nutrients, 50 kg of DHA, 80 kg of EPA, 0.5 kg of arginine, and 0.5 kg of tryptophan.

Among them, the compound nutrients comprised about 3.0 kg of compound vitamin nutrient package, about 2.0 kg of choline chloride nutrient package, about 12 kg of calcium powder nutrient package, 16 kg of sodium potassium nutrient package, about 2 kg of mineral nutrient package, and about 3.0 kg of choline chloride nutrient package. The base material of each nutrient package was maltodextrin.

The specific preparation process of the formula food containing lactoferrin and osteopontin was as follows:
1) Addition of powder: various powder raw materials were added to a powder mixing tank through an air delivery system after being measured according to the formula for storage.
2) Vacuum suction of powder: various powder raw materials in the powder mixing tank were sucked into a vacuum mixing tank through a vacuum system.
3) Melting oil: the oil specified in the formula was put into an oil melting room according to the formula requirements. The temperature of the oil melting room should be maintained at 50-90°C. After the oil was melted, it was pumped into the storage tank of mixed oil through an oil pump and a flow meter according to the formula ratio requirements.
4) Storage of mixed oil: the mixed oil was stored in an oil storage tank at a temperature of 40-50°C, and the storage time was less than 12 hours to prevent fat oxidation.
5) Weighing: the mixed oil was pumped into the mixing tank through an oil pump according to the formula requirements.
6) Dissolution and addition of nutrients: calcium powder, minerals, and vitamins were added separately, dissolved with 100-200kg of pure water separately, and then pumped into a wet mixing tank. After each one was pumped, 100kg of pure water was used to rinse the addition tank and pipeline.
7) Filtration: the mixed liquid was filtered through a filter to remove physical impurities that might be brought into the raw materials.
8) Homogenization: the mixed liquid was homogenized by a homogenizer, with a first-level homogenization pressure of 105±5bar and a second-level pressure of 32±3bar. The fat globules were mechanically treated to disperse into uniform fat globules.
9) Cooling and storage: the homogenized liquid entered a plate heat exchanger for cooling: it was cooled to below 20°C and temporarily stored in a pre-storage tank. It entered the next process within 6 hours, and an agitator was turned on according to the set requirements.
10) Concentration and sterilization: double-effect concentration was used during production, the sterilization temperature was ≥ 83°C, and the sterilization time was 25 seconds. The discharge concentration was 48%-52% dry matter.
11) Storage, preheating, filtration, and spray drying of liquid: the concentrated liquid was temporarily stored in a liquid balance tank. It was preheated to 60-70°C by the scraper preheater. After preheated, the material was filtered through a filter with a pore size of 1mm, and then pumped by a high-pressure pump into a drying tower for spray drying. The fine powder was agglomerated at the top of the tower or in the fluidized bed as required. Inlet air temperature: 165-180°C, exhaust air temperature 75-90°C, high-pressure pump pressure 160-210bar, tower negative pressure -4 to -2mbar.
12) Fluidized bed drying and cooling: the powder coming out of the drying tower was dried twice in a fluidized bed (first level) and cooled to 25-30°C in a fluidized bed (second level).
13) Packaging: the staff of the powder -making workshop weighed the DHA, ARA, and lactoferrin in bags, which were then sealed into bags according to the formula requirements.
14) Dry mixing: the weighed DHA, EPA, ARA, lactoferrin, osteopontin and powdered materials were mixed evenly in a dry mixer. If the protein content was high, some casein, whey protein powder and sodium caseinate could be mixed and added evenly by dry mixing to obtain powdered formula food.
15) Powder screening: the particle size of powdered formula food was uniformed by vibrating screen, and the powder residue was discarded.
16) Powder discharging: a sterilized powder collecting box was used to collect the powder, which was then transported from a powder discharge room to a powder loading room.
17) Powder loading: the powdered formula food was poured into a powder storage tank of large and small packaging machines according to the packaging requirements.
18) Packaging: 800g of the milk powder was packaged with nitrogen flushed by automatic packaging machine. The oxygen content was less than 1% when nitrogen was flushed. 900g of milk powder was packaged by iron cans automatically with nitrogen flushed, and the oxygen content was less than 5%.
19) Packing: the packaged small bags were put into a carton and a powder spoon was added at the same time, and the carton was sealed with a carton sealing machine.
20) Inspection of finished products: sampling inspection was carried out on the packaged products according to the inspection plan.
21) Storage: products that had passed the inspection were stored in a warehouse, and were required to be stored at room temperature with a humidity of ≤ 65%.

The protein content of this product was 32g/100g, the fat content was 12g/100g, the carbohydrate content was 34g/100g, the lactoferrin content was 600mg/100g, and the osteopontin content was 3000mg/100g.

Product Example 12: the raw materials for the preparation of a formula food for patients with traumatic stress containing nutritional components that can protect the intestinal mucosal barrier integrity (preparation of 1000 kg) comprised:
60 kg of sodium caseinate, 80 kg of casein, 200 kg of whey protein powder, 340 kg of maltodextrin, 40 kg of corn oil, 45 kg of soybean oil, 50 kg of medium-chain triglycerides, 30 kg of fructo-oligosaccharide, 6 kg of lactoferrin, 30 kg of osteopontin, 1 kg of glutamine, 38 kg of compound nutrients, 30 kg of DHA, 60 kg of EPA, 1 kg of arginine, and 1 kg of tryptophan.

Among them, the compound nutrients comprised about 3.0 kg of compound vitamin nutrient package, about 2.0 kg of choline chloride nutrient package, about 12 kg of calcium powder nutrient package, 16 kg of sodium potassium nutrient package, about 2 kg of mineral nutrient package, and about 3.0 kg of choline chloride nutrient package. The base material of each nutrient package was maltodextrin.

The product preparation process was as in Example 10.

The protein content of this product was 34g/100g, the fat content was 13.5g/100g, the carbohydrate content was 34g/100g, the lactoferrin content was 600mg/100g, and the osteopontin content was 3g/100g.

The above are only preferred embodiments of the present disclosure. It should be noted that for those of ordinary skills in the art, several changes and modifications can be made without departing from the principle of the present disclosure, and these changes and modifications should also be regarded as the scope of protection of the present disclosure.

## Claims

1. A composition, consisting of lactoferrin, osteopontin and calcium salt, and a mass ratio of the lactoferrin, osteopontin and calcium in calcium salt is 1: (0.05-5): (0.001-40).

2. The composition according to claim 1, wherein the mass ratio of the lactoferrin, osteopontin and calcium in calcium salt is 1: (0.1-5): (0.001-40).

3. The composition according to claim 1, wherein the mass ratio of the lactoferrin, osteopontin and calcium in calcium salt is 1: (0.15-5): (0.006-20).

4. The composition according to claim 1, wherein the mass ratio of the lactoferrin, osteopontin and calcium in calcium salt is 1: (0.15-5): (0.01-10).

5. The composition according to claim 1, wherein the mass ratio of lactoferrin, osteopontin and calcium in calcium salt is 1: (0.15-5): (0.01-0.1).

6. The composition according to claim 1, wherein the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

7. Use of the composition according to any one of claims 1 to 6 in the manufacture of an inhibitor of inflammatory factor expression.

8. The use according to claim 7, wherein the inflammatory factor is IL-1β and/or IL-6.

9. Use of the composition according to any one of claims 1 to 6 in the manufacture of an anti-inflammation product.

10. The use according to claim 9, wherein the anti-inflammation comprises inhibiting expression of an inflammatory pathway protein.

11. A food, comprising the composition according to any one of claims 1 to 6.

12. A dairy product, comprising lactoferrin, osteopontin and calcium salt,
wherein,
a mass fraction of the lactoferrin in the dairy product is 0.006% to 0.7%;
a mass fraction of the osteopontin in the dairy product is 0.01% to 0.04%; and
a mass fraction of the calcium in calcium salt in the dairy product is 0.01% to 1.5%.

13. The dairy product according to claim 12, wherein a mass ratio of the lactoferrin, osteopontin and calcium is 1: (0.1 to 5): (0.001 to 40).

14. The dairy product according to claim 12, wherein the lactoferrin is derived from one or more selected from the group consisting of cow milk or its products, goat milk or its products, and camel milk or its products.

15. The dairy product according to claim 12, wherein the osteopontin is derived from one or more selected from the group consisting of cow milk or its products, goat milk or its products, and camel milk or its products.

16. The dairy product according to claim 12, wherein the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

17. The dairy product according to claim 12, wherein the dairy product further comprises one or more selected from the group consisting of milk fat, vegetable oil, fish oil, dietary fiber and probiotics.

18. The dairy product according to claim 17, wherein the vegetable oil is one or more selected from the group consisting of sunflower oil, corn oil, coconut oil, rapeseed oil, flaxseed oil, peanut oil and soybean oil;
the dietary fiber is one or more selected from the group consisting of galacto-oligosaccharide, fructo-oligosaccharide and breast milk oligosaccharide; and
the probiotics are one or more selected from the group consisting of bifidobacteria, yeast, probiotic spore bacteria, *Clostridium butyricum* and lactobacillus.

19. The dairy product according to any one of claims 12 to 18, wherein the dairy product is one or more selected from the group consisting of a dairy product for infants, a dairy product for pregnant women or new mothers, and a dairy product for pets.

20. The dairy product according to any one of claims 12 to 19, wherein, the dairy product comprises, in parts by weight, 0 to 2000 parts of raw cow milk, 120 to 350 parts of lactose, 0 to 300 parts of whole milk powder, 0 to 450 parts of skimmed milk powder, 0.01 to 375 parts of desalted whey powder, 0.01 to 170 parts of whey protein powder, 0 to 80 parts of sunflower oil, 0 to 40 parts of corn oil, 0 to 80 parts of soybean oil, 0 to 30 parts of coconut oil, 0 to 85 parts of low-erucic acid rapeseed oil, 0-20 parts of flaxseed oil, 0-240 parts of 1,3-dioleyl-2-palmitoyl triglyceride, 0-55 parts of α-lactalbumin powder, 0-40 parts of β-casein powder, 0-5 parts of soybean phospholipid, 0-2 parts of anhydrous milk fat, 0-25 parts of fructo-oligosaccharide powder, 0-100 parts of galacto-oligosaccharide syrup, 0.01-5 parts of milk mineral salt, 2-5 parts of vitamins, 0-3 parts of choline chloride, 0-15 parts of minerals, 0-3 parts of magnesium chloride, 0-5 parts of potassium chloride, 0-20 parts of docosahexaenoic acid, 0-25 parts of eicosatetraenoic acid, 0-0.2 parts of bifidobacteria, 0-8.5 parts of lactoferrin, and 0-1 part of nucleotides.

21. Use of the dairy product according to any one of claims 12 to 20 in the manufacture of an anti-inflammation product.

22. A nutritional composition, comprising protein, fat, carbohydrate and calcium salt, wherein
the protein comprises lactoferrin and osteopontin;
in the nutritional composition,a total content of the protein is (9-50g)/100g, a content of the fat is (12-30g)/100g, a content of the carbohydrate is (30-60g)/100g, a content of the lactoferrin is (0.005-3g)/100g, a content of the osteopontin is (0.01-1g)/100g, and a content of the calcium is (0.1-2g)/100g.

23. The nutritional composition according to claim 22, wherein a mass ratio of the lactoferrin, osteopontin and calcium is 1: (0.1-5): (0.001-40).

24. The nutritional composition according to claim 22, wherein in the nutritional composition, a total content of the protein is (15-35g)/100g, a content of the fat is (12-20g)/100g, a content of the carbohydrate is (32-50g)/100g, a content of the lactoferrin is (0.005-0.8g)/100g, a content of the osteopontin is (0.03-0.5g)/100g, and a content of the calcium is (0.1-1g)/100g.

25. The nutritional composition according to claim 22, wherein the lactoferrin is derived from one or more selected from the group consisting of cow milk or its products, goat milk or its products, and camel milk or its products;
the osteopontin is derived from one or more selected from the group consisting of cow milk or its products, goat milk or its products, and camel milk or its products; and
the calcium salt is one or more selected from the group consisting of calcium carbonate, calcium gluconate, calcium citrate, calcium lactate, calcium hydrogen phosphate, calcium L-threonine, calcium glycinate, calcium aspartate, calcium acetate, calcium chloride, tricalcium phosphate, vitamin E calcium succinate, calcium glycerophosphate, calcium sulfate, calcium dihydrogen phosphate, milk mineral salt, calcium caseinate, calcium malate and calcium ascorbate.

26. The nutritional composition according to claim 22, wherein a raw material for providing the protein comprises one or more selected from the group consisting of sodium caseinate, calcium caseinate, casein, hydrolyzed whey protein, hydrolyzed casein, hydrolyzed milk protein, hydrolyzed milk fat globule membrane protein, and isolated whey protein; and
a raw material for providing the fat comprises one or more selected from the group consisting of milk fat, vegetable oil, fish oil, and triglyceride.

27. The nutritional composition according to claim 22, wherein the nutritional composition further comprises one or more selected from the group consisting of amino acids, nucleotides, vitamins, probiotics, and inorganic salts.

28. The nutritional composition according to claim 22, wherein the nutritional composition specifically comprises 0-200 parts by weight of whey protein powder; 0-100 parts by weight of sodium caseinate; 0-100 parts by weight of casein; 0-400 parts by weight of solid corn syrup; 0-400 parts by weight of maltodextrin; 0-50 parts by weight of resistant dextrin; 0-50 parts by weight of fructo-oligosaccharide; 0-150 parts by weight of sunflower oil; 0-40 parts by weight of corn oil; 20-80 parts by weight of soybean oil; 0-100 parts by weight of medium-chain triglycerides; 0-10 parts by weight of tryptophan; 0-10 parts by weight of arginine; 0.01-7.5 parts by weight of lactoferrin; 0.75-37.5 parts by weight of osteopontin; 0.0075-300 parts by weight of calcium salt; 7-50 parts by weight of a compound nutrient package comprising calcium powder, vitamins and minerals; 5-80 parts by weight of DHA; 0-100 parts by weight of glutamine; and 10-140 parts by weight of EPA.

29. Use of the nutritional composition according to any one of claims 22 to 28 in the manufacture of an anti-inflammation product.

30. The use according to claim 29, wherein the anti-inflammation product is a food.

31. The use according to claim 30, wherein the anti-inflammation product is a formula food for special medical purpose.

32. The use according to claim 29, wherein the anti-inflammation is to resist chronic inflammation.

33. The use according to claim 32, wherein a patient with the chronic inflammation is a patient with tumor or a patient with traumatic stress.

34. Use of a nutritional component in the manufacture of a product for resisting enteritis and/or protecting integrity of intestinal barrier, wherein the nutritional component comprises a complex of lactoferrin and osteopontin.

35. The use according to claim 34, wherein the lactoferrin and osteopontin are combined to each other through electrostatic interaction;
preferably, a mass ratio of the lactoferrin to osteopontin is 1:10-1:0.05, preferably 1:5-1:0.1.

36. The use according to claim 34, wherein sources of lactoferrin and osteopontin include cow milk, goat milk and their products, including but not limited to whole milk powder, skimmed milk powder, casein, and whey protein powder.

37. The use according to claim 34, wherein the enteritis includes *Escherichia coli* enteritis.

38. The use according to claim 34, wherein the resisting enteritis includes reducing the number of serum cytokines IL-1β, IL-4, and IL-10, reducing the number of neutrophils in intestinal tissues, and reducing the number of CD4⁺ T cells in intestinal tissues; and
the protecting integrity of intestinal barrier includes protecting against intestinal tissue damage, protecting mucus barrier, and protecting integrity of intestinal mucosal mechanical barrier.

39. The use according to claim 34, wherein the product is a food, a healthcare food, or a nutrient supplement.

40. The use according to claim 34, wherein the product includes a dairy product, and the dairy product includes milk powder, liquid milk, cheese, condensed milk, milk fat or other dairy products.

41. The use according to claim 34, wherein the product is a formula food.

42. The use according to claim 41, wherein a nutritional component in the formula food is a protein complex, which is selected from a protein complex formed by lactoferrin and osteopontin via electrostatic interaction in a mass ratio of 1:10 to 1:0.05.

43. The use according to claim 41, wherein the formula food is a formula milk powder, a formula food for special medical purpose or a health-care food.

44. The use according to claim 41, wherein a mass ratio of the lactoferrin and osteopontin is 1:5 to 1:0.1.

45. The use according to claim 41, wherein sources of the lactoferrin and osteopontin include cow milk, goat milk and their products, including but not limited to one or more selected from the group consisting of whole milk powder, skimmed milk powder, casein and whey protein powder.

46. The use according to claim 41, wherein the enteritis includes *Escherichia coli* enteritis.

47. The use according to claim 41, wherein the resisting enteritis includes reducing the number of serum cytokines IL-1β, IL-4, and IL-10, reducing the number of neutrophils in intestinal tissue, and reducing the number of CD4⁺ T cells in intestinal tissue.

48. The use according to claim 41, wherein the protecting integrity of intestinal barrier includes protecting against intestinal tissue damage, protecting mucus barrier, and protecting integrity of intestinal mucosal mechanical barrier.

49. The use according to claim 41, wherein a content of the lactoferrin in the formula food is 1-750 mg/100g, and a content of the osteopontin in the formula food is 20-500 mg/100g.

50. The use according to claim 41, wherein in the formula food, a content of total protein is 10-20g/100g, a proportion of the whey protein in the total protein is 40-70%; a content of the fat is 15-30g/100g; and a content of the dietary fiber is 0.95-6.3g/100g.

51. The use according to claim 41, wherein the formula food further comprises one or more of compound nutrients of probiotics, lactose, DHA, ARA, nucleotides, calcium powder, vitamins and minerals.

52. The use according to claim 41, wherein the formula food is infant formula milk powder, and the infant formula milk powder comprises by weight:
0-2000 parts of raw cow milk, 120-350 parts of lactose, 0-300 parts of whole milk powder, 0-325 parts of skimmed milk powder, 0-375 parts of desalted whey powder, 0-170 parts of whey protein powder, 0-80 parts of sunflower oil, 0-40 parts of corn oil, 0-80 parts of soybean oil, 0-30 parts of coconut oil, 0-85 parts of low-erucic acid rapeseed oil, 0-20 parts of flaxseed oil, 0-240 parts of 1,3-dioleyl-2-palmitoyl triglyceride, 0-55 parts of α-whey protein powder, 0-40 parts of β-casein powder, 0-5 parts of soybean phospholipid, 0-2 parts of anhydrous milk fat, 0-25 parts of fructo-oligosaccharide powder, 0-100 parts of galacto-oligosaccharide syrup, 2-5 parts of compound vitamin nutrient package, 0-3 parts of choline chloride nutrient package, 0-2 parts of mineral I nutrient package, 0-13 parts of mineral II nutrient package, 0-3 parts of magnesium chloride nutrient package, 0-5 parts of potassium chloride nutrient package, 0-20 parts of DHA, 0-25 parts of ARA, 0-0.2 parts of bifidobacterium, 0-8.5 parts of lactoferrin, and 0-1 part of nucleotide;
wherein, amounts of the raw cow milk, whole milk powder, and skimmed milk powder used are not all equal to 0; and amounts of the sunflower oil, corn oil, soybean oil, coconut oil, low-erucic acid rapeseed oil, flaxseed oil, and 1,3-dioleyl-2-palmitoyl triglyceride used are not all equal to 0.

53. The use according to claim 41, wherein the formula food is a formula food for special medical purpose.

54. The use according to claim 53, wherein in the formula food for special medical purpose, the nutritional component is selected from a protein complex formed by lactoferrin and osteopontin via electrostatic interaction in a mass ratio of 1:10-1:0.1.

55. The use according to claim 53, wherein the resisting enteritis and/or protection of integrity of intestinal barrier includes preventing enteritis in premature/low birth weight infants and/or protecting integrity of intestinal barrier of premature/low birth weight infants, preventing enteritis in lactose-intolerant infants and/or protecting integrity of intestinal barrier of lactose-intolerant infants, preventing enteritis in patients with tumor and/or protecting integrity of intestinal barrier of patients with tumor, preventing enteritis in patients with traumatic stress and/or protecting integrity of intestinal barrier of patients with traumatic stress.

56. The use according to claim 53, wherein a mass ratio of the lactoferrin to osteopontin is 1:5-1:0.1.

57. The use according to claim 53, wherein a mass ratio of the lactoferrin to osteopontin is 1:5-1:0.2.

58. The use according to claim 53, wherein the enteritis includes *Escherichia coli* enteritis.

59. The use according to claim 53, wherein the resisting enteritis includes reducing the number of serum cytokines IL-1β, IL-4, and IL-10, reducing the number of neutrophils in intestinal tissue, and reducing the number of CD4⁺ T cells in intestinal tissue.

60. The use according to claim 53, wherein the protecting integrity of intestinal barrier includes protecting against intestinal tissue damage, protecting mucus barrier, and protecting integrity of intestinal mucosal mechanical barrier.

61. The use according to claim 53, wherein a content of the lactoferrin in the formula food for special medical purpose is 1-750 mg/100g.

62. The use according to claim 53, wherein a content of the osteopontin in the formula food for special medical purpose is 20-600 mg/100g.

63. The use according to claim 53, wherein the formula food for special medical purpose includes a dairy product, and the dairy product includes milk powder or liquid milk.

64. The use according to claim 53, wherein the formula food for special medical purpose is a formula food for premature/low birth weight infants, and comprises raw materials of:
| | |
|---|---|
| whey protein powder | 80-180 parts by weight; |
| lactose | 0-580 parts by weight; |
| solid corn syrup | 0-580 parts by weight; |
| sunflower oil | 0-150 parts by weight; |
| corn oil | 0-40 parts by weight; |
| soybean oil | 20-80 parts by weight; |
| OPO structured lipid | 0-140 parts by weight; |
| lactoferrin | 0-7.5 parts by weight; |
| osteopontin | 0-75 parts by weight; |
| compound nutrients comprising calcium powder, vitamins and minerals | 7-50 parts by weight; |
| DHA | 2-15 parts by weight; |
| ARA | 3-22 parts by weight; |
| bifidobacteria | 0.1-0.2 parts by weight; and |
| nucleotides | 0.1-5 parts by weight; or |
the formula food for special medical purpose is a formula food for lactose-intolerant infants, and comprises raw materials of:
| | |
|---|---|
| isolated whey protein powder | 90-150 parts by weight; |
| lactose-free whole milk powder | 0-500 parts by weight; |
| solid corn syrup | 0-600 parts by weight; |
| sunflower oil | 0-150 parts by weight; |
| corn oil | 0-40 parts by weight; |
| soybean oil | 20-80 parts by weight; |
| OPO structured fat | 0-140 parts by weight; |
| lactoferrin | 0-7.5 parts by weight; |
| osteopontin | 0-75 parts by weight; |
| compound nutrients comprising calcium powder, vitamins and minerals | 7-50 parts by weight; |
| DHA | 2-15 parts by weight; |
| ARA | 3-22 parts by weight; and |
| nucleotide | 0.1-5 parts by weight; or |
the formula food for special medical purpose is a formula food suitable for patients with tumor, and comprises raw materials of:
| | |
|---|---|
| whey protein powder | 90-200 parts by weight; |
| sodium caseinate | 0-100 parts by weight; |
| casein | 0-100 parts by weight; |
| solid corn syrup | 0-400 parts by weight; |
| maltodextrin | 0-400 parts by weight; |
| sunflower oil | 0-150 parts by weight; |
| corn oil | 0-40 parts by weight; |
| soybean oil | 20-80 parts by weight; |
| medium chain triglycerides | 0-100 parts by weight; |
| fructo-oligosaccharides | 0-50 parts by weight; |
| lactoferrin | 0-7.5 parts by weight; |
| osteopontin | 0-75 parts by weight; |
| compound nutrients comprising calcium powder, vitamins and minerals | 7-50 parts by weight; |
| DHA | 5-80 parts by weight; |
| tryptophan | 0-10 parts by weight; |
| arginine | 0-10 parts by weight; |
| glutamine | 0-100 parts by weight; and |
| EPA | 10-140 parts by weight; or |
the formula food for special medical purpose is a formula food suitable for patients with traumatic stress, and comprises raw materials of:
| | |
|---|---|
| whey protein powder | 90-200 parts by weight; |
| sodium caseinate | 0-100 parts by weight; |
| casein | 0-100 parts by weight; |
| solid corn syrup | 0-400 parts by weight; |
| maltodextrin | 0-400 parts by weight; |
| sunflower oil | 0-150 parts by weight; |
| corn oil | 0-40 parts by weight; |
| soybean oil | 20-80 parts by weight; |
| medium chain triglycerides | 0-100 parts by weight; |
| fructo-oligosaccharides | 0-50 parts by weight; |
| lactoferrin | 0-7.5 parts by weight; |
| osteopontin | 0-75 parts by weight; |
| compound nutrients comprising calcium powder, vitamins and minerals | 7-50 parts by weight; |
| DHA | 5-50 parts by weight; |
| tryptophan | 0-10 parts by weight; |
| arginine | 0-10 parts by weight; |
| glutamine | 0-100 parts by weight; and |
| EPA | 10-100 parts by weight. |

65. A product for resisting enteritis and/or protecting integrity of intestinal barrier, comprising a complex of lactoferrin and osteopontin.

66. The product according to claim 65, wherein the product includes a dairy product, preferably, the dairy product includes milk powder, liquid milk, cheese, condensed milk, milk fat or other dairy products.

67. The product according to claim 65, wherein an amount of the lactoferrin added to the product is 1-750 mg/100g product, and an amount of the lactoferrin added to the product is 20-200 mg/100g product.

68. The product according to claim 65, wherein the lactoferrin and osteopontin are combined to each other through electrostatic interaction;
preferably, a mass ratio of the lactoferrin to osteopontin is 1:10-1:0.05, preferably 1:5-1:0.1.

69. The product according to claim 65, wherein sources of the lactoferrin and osteopontin include cow milk, goat milk and their products, including but not limited to whole milk powder, skimmed milk powder, casein and whey protein powder.
